# EUROPEAN PATENT SPECIFICATION

(11) **EP 4 129 308 B1**
(45) Date of publication and mention of the grant of the patent: **02.12.2026**
(21) Application number: 22155977.6
(22) Date of filing: 21.10.2015
(51) Int. Cl.: A01K 67/0276, A61K 35/12, A61K 35/407, A61P 17/02, A61P 43/00

(54) **TRIPLE TRANSGENIC PIGS SUITABLE FOR XENOGRAFT**
FÜR XENOTRANSPLANTAT GEEIGNETE, DREIFACH TRANSGENE SCHWEINE
PORCS TRIPLES TRANSGÉNIQUES APPROPRIÉS POUR LA XÉNOGREFFE

(30) Priority: 22.10.2014 US 201462067129 P
(43) Date of publication of application: 08.02.2023
(62) Document number(s) of the earlier application(s) in accordance with Art. 76 EPC: 15852175.7 / 3 220 925
(73) Proprietor: Indiana University Research & Technology Corporation, Bloomington IN 47405 (US)
(72) Inventor: TECTOR, Joseph A., Carmel, IN 46032 (US)
(74) Representative: Wilding, James Roger

(56) References cited:
- WO-A1-2014/066505
- US-A1- 2013 111 614
- GUERARD W. BYRNE ET AL: "Identification of New Carbohydrate and Membrane Protein Antigens in Cardiac Xenotransplantation :", TRANSPLANTATION, vol. 91, no. 3, February 2011 (2011-02-01), GB, pages 287 - 292, XP055481327, ISSN: 0041-1337, DOI: 10.1097/TP.0b013e318203c27d
- GUERARD W. BYRNE ET AL: "Cloning and expression of porcine [beta]1,4 N-acetylgalactosaminyl transferase encoding a new xenoreactive antigen", XENOTRANSPLANTATION, vol. 21, no. 6, September 2014 (2014-09-01), US, pages 543 - 554, XP055481364, ISSN: 0908-665X, DOI: 10.1111/xen.12124

## Description

### FIELD OF THE INVENTION

The present invention relates generally to the field of xenotransplantation and genetic modification to develop transgenic pigs, transgenic porcine organs, tissue or cells suitable for transplant into a human, particularly transgenic pigs with a reduced propensity to cause thrombocytopenia, a hyperacute rejection (HAR) response or platelet uptake.

### BACKGROUND

It is well known that transplants from one animal into another animal of the same species, such as human to human, are a routine treatment option for many serious conditions including kidney, heart, lung, liver and other organ disease and skin damage such as severe burn disease. However, it is well known that there are not enough suitable organs available for transplant to meet current or expected clinical demands for organ transplants. Approximately 100,000 patients are on the kidney transplant list, and they remain on the waiting list an average of nearly five years before receiving a transplant or dying. In patients with kidney failure, dialysis increases the length of time the patient can wait for a transplant. More than 18,000 patients are on the UNOS liver transplant national waiting list, yet less than 7,000 transplants are performed annually in the United States. There is no system comparable to dialysis available for patients with liver disease or liver failure.

Xenotransplantation, the transplant of organs, tissues or cells from one animal into another animal of a different species, such as the transplantation of a pig organ into a human recipient has the potential to reduce the shortage of organs available for transplant, potentially helping thousands of people worldwide. However, xenotransplantation using standard, unmodified pig tissue into a human or other primate is accompanied by severe rejection of the transplanted tissue. The rejection may be a hyperacute rejection, an acute rejection, a chronic rejection, may involve survival limiting thrombocytopenia coagulopathy or may be an acute humoral xenograft reaction (AHXR). The human hyperacute rejection response to pig antibodies present on transplanted tissue is so strong that the transplant tissue is typically damaged by the human immune system within minutes or hours of transplant into the human. Furthermore, different rejection mechanisms may predominate in an organ-preferred manner. See Demetris et al. 1998 "Antibody-mediated Rejection of Human Orthotopic Liver Allografts. A study of liver transplantation across ABO blood group barriers", Am J. Pathol 132:489-502; Nakamura et al 1993 "Liver allograft rejection in sensitized recipients. Observations in a Clinically Relevant Small Animal Model" Am J. Pathol. 142:1383-91; Furuya et al 1992. "Preformed Lymphocytotoxic Antibodies: the Effects of Class, Titer and Specificity on Liver v Heart Allografts" Hepatology16:1415-22; Tector et al 2001. "Rejection of Pig Liver Xenografts in Patients with Liver Failure: Implications for Xenotransplantation", Liver Transpl pp.82-9 . For example, early development of thrombocytopenic coagulopathy is a major factor in non-human primate recipient death following xeno-transplant of a pig liver. Yet, if antibody mediated xenograft rejection (AMXR, AMR) is prevented, non-human primate (NHP) recipients of pig kidneys do not develop significant thrombocytopenia nor exhibit clinical manifestations of coagulopathy. See for example Ekser et al. 2012 "Genetically Engineered Pig to Baboon Liver Xenotransplantation: Histopathology of Xenografts and Native Organs" PLoS ONE pp e29720; Knosalla et al 2009, "Renal and Cardia Endothelial Heterogeneity Impact Acute Vascular Rejection in Pig to Baboon Xenotransplantation", Am J Transplant 1006-16; Shimizu et al 2012. "Pathologic Characteristics of Transplanted Kidney Xenografts", J. Am. Soc. Nephrology 225-35 .

In addition to the need for organs, tissues and cells for transplantation, there is a shortage of safe blood for transfusion. Eleven million blood transfusions utilizing packed human red blood cells (RBC) are administered in the U.S. each year (National Blood Data Source, 1998). The U.S. blood supply is chronically inadequate. In 2001 it was anticipated that U.S. Blood Banks would obtain about 250,000 units less than optimally desired. Officials routinely forecast a critical national shortage during the summer months when regular blood donors go on vacation and college students also leave the major urban centers. Because the nation has a robust and competitive blood collection and distribution system, periodic shortages do not usually result in deaths, but elective surgeries may need to be postponed and non-critical needs are not met. As normally donated blood can only be stored for about 42 days and less than 5% of eligible donors give blood, severe weather conditions such as snowstorms or hurricanes often result in dangerously low blood reserves.

Not only is human blood a scarce resource, it also comes with a potential risk to the recipient. Despite viral screening processes, donated human blood is not 100% safe (FDA, Annual Summary of Fatalities Reported to the FDA Following Blood Collection and Transfusion, FY2013). The incidence of Hepatitis C and HIV in the general population necessitates costly and difficult testing of donated blood products. Because of the difficulty and expense of ensuring that human blood is free of any infectious microorganisms, it would be highly desirable to develop a source of red blood cells and other transfusion products that would be unlimited in quantity and free of infectious agents.

Pig cells express α(1,3) galactosyltransferase (αGal), and cytidine monophosphate-N-acetylneuraminic acid hydroxylase (CMAH), which are not found in human cells. Pig cells also express porcine β1,4 N-acetylgalactosaminyltransferase (β4GalNT2); many humans are thought to express a form of β4GalNT2 (Morton et al (1970) Vox Sang 19:472-482). The αGal enzyme catalyzes the formation of galactose-α1,3-galactose (αGal) residues on glycoproteins. CMAH converts the sialic acid N-acetylneuraminic acid (Neu5Ac) to N-glycolylneuraminic acid (Neu5Gc). Porcine β1,4 N-acetylgalactosaminyltransferase (β4GalNT2) catalyzes the terminal addition of N-acetylgalactosamine to a sialic acid modified lactose amine to yield Sd^{a}-like antigens including, but not limited to, Sd^{a}, and GalNAc β1,4[Neu5Ac α2,3] Gal β1-4GlcNAc β1-3 Gal, also known as the CAD or CT blood group antigen (Blanchard et al (1983) JBC 258:7691-7695). Porcine β4GalNT2 may catalyze the formation of additional glycans as well. Antibodies to the Neu5Gc, αGal and Sd^{a} epitopes are present in human blood prior to implantation of the tissue, and are involved in the intense and immediate antibody mediated rejection of implanted tissue. Antibodies to additional β4GalNT2 (Sd^{a} - like) epitopes may also be present in the patient's blood and may contribute to the antibody mediated rejection of implanted tissue.

Many strategies have been employed to address the rejection response including removing the genes encoding α(1,3) galactosyltransferase and CMAH to prevent expression of the enzymes, modifying the genes encoding α(1,3) galactosyltransferase and CMAH to reduce or limit expression of the enzymes, or otherwise limit the rejection response. U.S. Patent 7,795,493 to Phelps et al describes a method for the production of a pig that lacks any expression of functional αGal. For instance, U.S. Patent 7,547,816 to Day et al*,* describes a knockout pig with decreased expression of α(1,3) galactosyltransferase as compared to wild-type pigs. Although the Day pigs may have decreased expression of α(1,3) galactosyltransferase, Neu5Gc antigenic epitopes remain present and glycolipids from the Day pigs have αGal antigenic epitopes. Unfortunately, while the GTKO pig may have reduced anti-α-Gal antibodies as a barrier to xenotransplantation, studies using GTKO cardiac and renal xenografts in baboons show that the GTKO organs still trigger an immunogenic response, resulting in rejection or damage to the transplanted organ. Baboons transplanted with GTKO kidneys and treated with two different immunosuppressive regimens died within 16 days of surgery. Chen et *al* concluded "genetic depletion of Gal antigens does not provide a major benefit in xenograft survival" (Chen et al., (2005) Nature Med 11(12):1295-1298. U.S. Patent 7,560,538 to Koike et al and U.S. Patents 7,166,378 and 8,034,330 to Zhu et al describe methods for making porcine organs for transplantation that are less likely to be subject to delayed xenograft rejection and hyperacute rejection, respectively. The Zhu patents discuss reduction of CMAH activity or epitopes on porcine cells. However, Basnet *et al* examined the cytotoxic response of human serum to CMAH-/- mouse cells. Basnet *et al* concluded "the anti-Neu5Gc Ab-mediated immune response may be significantly involved in graft loss in xenogeneic cell transplantation, but not in organ transplantation" (Basnet et al., 2010 Xenotransplantation 17(6):440-448). U.S. Patent 6,166,288 to Diamond et al describes methods of preparing organs, tissues or cells for xenotransplantation into human beings with reduced rejection of the xenotransplant material. U.S. Patent 6,166,288 describes transgenic pigs expressing a fucosyltransferase gene that encodes an enzyme that purportedly removes certain xenoreactive antigens from porcine organs, tissues and cells. U.S. Patent 6,166,288 does not provide transgenic pigs with alterations in three porcine genes.

U.S. Patent 6,572,867 to Schwarz et al provide immunosuppressive compositions for reducing plasma levels of anti-(αGal(1,3)Gal) antibodies in a primate. Immunosuppressive therapies are known in the transplant arts. Immunosuppressive drug regimens increase the risk of infection as they dampen the patient's immune responses, require costly maintenance medicines, may include drugs that interact with other medications and may cause additional side effects such as weight gain. WO 2014/066505 A1 describes double knockout (GT/CMAH) pigs, organs and tissues. US 2013/0111614 A1 relates to methods and materials for reducing cardiac xenograft rejection.

Progress in this field is critically dependent upon the development of genetically modified pigs with modifications that reduce the rejection response. Unfortunately, developing homozygous transgenic pigs is a slow process, requiring as long as three years using traditional methods of homologous recombination in fetal fibroblasts followed by somatic cell nuclear transfer (SCNT), and then breeding of heterozygous transgenic animals to yield a homozygous transgenic pig. The development of new transgenic pigs for xenotransplantation has been hampered by the lack of pluripotent stem cells, relying instead on the fetal fibroblast as the cell upon which genetic engineering was carried out.

Thus there is a need in the art for an improved, simple, replicable, efficient and standardized method of producing triple knockout (αGal, β4GalNT2, CMAH) pigs having reduced αGal, Sd^{a}-like and Neu5Gc epitopes as a source of transplant material for organs, tissue, blood and cells for human transplant recipients.

### SUMMARY OF THE INVENTION

The invention provides a transgenic pig comprising a disrupted α(1,3)-galactosyltransferase gene, a disrupted cytidine monophosphate-N-acetylneuraminic acid hydroxylase (CMAH) gene and a disrupted β1,4 N-acetylgalactosaminyltransferase (β4GalNT2) gene in the nuclear genome of cells of said pig, wherein expression of α(1,3)-galactosyltransferase, CMAH, and β4GalNT2 is decreased as compared to a wild-type pig.

The invention also provides a porcine organ, tissue, or cell isolated from said transgenic pig, wherein the porcine organ, tissue, or cell has reduced α(1,3)-galactosyltransferase, CMAH and β4GalNT2 expression as compared to a porcine organ, tissue, or cell isolated from a wild-type pig. Said porcine organ, tissue, or cell may be selected from the group consisting of skin, heart, liver, kidney, lung, pancreas, thyroid, small bowel, blood and components of any thereof.

The invention also provides said organ for use in a method of increasing the duration of the period between when a human subject is identified as a subject in need of a human organ transplant and when the human organ transplant occurs, said method comprising providing said organ, and surgically attaching said organ to said human subject in a therapeutically effective manner.

The invention also provides said liver for use in a method of increasing the duration of the period between when a human subject is identified as a subject in need of a human liver transplant and when said human liver transplant occurs, said method comprising providing said liver, and surgically attaching said liver to said human subject in a therapeutically effective manner.

The invention also provides a transfusion product or skin related product obtained from said transgenic pig, wherein said transfusion product or skin related product is for transplantation into a human, said disruption of the α(1,3)-galactosyltransferase, CMAH and β4GalNT2 genes reduces the level of the αGal, Sd^{a}-like and Neu5Gc antigens in the transfusion product or skin related product, and said skin related product exhibits reduced premature separation from a wound.

The invention also provides said skin related product for use in treating a skin wound in a human subject, the use comprising the steps of providing said transgenic pig, preparing said skin related product, and applying said skin related product to the human subject, wherein premature separation of the skin related product from the human subject is reduced compared to a skin related product obtained from a wild type pig.

The invention also provides a method of preparing transplant material for xenotransplantation into a human subject, the method comprising providing said transgenic pig as a source of said transplant material and wherein said transplant material is selected from the group consisting of organs, tissues, transfusion products and cells, and wherein said transplant material has a reduced level of αGal antigens, a reduced level of Neu5GC antigens, and a reduced level of Sd^{a}-like antigens.

The invention also provides a porcine transplant material for use in a method of improving a hyperacute rejection related symptom in a human subject, wherein said porcine transplant material is isolated from said transgenic pig, further wherein said disruption of the α(1,3)-galactosyltransferase, CMAH and β4GalNT2 genes reduces the level of αGal, Sd^{a}-like and Neu5GC antigens in said porcine transplant material, the method comprising transplanting the porcine transplant material into a subject in need of a transplant, wherein a hyperacute rejection related symptom is improved as compared to when porcine transplant material from a wild-type pig is transplanted into a human subject.

The invention also provides a cell culture reagent that exhibits an altered epitope profile, wherein said cell culture reagent is isolated from said transgenic pig, further wherein said disruption of the α(1,3)-galactosyltransferase, CMAH and β4GalNT2 genes reduces the level of αGal, Sd^{a}-like and Neu5Gc epitopes in the cell culture reagent.

The invention also provides a method of producing a compound of interest with an altered epitope profile, said method comprising the steps of providing said cell culture reagent, and incubating an isolated cell capable of expressing said compound of interest with said cell culture reagent; and wherein the level of Neu5Gc, Sd^{a}-like or αGal epitopes on said compound of interest is lower than the level of said epitopes on said compound of interest when said compound of interest is produced from an isolated cell incubated with a cell culture reagent isolated from a wild-type pig.

The invention also provides a porcine transplant material for transplantation into a human subject, wherein said transplant material is isolated from said pig, further wherein said disruption of the α(1,3)-galactosyltransferase, CMAH and β4GalNT2 genes reduces the level of αGal, Sd^{a}-like and Neu5GC epitopes in said transplant material.

The invention also provides a transgenic pig comprising a disrupted α(1,3)-galactosyltransferase gene, a disrupted CMAH gene and a disrupted β4GalNT2 gene in the nuclear genome of cells of said pig, wherein the disruption of said α(1,3)-galactosyltransferase gene is selected from the group of disruptions comprising a 3 base pair deletion adjacent to a G to A substitution, a single base pair deletion, a single base pair insertion, a two base pair insertion, a six base pair deletion, a ten base pair deletion, a seven base pair deletion, an eight base pair insertion for a five base pair deletion, a five base pair insertion, an eleven base pair deletion and an eighteen base pair deletion; wherein the disruption of said CMAH gene is selected from the group of disruptions comprising a four base pair insertion, a one base pair deletion, a two base pair deletion, a three base pair deletion, a five base pair deletion, an eight base pair deletion, a twenty base pair deletion, an eleven base pair deletion, a twelve base pair deletion, a single base pair insertion, a two base pair insertion for single base pair deletion, a three base pair deletion for a four base pair insertion, a sixty-six base pair deletion and twelve base pair insertion, and a five base pair deletion with a one base pair substitution wherein the disruption of said β4GalNT2 gene is selected from the group of disruptions comprising a twelve base pair deletion, a five base pair deletion, a fourteen base pair deletion, a twelve base pair deletion and one base pair substitution, a 271 base pair deletion with a one base pair insertion, and a single base pair insertion, and wherein expression of α(1,3)-galactosyltransferase, CMAH and β4GalNT2 are decreased as compared to a wild-type pig, and when tissue from said transgenic pig is transplanted into a human subject, a hyperacute rejection related symptom is improved as compared to when tissue from a wild-type pig is transplanted into a human subject.

These and further embodiments are set out in the attached claims.

### BRIEF DESCRIPTION

The technical information set out below may in some respects go beyond the scope of the invention, which is defined by the appended claims. The additional technical information is provided to place the actual invention in a broader technical context and to illustrate possible related technical developments.

Any incidental references to methods for treatment of the human or animal body by surgery or therapy and diagnostic methods practiced on the human or animal body are not to be construed as claiming protection for such methods as such, but are instead to be construed as referring to products, in particular substances or compositions, for use in any of these methods.

This disclosure relates generally to methods of making porcine organs, tissues or cells with reduced α(1,3)galactosyltransferase, CMAH and β4GalNT2 expression for transplantation into a human. The invention is defined in the attached claims.

A transgenic pig comprising a disrupted α(1,3)-galactosyltransferase, CMAH and β4GalNT2 gene in the nuclear genome of cells of the pig is provided. Expression of α(1,3)-galactosyltransferase, CMAH and β 1,4 N-acetylgalactosaminyltransferase in the transgenic pig is decreased as compared to expression in a wild-type pig. A porcine organ, tissue, transfusion product, or cell obtained from the triple transgenic pig is provided as defined in the claims. The porcine organ, tissue or cell may be selected from the group consisting of skin, heart, liver, kidneys, lung, pancreas, thyroid, small bowel and components thereof. In an aspect, when tissue from the triple transgenic pig is transplanted into a human, a rejection related symptom is improved as compared to when tissue from a wild-type pig is transplanted into a human. In an aspect, the rejection related symptom is selected from the group comprising a cellular rejection response related symptom, a humoral rejection response related symptom, a hyperacute rejection related symptom, an acute humoral xenograft reaction rejection related symptom, and an acute vascular rejection response related symptom. In an aspect, when an organ, tissue, transfusion product or cell from the triple transgenic pig is transplanted into a human, thrombocytopenia is decreased as compared to when an organ, tissue, transfusion product or cell from a wild-type pig is transplanted into a human. In an aspect, when a liver from the triple transgenic pig is exposed to human platelets, the liver exhibits reduced uptake of human platelets as compared to when a liver from a wild-type pig is exposed to human platelets. In an aspect, when a kidney from the triple transgenic pig is transplanted into a human, a rejection related symptom is decreased as compared to when a kidney from a wild-type pig is transplanted into a human.

In an embodiment, a skin related product obtained from the triple transgenic pig is provided, wherein said skin related product is for transplantation into a human, said disruption of the α(1,3)-galactosyltransferase, CMAH and β4GalNT2 genes reduces the level of αGal, Sd^{a}-like and Neu5Gc antigens in the skin related product, and the skin related product exhibits reduced premature separation from a wound, particularly from a human skin wound.

Methods of preparing transplant material for xenotransplantation into a human are provided. The methods comprise providing a triple transgenic pig of the invention as a source of the transplant material and wherein the transplant material is selected from the group consisting of organs, tissues, transfusion products and cells and wherein the transplant material has reduced levels of αGal, Sd^{a}-like antigens and Neu5Gc antigens.

Triple transgenic pigs comprising a disrupted α(1,3)-galactosyltransferase, CMAH and β1,4GalNT2 gene in the nuclear genome of cells of the pig are provided as defined in claim 1. In an embodiment the disruption of the α(1,3)-galactosyltransferase gene is selected from the group of disruptions including but not limited to a three base pair deletion adjacent to a G to A substitution, a single base pair deletion, a single base pair insertion, a two base pair insertion, a six base pair deletion, a ten base pair deletion, a seven base pair deletion, an eight base pair insertion for a five base pair deletion, a five base pair insertion, an eleven base pair deletion, and an eighteen base pair deletion; the disruption of the CMAH gene is selected from the group of disruptions including but not limited to a four base pair insertion, a one base pair deletion, a two base pair deletion, a three base pair deletion, a four base pair insertion, a five base pair deletion, an eight base pair deletion, an eleven base pair deletion, a twelve base pair deletion, a single base pair insertion, a two base pair insertion for a single base pair deletion, a twelve base pair insertion for a sixty-six base pair deletion, a a four base pair insertion for a three base pair deletion; and a five base pair deletion/one base pair substitution, and the disruption of the β4GalNT2 gene is selected from the group of disruptions including but not limited to a one base pair insertion, a twelve base pair deletion/one base pair substitution, a twelve base pair deletion, a fourteen base pair deletion, a five base pair deletion and a 271 base pair deletion/1 base pair insertion. In an embodiment, the disruption of the α(1,3)-galactosyltransferase gene is selected from the group of disruptions including a five base pair deletion and a seven base pair deletion, the disruption of the CMAH gene is selected from the group of disruptions including a twelve base pair deletion and a three base pair deletion/four base pair insertion, the disruption of the β4GalNT2 gene is selected from the group of disruptions including a one base pair insertion, a twelve base pair deletion and a five base pair deletion. In an embodiment, the disruption of the α(1,3)-galactosyltransferasegene is selected from the group of disruptions including an eleven base pair deletion and an eighteen base pair deletion, the disruption of the CMAH gene is selected from the group of disruptions including a sixty-six base pair deletion/twelve base pair insertion and a five base pair deletion/one base pair substitution and the disruption of the β4GalNT2 gene is selected from the group of disruptions including a fourteen base pair deletion, a twelve base pair deletion/one base pair substitution, and a 271 base pair deletion/1 base pair insertion. Expression of functional α(1,3)-galactosyltransferase, CMAH and β4GalNT2 in the triple transgenic pig is decreased as compared to a wild-type pig. When tissue from the triple transgenic pig is transplanted into a human, a hyperacute rejection related syndrome is improved as compared to when tissue from a wild-type pig is transplanted into a human.

An organ for use in methods of increasing the duration of the period between when a human subject is identified as a subject in need of a human organ transplant and when the human organ transplant occurs is provided. The methods involve providing an organ from the triple transgenic pig, wherein expression of α(1,3)-galactosyltransferase, CMAH and β1,4 N-acetylgalactosaminyltransferase are decreased as compared to a wild-type pig and surgically attaching the organ from the triple transgenic pig to the human subject in a therapeutically effective manner. In an aspect, the organ is surgically attached internal to the human subject. In an aspect, the organ is surgically attached external to the human subject. The organ may be directly or indirectly attached to the subject.

A liver for use in methods of increasing the duration of the period between when a human subject is identified as a subject in need of a human liver transplant and when the human liver transplant occurs is provided. The methods involve providing the liver from the triple transgenic pig, wherein expression of α(1,3)-galactosyltransferase, CMAH and β1,4 N-acetylgalactosaminyltransferase are decreased as compared to a wild-type pig and surgically attaching the liver from the triple transgenic pig to the human subject in a therapeutically effective manner. In an aspect, the liver is surgically attached internal to the human subject. In an aspect, the liver is surgically attached external to the human subject. The liver may be directly or indirectly attached to the subject.

The kidney of the triple transgenic pig can be used in methods of increasing the duration of the period between when a human subject is identified as a subject in need of a human kidney transplant and when the human kidney transplant occurs . The methods involve providing a kidney from a triple transgenic pig comprising disrupted α(1,3)-galactosyltransferase, CMAH and β4GalNT2 genes wherein expression of α(1,3)-galactosyltransferase, CMAH and β1,4 N-acetylgalactosaminyltransferase are decreased as compared to a wild-type pig and surgically attaching a kidney from the triple transgenic pig to the human subject in a therapeutically effective manner. In an aspect, the kidney is surgically attached internal to the human subject. In an aspect, the kidney is surgically attached external to the human subject. The kidney may be directly or indirectly attached to the subject.

The skin related product of the triple transgenic pig can be used in methods of reducing premature separation of a skin related product from a human subject . The methods involve the steps of providing a triple transgenic pig comprising disrupted α(1,3)-galactosyltransferase, CMAH and β4GalNT2 genes and preparing a skin related product from the triple transgenic pig. Expression of α(1,3)-galactosyltransferase, CMAH and β4GalNT2 in the triple transgenic pig is decreased as compared to a wild-type pig.

A porcine transplant material isolated from the triple transgenic pig for use in methods of improving a hyperacute rejection related symptom in a patient is provided. The methods involve transplanting porcine transplant material having a reduced level of αGal antigens, Sd^{a}-like antigens, and Neu5Gc antigens into a subject. A hyperacute rejection related symptom is improved as compared to when porcine transplant material from a wild-type pig is transplanted into a human.

A cell culture reagent that exhibits an altered epitope profile is provided as defined in the claims. The cell culture reagent is selected from the group comprising cell culture media, cell culture serum, cell culture additives and isolated cells capable of proliferation. In an aspect, the cell culture reagent is isolated from a triple transgenic pig wherein the disruption of the α(1,3)-galactosyltransferase gene is selected from the group of disruptions consisting of a five base pair deletion, a seven base pair deletion or a single base pair insertion at the indicated position, the disruption of the CMAH gene is selected from the group of disruptions consisting of a twelve base pair deletion, a three base pair deletion/four base pair insertion, a seven base pair deletion and an eleven base pair deletion, and the disruption of the β4GalNT2 gene is selected from a single base pair insertion at the indicated site, a twelve base pair deletion and a five base pair deletion. In an aspect, the cell culture reagent is isolated from a triple transgenic pig wherein the disruption of the α(1,3)-galactosyltransferasegene is selected from the group of disruptions including an eleven base pair deletion and an eighteen base pair deletion, the disruption of the CMAH gene is selected from the group of disruptions including a sixty-six base pair deletion/twelve base pair insertion and a five base pair deletion/one base pair substitution and the disruption of the β4GalNT2 gene is selected from the group of disruptions including a fourteen base pair deletion, a twelve base pair deletion/1 base pair substitution, and a 271 base pair deletion/1 base pair insertion.

Methods of producing a compound of interest with an altered epitope profile as defined in the claims are provided. The method involves the steps of providing a cell culture reagent that exhibits an altered epitope profile and incubating an isolated cell capable of expressing the compound of interest with the cell culture reagent that exhibits an altered epitope profile. The cell culture reagent with an altered epitope profile is isolated from a triple transgenic pig comprising disrupted α(1,3)-galactosyltransferase, CMAH and β4GalNT2 genes. Expression of α(1,3)-galactosyltransferase, CMAH and β4GalNT2 in the triple transgenic pig is decreased as compared to a wild-type pig. The level of Neu5Gc, alphaGal and Sd^{a}-like epitopes on the compound of interest is lower than the level of Neu5Gc, alphaGal and Sd^{a}-like epitopes on the compound of interest when the compound of interest is produced from an isolated cell incubated with a cell culture reagent isolated from a wild-type pig. In an embodiment the compound of interest is selected from the group comprising glycolipids and glycoproteins. In various aspects, the compound of interest is a glycoprotein selected from the group of glycoproteins comprising antibodies, growth factors, cytokines, hormones and clotting factors. In an embodiment the disruption of the α(1,3)-galactosyltransferase gene is selected from the group of disruptions consisting of a five base pair deletion, a seven base pair deletion or a single base pair insertion at the indicated position, the disruption of the CMAH gene is selected from the group of disruptions consisting of a twelve base pair deletion, a three base pair deletion/four base pair insertion, a seven base pair deletion and an eleven base pair deletion, and the disruption of the β4GalNT2 gene is selected from a single base pair insertion at the indicated site, a twelve base pair deletion and a five base pair deletion. In an embodiment the disruption of the α(1,3)-galactosyltransferase gene is selected from the group of disruptions including an eleven base pair deletion and an eighteen base pair deletion, the disruption of the CMAH gene is selected from the group of disruptions including a sixty-six base pair deletion/twelve base pair insertion and a five base pair deletion/one base pair substitution and the disruption of the β4GalNT2 gene is selected from the group of disruptions including a fourteen base pair deletion, a twelve base pair deletion/one base pair substitution, and a 271 base pair deletion/1 base pair insertion.

Porcine transplant materials for transplantation into a human are provided. Lipids and proteins of the porcine transplant material have a reduced level of αGal epitopes, and the transplant material has reduced level of Neu5Gc and Sd^{a}-like epitopes.

Transgenic pigs comprising a disrupted α1,3-galactosyltransferase, CMAH and β4GalNT2 gene in the nuclear genome of cells of the pig wherein expression of α1,3-galactosyltransferase, CMAH and β4GalNT2 is decreased as compared to a wild-type pig and wherein VVL binding is reduced, are provided.

### BRIEF DESCRIPTION OF THE DRAWINGS

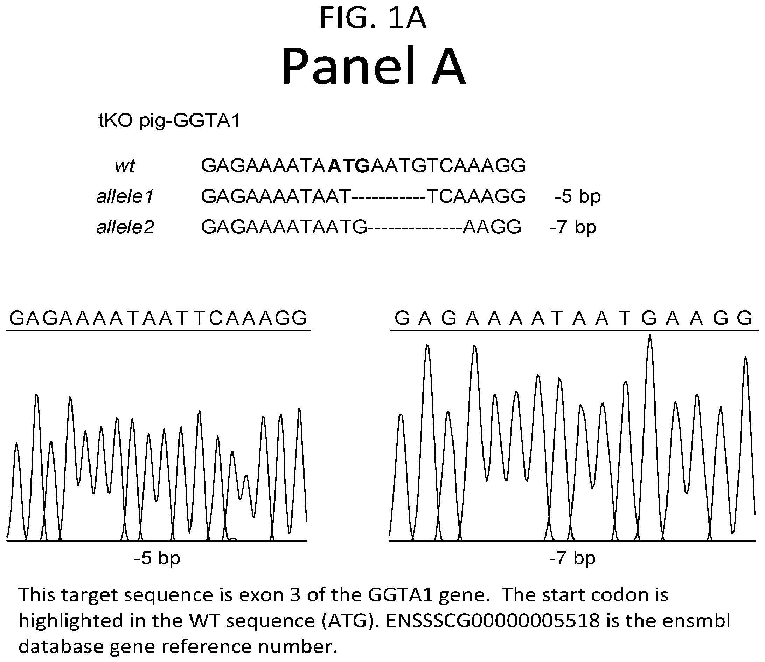

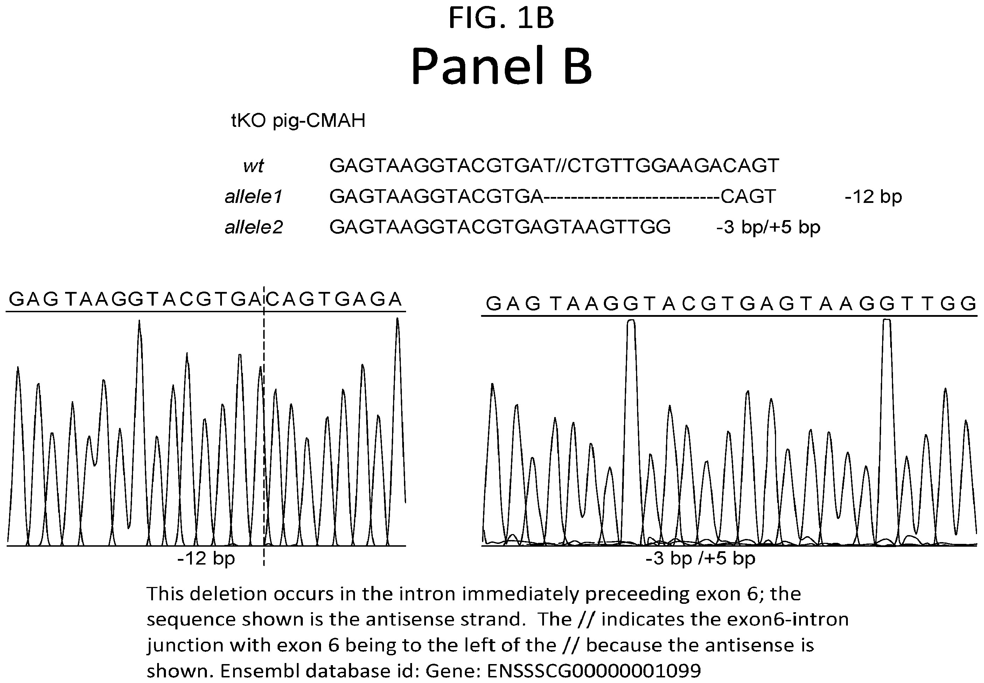

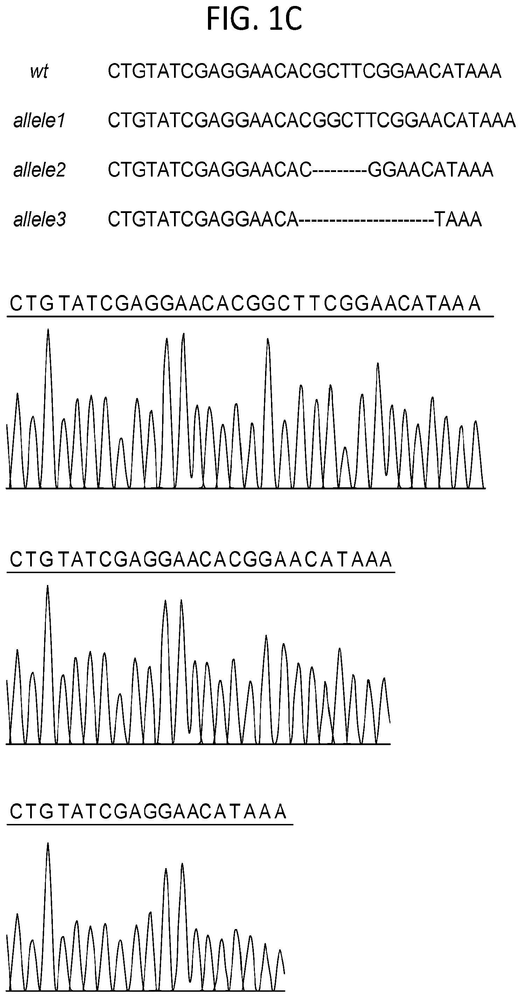

Figure 1 depicts a schematic of the sequence alterations in exemplary knockout pig (pig isolate identifier 47-1). Panel A presents portions of the wild-type (wt) and disrupted α1,3 galactosyltransferase gene (GGTA-1) nucleotide sequences. A portion of the wild-type GGTA-1 nucleotide sequence (WT) is shown in the top line. The same region of the altered GGTA-1 nucleotide sequence from a triple transgenic pig are shown in the two bottom lines each of which provides the altered sequence of one GGTA-1 allele. The disrupted GGTA-1 nucleotide sequences from an exemplary triple transgenic pig are a five base pair and seven base pair deletions. Dashes indicate the portion of the nucleotide sequence where the deleted nucleotides would occur in the wild-type sequence.
Panel B presents portions of wild-type and disrupted CMAH genes for an exemplary triple transgenic pig. A portion of the wildtype (wt) CMAH gene is shown in the top line. The nucleotide sequences of both alleles of the comparable regions from the same triple transgenic pig are shown below the wild type sequence. The disrupted CMAH sequences of the triple transgenic pig include a twelve base pair deletion and a three base pair deletion/four base pair insertion. Dashes indicate the region of the nucleotide sequence in which the deletions occurred.
Panel C presents portions of wild-type and disrupted β4GalNT2 genes for an exemplary triple transgenic pig. A portion of the wildtype (wt) β4GalNT2 gene is shown in the top line. The nucleotide sequences of the comparable regions from the same exemplary triple transgenic pig are shown below the wild type sequence. As shown, the disrupted β4GalNT2 sequences of the triple transgenic pig include a single base pair insertion, a twelve base pair deletion and a five base pair deletion. Unlike the GGTA1 and CMAH sequences, three different alleles were detected. It is possible that the β4GalNT2 sequence occurs at more than one distinct locus in the porcine genome. Dashes indicate the region of the nucleotide sequence in which the β4GalNT2 deletions occurred.

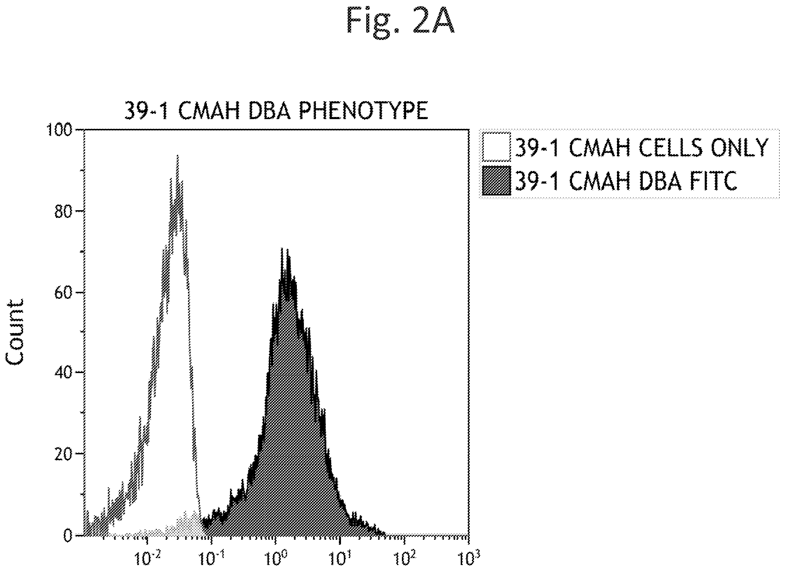

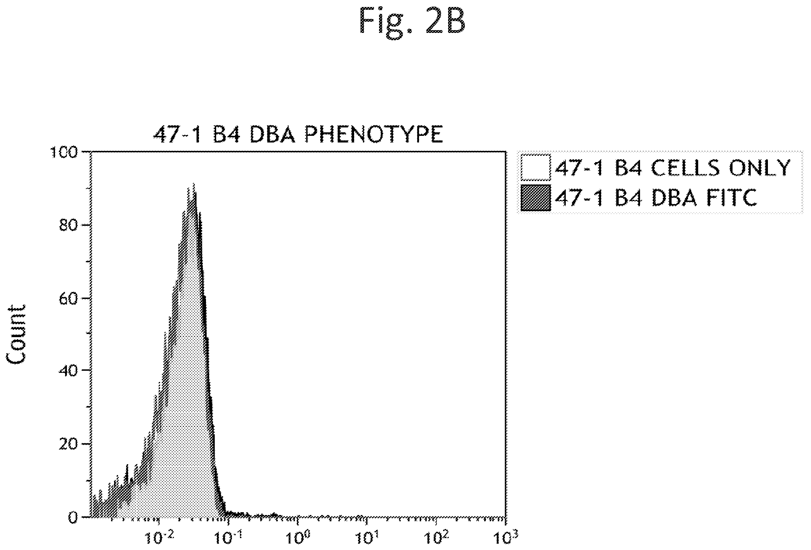

Figure 2 depicts a graph of flow cytometry data obtained from peripheral blood monocyte cells (PBMC) from either the CMAH/αGal double transgenic pig (CMAH, upper panel) or the CMAH/αGal/β4GalNT2 triple transgenic pig (B4, lower panel). Cells were either unstained (cells only, white curves) or stained with *Dolichus biflorus* agglutinin conjugated with FITC (DBA-FITC, solid black curve). DBA binds *α*-*N*-acetylgalactosamine bearing glycans or Sd^{a}-like glycans produced by β4GalNT2. In the upper panel the profile of PBMC's from double knockout (CMAH/αGal) pigs with wildtype β4GalNT2 sequence (CMAH) after incubation with DBA (black curve) is distinctly shifted from the profile of unstained cells from the double transgenic CMAH/αGal pig (white curve). In the lower panel the profile of PMBC from triple transgenic pigs with disrupted β4GalNT2 sequences (B4) after incubation with DBA predominantly overlies the profile from the unstained cells from the triple transgenic CMAH/αGal/β4GalNT2 pig (regions of white and black curves that overlay are represented as gray). The limited change in the profile of DBA-FITC treated cells indicates a lack of DBA binding to the PBMC's from the triple transgenic pig and reduced occurrence of Sd^{a}-like antigens on cells from the triple transgenic pig.

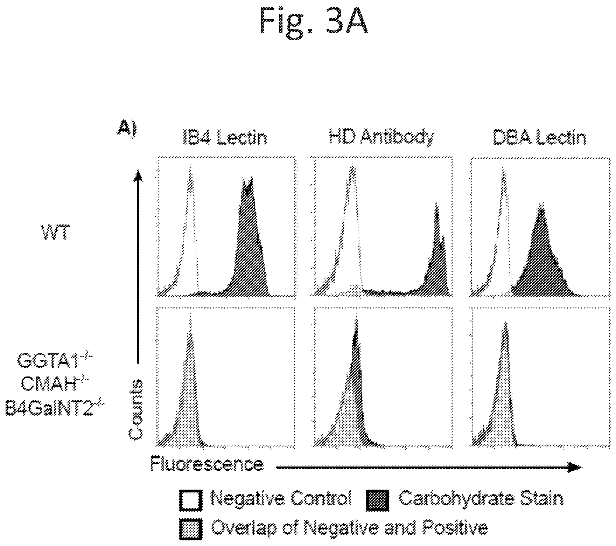

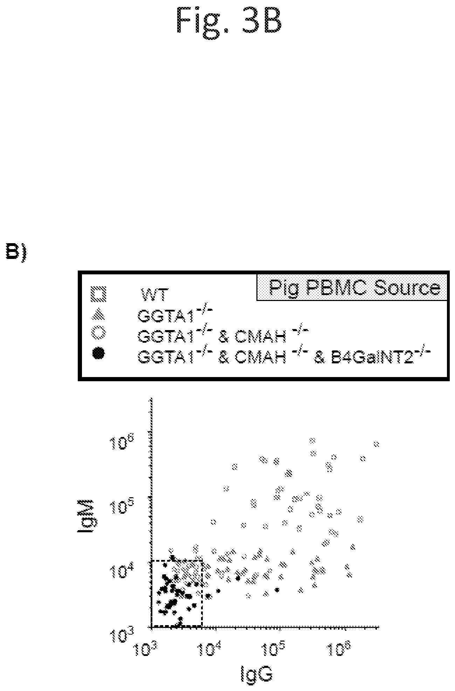

Figure 3 presents results epitope analysis of PBMC's from a variety of pigs. Panel A presents flow cytometry results of experiments performed with PBMC's from wildtype (WT) and triple transgenic (GGTA1-/-, CMAH-/-, B4GalNT2-/-) pigs. Cell counts are shown on the y axis; fluorescence is shown on the x-axis.

The far left graphs depict data from a negative control (white) and cells incubated with IB4 lectin (dark). IB4 interacts with the alpha galactose linked carbohydrates created by the gene product of GGTA1. Curves with overlap between the control (negative) and experimental (positive) results are shown with gray. Wildtype cells incubated with IB4 show a distinct separate peak from unstained wildtype cells. Cells from the triple transgenic pig incubated with IB4 show no significant second peak, indicative of significantly reduced αGal linked carbohydrates.

The center graphs depict data from a negative control (white) and cells incubated with HD antibody (dark). The negative control was an irrelevant isotype control antibody. The HD antibody interacts with the Neu5Gc carbohydrate produced by the product of the CMAH gene. Curves with overlap between the control (negative) and experimental (positive) results are shown with gray. Wildtype cells incubated with HD antibody show a distinct separate peak from wildtype cells treated with an unrelated antibody. Cells from the triple transgenic pig incubated with HD antibody show no significant second peak, indicative of significantly reduced Neu5Gc epitope levels.

The far left graphs depict data from a negative control (white) and cells incubated with DBA lectin (dark). DBA lectin interacts with the carbohydrate structure produced by the gene product of β4GalNT2. Curves with overlap between the control (negative) and experimental (positive) results are shown with gray. Wildtype cells incubated with DBA show a distinct separate peak from unstained wildtype cells. Cells from the triple transgenic pig incubated with DBA show no significant second peak, indicative of significantly reduced carbohydrates produced by the gene product of β4GalNT2.

Panel B presents a scatter graph of results obtained from flow cytometry experiments to evaluate the relative binding of human IgG (X-axis) and IgM (Y-axis) in serum from 44 unique humans to PBMCs from various pig types. Results with PBMC from wildtype (WT) pigs are shown with open squares, results from single transgenic αGal disrupted (GGTA1^{-/-}) pigs are shown with triangles, results from double transgenic CMAH/ αGal disrupted (GGTA1^{-/-} and CMAH^{-/-}) pigs are shown with empty circles, and results from triple transgenic CMAH/αGal/B4GalNT2 disrupted (GGTA1^{-/-} and CMAH^{-/-} and β4GalNT2^{-/-}) pigs are shown with solid circles. Results from triple transgenic CMAH/αGal/B4GalNT2 disrupted (GGTA1^{-/-} and CMAH^{-/-} and β4GalNT2^{-/-}) pigs are clustered at low levels of IgM and IgG binding; a few data points indicate moderate IgG binding to triple transgenic cells.

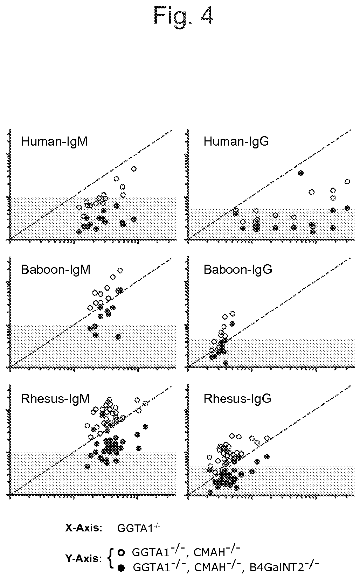

Figure 4 presents a scatter graph of results obtained from flow cytometry experiments to evaluate the relative binding of Rhesus Macaque IgG (X-axis) and IgM (Y-axis) in serum from Rhesus macaques, baboons and humans to PBMCs from various pig types. Results with PBMC from single transgenic αGal disrupted (GGTA1^{-/-}) pigs are shown on the x-axis, results from double transgenic CMAH/ GGTA1^{-/-} disrupted (GGTA1^{-/-} and CMAH^{-/-}) pigs are shown with empty circles, and results from triple transgenic CMAH/ GGTA1^{-/-} /B4GalNT2 disrupted (GGTA1^{-/-} and CMAH^{-/-} and β4GalNT2^{-/-}) pigs are shown with solid circles. CMAH/ GGTA1^{-/-} and CMAH/ GGTA1^{-/-}/β4GalNT2 are on the y-axis. Binding below the line indicates binding to the pig cells on the x axis is more antigenic than the pig cells on the y axis. Human (top), baboon (middle) and rhesus monkey (bottom) sera were tested with the pig cells. IgM results are shown on the left; IgG results are shown on the right.

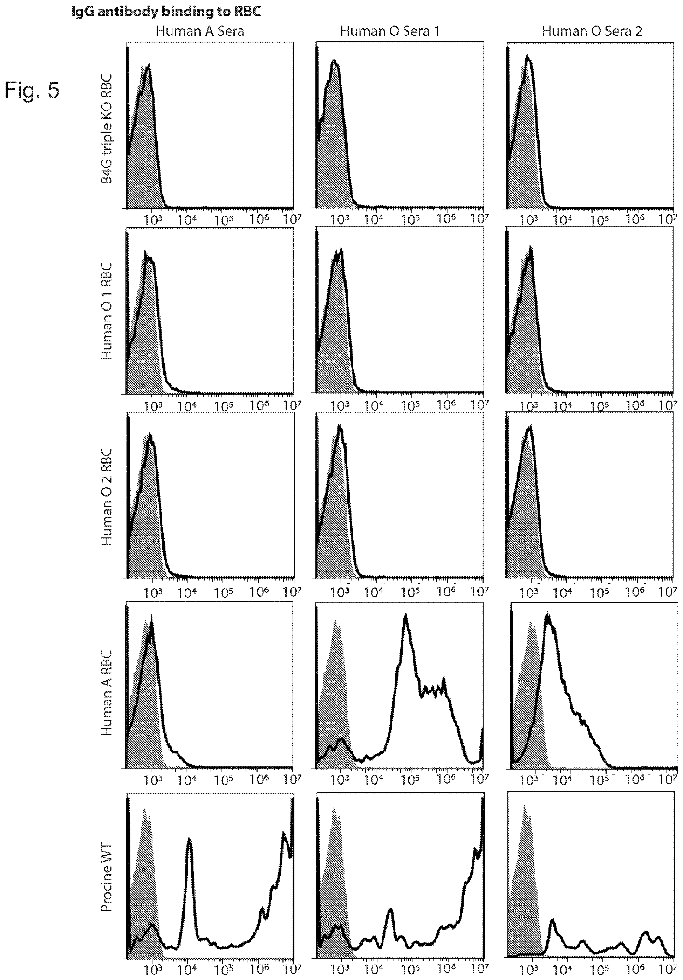

Figure 5 provides series of flow cytometry results indicating the level of IgG antibody binding to red blood cells (RBC). Multiple types of RBC were evaluated against three human sera. Results from human A sera are in the left column, human O sera 1 in the middle column and human O sera 2 in the right column. B4Gal/CMAH/Gal triple knockout (B4G triple KO) RBC results are in the top row. Human O 1 RBC results are in the second row. Human O 2 RBC results are in the third row. Human A RBC results are in the fourth row. Porcine wildtype RBC are in the bottom row. Multiple significant peaks are present when wildtype porcine RBC is evaluated with human A and human O sera 1; multiple minor peaks are present when wildtype porcine RBC is evaluated with human O sera 2. In the trace of human A RBC and two human O sera, there are significant peaks. However, the human A RBC and human A sera show only one overlapping peak. As expected both human O RBC show only a single overlapping peak when tested against all three sera. The B4Gal/CMAH/Gal triple knockout RBC show only a single overlapping peak when tested against all three sera, similar to human O RBC.

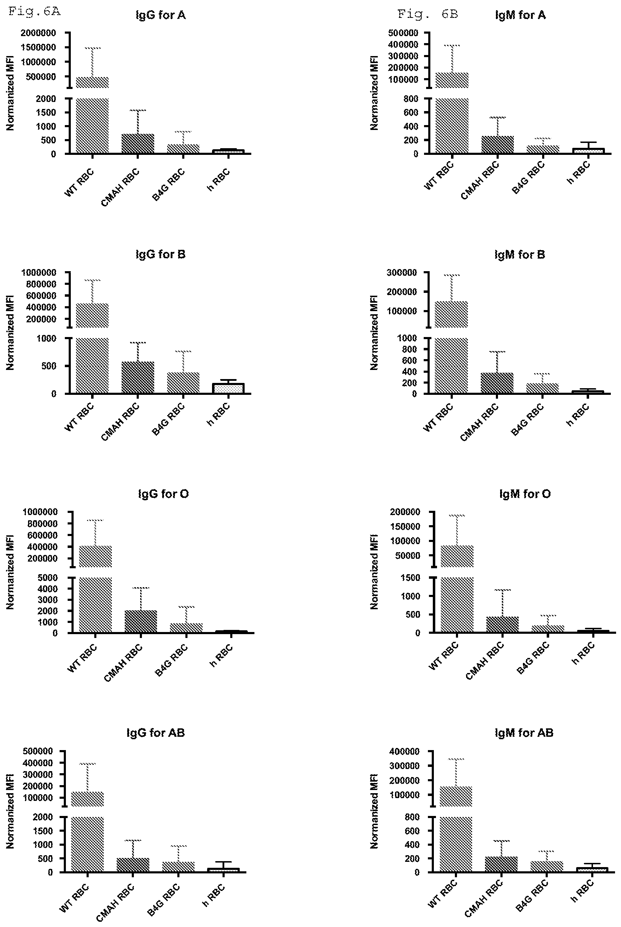

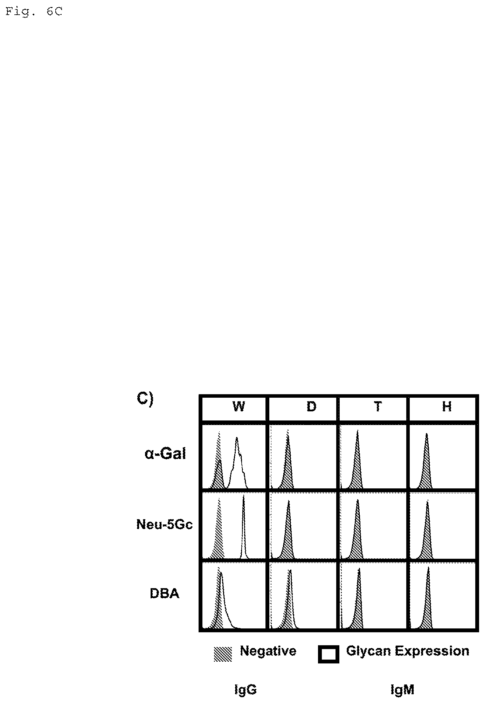

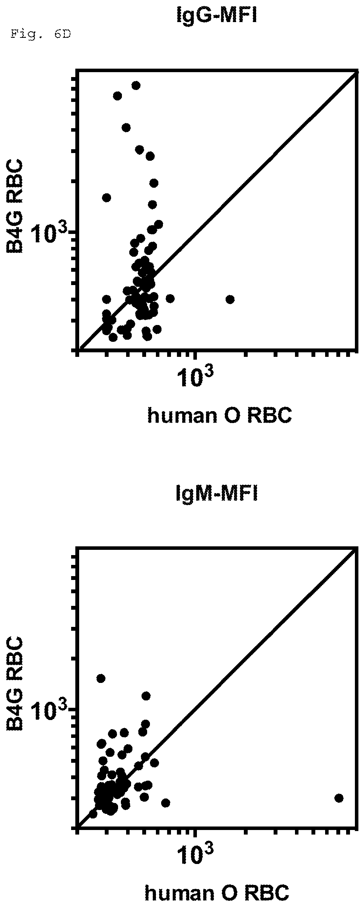

Figure 6 provides a summary of data obtained from flow cytometry comparisons of human antibody binding to various swine and human RBC. Sera from 74 humans were incubated with pig and human RBC. Data from wildtype swine RBC (W), animals lacking GGTA1 and CMAH (CMAH RBC), or GGTA1/CMAH/β4GalNT2 (B4G RBC) and human allogeneic RBC (h) are shown. Panel A shows a summary of IgG binding to various RBC. The data represents a normalized median fluorescence intensity (MFI) and standard deviation for IgG. Human A, B, O and AB sera were utilized. Intergroup comparisons of antibody binding were performed using repeated measures one way ANOVA and Tukey's multiple comparison test. Human antibody binding to the various RBC was maximal on wild type cells and diminished as each glycan producing gene was inactivated. The triple knockout-RBC (B4G) most closely approximated the levels of antibody binding seen on human allo-RBC (h). Panel B shows a summary of IgM binding to various RBC. The data represents a normalized median fluorescence intensity (MFI) and standard deviation for IgM. The trend of wildtype>double knockout>triple knockout ≈ human was observed for both IgG and IgM. In Panel C, flow cytometry was used to reveal the effects of inactivating the GGTA1, CMAH and β4GalNT2 genes on the expression of the α-Gal, Neu5Gc and DBA-reactive glycans. Results from porcine wildtype (W) red blood cells, CMAH/αGal double knockout (D) red blood cells, CMAH/αGal/βGalNT2 triple transgenic red blood cells (T) and human blood group O red blood cells are shown in the figure. Flow cytometry results indicate loss of antigenic structures with each gene disruption. In Panel D, the data were plotted to display the individual MFI of each human serum sample when examining antibody binding to human O RBC on the x-axis and the individual MFI of each human serum sample when examining antibody binding to triple knockout swine RBC (B4G) on the y-axis. Data points falling below the diagonal line on the graphs represent less binding to the pig triple knockout cells than to human blood group O cells. Human group O blood is considered a universal donor; transfused human group O RBC undergo limited humoral destruction.

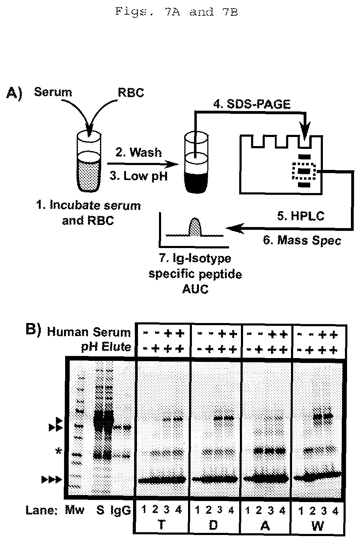

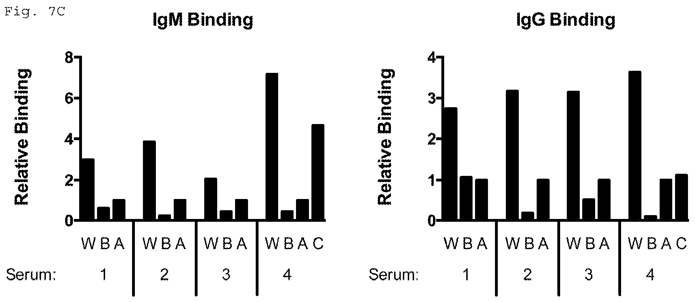

Figure 7 provides results of immunoglobulin analysis using quantitative mass spectrometry to measure the abundance of individual antibody isotypes. Panel 7A is a schema describing the biochemical approach used to evaluate the binding of IgG and IgM to RBC. RBC from wildtype swine (W), GGTA1/CMAH deficient swine (D), GGTA/CMAH/β4GalNT2 swine (T) and autologous human RBC (H) were evaluated. The details of the process are described elsewhere herein. A representative gel showing material eluted from RBC is shown in Panel B. Molecular weight markers (Mw), crude starting serum (S) and purified human IgG were loaded for comparison. Other controls included untreated RBC (lane 1 all samples), and RBC that were acid washed but not incubated with serum (lane 2 all samples). Material was stripped from serum-treated RBC by low pH incubation for either 2 or 3 minutes (lanes 3 and 4 respectively, all samples). The single arrowhead marks a protein migrating with a size similar to albumin. Although autologous human RBC released less of this protein than the swine cells, this result was not reproduced in other experiments. Double arrowheads highlight a protein migrating with a size similar to IgG (lanes 3 and 4). The intensity of the IgG heavy chain sized band varied between the different RBC. Incubating cells at low pH for either two or three minutes released similar protein levels. To quantitate the amount of antibody released from the cells, gel slices encompassing the approximate size of immunoglobulin heavy chain in the two minute elution samples were collected, incubated with trypsin and assessed by mass spectrometry (see below herein). Three arrowheads note a protein that migrates with a size identical to hemoglobin. The presence of hemoglobin in the supernatant of serum-free samples indicated that RBC lysis occurred during the manipulations. Variations between lanes 1 and 2 for all RBC types suggest acid washing accelerated lysis and released increasing amounts of hemoglobin from the cells. Unknown polypeptides, marked by an *, that migrated as a double slightly above the immunoglobulin light chains were released from RBC even in the absence of serum.

Panel C shows the relative levels of IgM and IgG that eluted from each type of RBC after incubation with separate aliquots of the same serum as determined by mass spectroscopy. The AUC from the mass spectrometry analyses were all normalized to the values obtained for the total IgG or IgM binding to the autologous human red blood cells for each serum. Total antibody was calculated for IgG by summing the AUC for each isotype. Results from wildtype pig red blood cells (W), triple knockout pig red blood cells (B), and autologous human red blood cells (A) are shown. Autologous human red blood cells are from the same subject from which the tested serum was obtained.

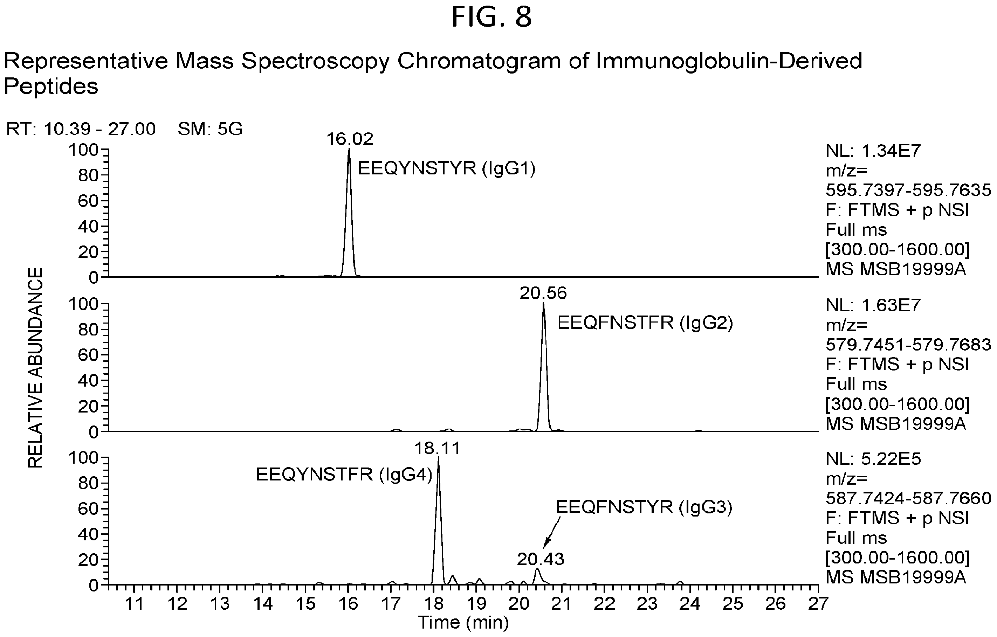

Figure 8 provides representative mass spectroscopy chromatograms of immunoglobulin-derived peptides. Relative abundance is shown on the y-axis. Time in minutes (min) is shown on the x-axis. Chromatograms such as this were used to calculate AUC.

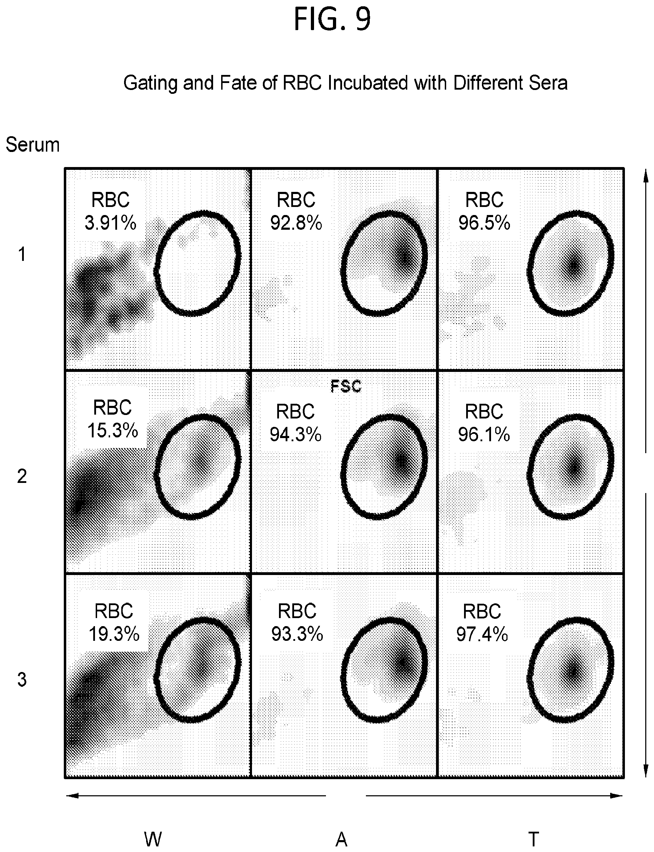

Figure 9 presents flow cytometry analysis gating and fate. Three human sera were incubated with RBC from wildtype pigs (W), autologous human RBC (A) and RBC from GGTA1/CMAH/β4GalNT2 deficient pigs (T). After incubating with fluorescent secondary antibodies to report bound human immunoglobulin, cells were analyzed by flow cytometry. Forward scatter (FSC, x-axis) and side scatter (SSC, y-axis) were used to identify RBC. Black ovals represent gates used to select RBC for analysis. The percentages shown next to each gate represents the fraction of total events that resided within the gate. Human sera disrupted wild type swine RBC as seen by an increase in debris and the reduction in RBC falling in the gated regions. Wildtype RBC high antigenicity and consequent cell destruction may have contributed to the histogram quality of the wildtype (W) samples in some experiments.

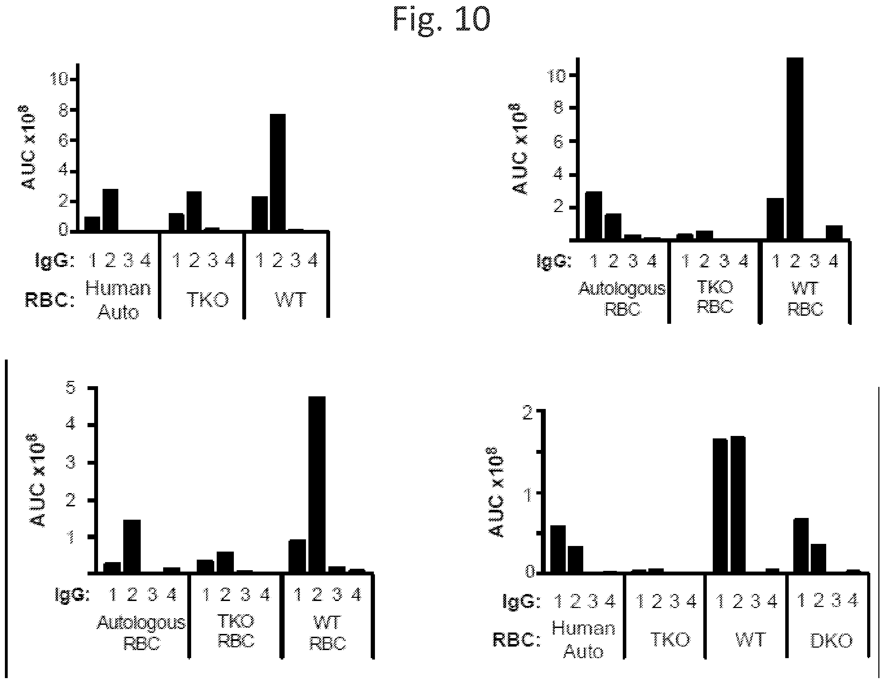

Figure 10 depicts area under the curve (AUC) values obtained for each IgG isotype as determined by mass spectrometry. AUC is shown on the y-axis; IgG isotypes and the RBC type are shown on the x-axis. Results from triple knockout pig red blood cells (TKO), human autologous red blood cells (Human Auto) and wildtype pig red blood cells(WT) are shown.

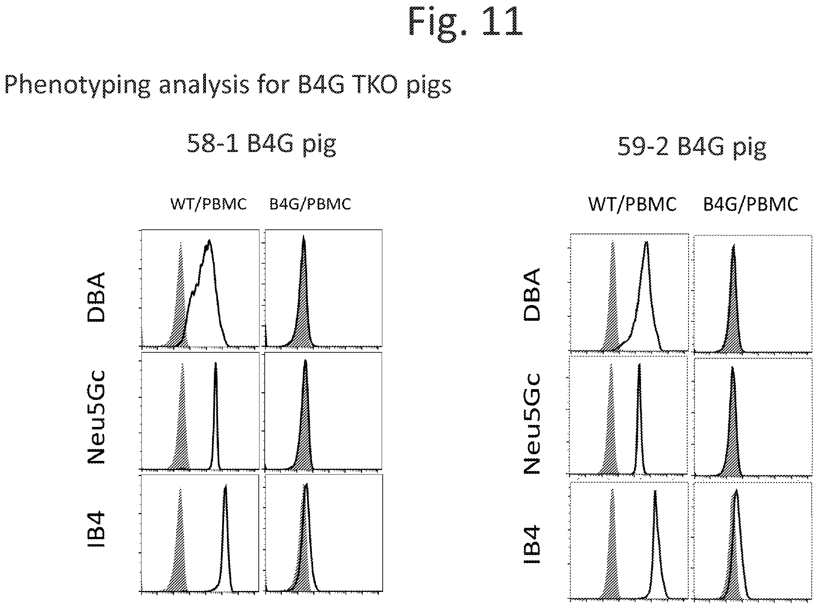

Figure 11 depicts flow cytometry results obtained from peripheral blood monocytes (PBMC) obtained from wildtype (WT) or Gal/CMAH/β4GalNT2 triple knockout pigs (B4G). PBMC from two pig isolates (58-1 and 59-2) are shown. IB4 is bound by αGal; in the presence of reduced αGal expression, IB4 binding is reduced. Neu5Gc is an epitope produced by the CMAH gene product. DBA is a lectin bound by the β4GalNT2 product. The grey histogram shows negative control results. Two peaks are clearly present for the wildtype cells with each of IB4, DBA and Neu5Gc. For both triple knockout pig isolates the second peak is eliminated or shifted to overlap with the negative control, indicating reduced binding.

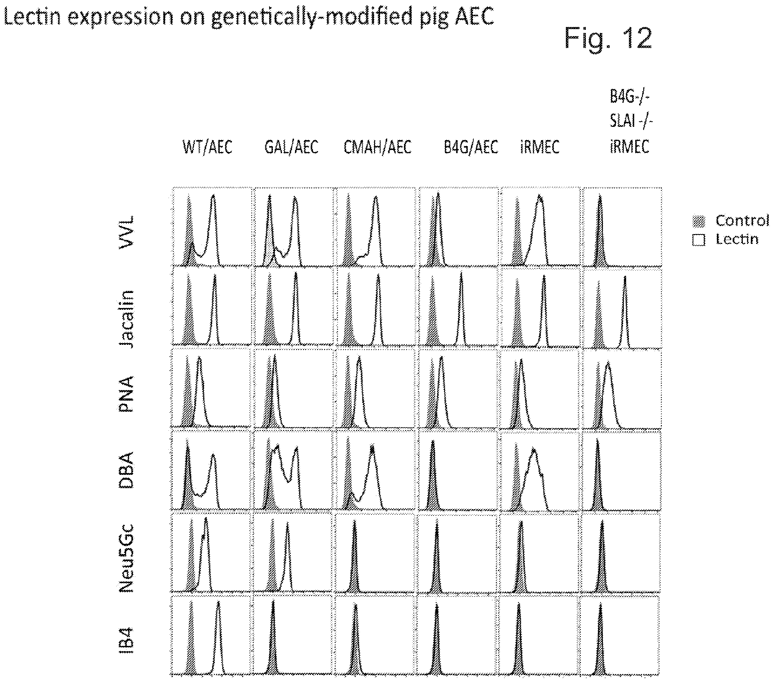

Figure 12 provides flow cytometry results obtained from aortic endothelial cells (AEC) or immortalized renal endothelial cells (iREC) after lectin staining. The indicated cell types were incubated with IB4, Neu5Gc, DBA, PNA, Jacalin or VVL. The left column shows results obtained from wildtype AEC (WT/AEC), the second column shows results obtained from αGal disrupted pigs (GAL/AEC), the middle column shows results obtained from double knockout CMAH/αGal pigs (CMAH/AEC), the fourth column shows results obtained from triple knockout CMAH/αGal/β4GalNT2 pigs (B4G/AEC), the fifth column shows results obtained from wildtype immortalized renal endothelial cells, the sixth column shows results obtained from GGTA1/CMAH/β4GalNT2 and SLA antigen disrupted immortalized renal endothelial cells. Gray histograms are no lectin negative controls. The solid black line represents lectin binding. Note the absence of a second IB4 peak in Gal disrupted cells, the absence of a second Neu5Gc peak in CMAH disrupted cells and the absence of a second DBA peak in β4GalNT2 disrupted cells. Second PNA and Jacalin peaks occur in cells from the single, double and triple knockout pigs. In the triple knockout AEC and the β4GalNT2/SLA triple knockout, Sla antigen disrupted iRMEC, the second VVL peak shifts substantially. While not being bound by mechanism, reduced VVL lectin recognition of an antigen on the triple knockout cells may contribute to the surprising results obtained from triple knockout CMAH/αGal/β4GalNT2 cells and pigs.

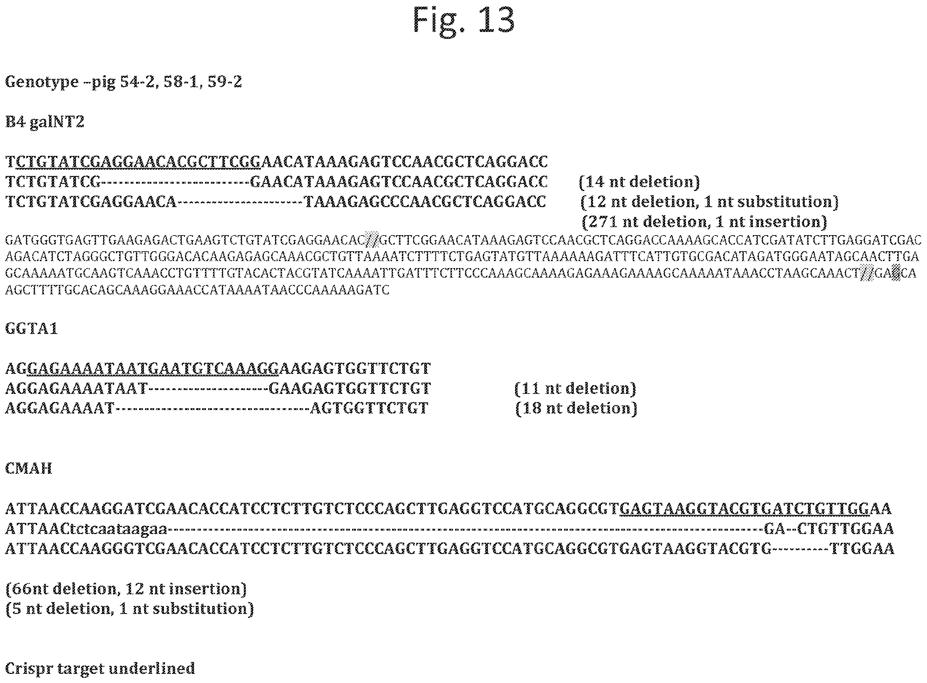

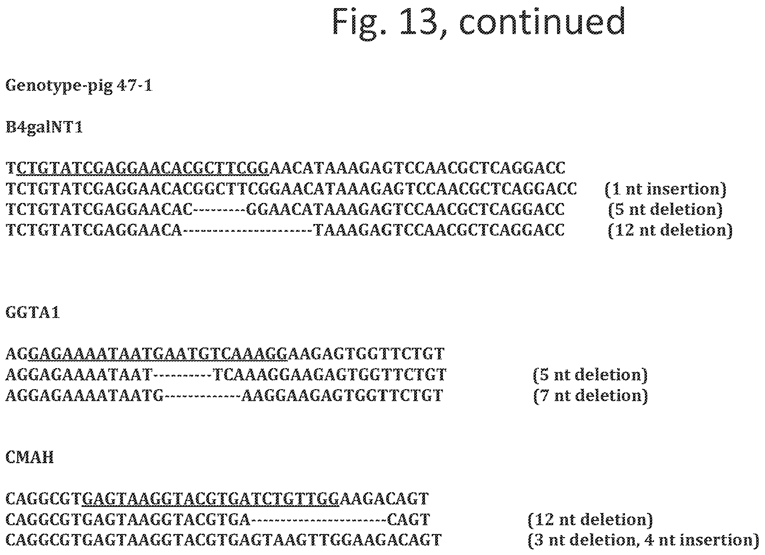

Figure 13 presents portions of wild-type and disrupted β4GalNT2, GGTA1 and CMAH genes in exemplary triple transgenic pigs. The underlined portion of each wild-type sequence indicates the Crispr target region. The figure shows sequence disruptions from multiple triple transgenic pigs (pig isolate identifiers 54-2, 58-1 and 59-2). Three β4GalNT2 variations are shown, a 14 nucleotide deletion, a 12 nucleotide deletion/1 nucleotide substitution and a 271 nucleotide deletion/1 nucleotide insertion. The third β4GalNT2 mutation depicted is a 271 nucleotide deletion/1 nucleotide insertion wherein double slashes (//) demark the deletion. Two αGal (GGTA1) variations are shown, an 11 nucleotide (nt) deletion and an 18 nucleotide (nt) deletion. Two CMAH variations are shown, a 66 nucleotide deletion/ 12 nucleotide insertion and a 5 nucleotide deletion/1 nucleotide substitution.

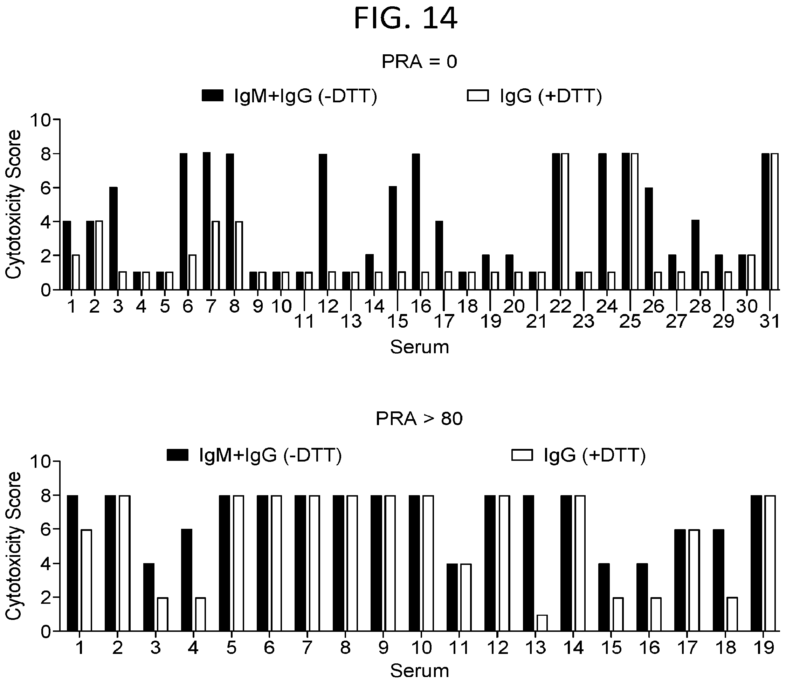

Figure 14 presents data obtained from triple transgenic cells from triple transgenic pigs (αGal/B4GalNT2/CMAH deficient) pigs evaluated with multiple human sera in a human clinical crossmatch test. The upper graph shows results from 31 human sera with a PRA=0; the lower graph shows results from 19 human sera with a PRA>80. IgM and IgG results in the absence of DTT are indicated with a solid bar; IgG results after treatment with DTT are indicated by an empty bar. The cytotoxicity score is shown on the y-axis. Cytotoxicity scores of 1 are very good candidates for allotransplant.

### DETAILED DESCRIPTION OF THE INVENTION

The present application provides transgenic pigs and porcine organs, tissues and cells for transplantation into a human that do not express the indicated pig genome encoded products and methods of making and using the same. In one embodiment the application provides a triple transgenic pig as defined in the claims.

### I. In General

In the specification and in the claims, the terms "including" and "comprising" are open-ended terms and should be interpreted to mean "including, but not limited to..." These terms encompass the more restrictive terms "consisting essentially of" and "consisting of".

As used herein and in the appended claims, the singular forms "a", "an", and "the" include plural reference unless the context clearly dictates otherwise. As well, the terms "a" (or "an"), "one or more" and "at least one" can be used interchangeably herein. It is also to be noted that the terms "comprising", "including", and "having" can be used interchangeably.

Unless defined otherwise, all technical and scientific terms used herein have the same meanings as commonly understood by one of ordinary skill in the art to which this invention belongs.

All references cited in this specification are to be taken as indicative of the level of skill in the art. Nothing herein is to be construed as an admission that the invention is not entitled to antedate such disclosure by virtue of prior invention.

### II. COMPOSITIONS AND METHODS

Transgenic pigs suitable for use in xenotransplantation and methods of preparing transplant material suitable for use in xenotransplantation into a human are provided. Specifically, the present application describes the production of triple transgenic pigs with decreased expression of alpha 1,3 galactosyltransferase (αGal), β 1,4 N-acetylgalactosaminyltransferase (β4GalNT2) and cytidine monophosphate-N-acetylneuraminic acid hydroxylase (CMAH).

In embodiments of the present invention, pigs and porcine organs, tissues and cells therefrom are provided in which the αGal, β4GalNT2 and CMAH genes are less active, such that the resultant αGal, β4GalNT2 and CMAH products no longer generate wild-type levels of α1,3-galactosyl epitopes, Sd^{a}-like epitopes, or Neu5Gc on a cell surface, glycoprotein or glycolipid. In an alternative embodiment the αGal, β4GalNT2 and CMAH genes are inactivated in such a way that no transcription of the gene occurs. In various embodiments triple αGal/B4GalNT2/CMAH knockout pigs were made. Methods of making transgenic pigs, and the challenges thereto, are discussed in Galli et al 2010 Xenotransplantation 17(6) p.397-410. Methods and cell cultures of the invention are further detailed below herein.

The term "transgenic pig " refers to a transgenic pig wherein a given gene has been altered, removed or disrupted. It is to be emphasized that the term is to be intended to include all progeny generations. Thus, the founder animal and all F1, F2, F3 and so on progeny thereof are included, regardless of whether progeny were generated by somatic cell nuclear transfer (SCNT) from the founder animal or a progeny animal or by traditional reproductive methods. By "single transgenic" is meant a transgenic pig wherein one gene has been altered, removed or disrupted. By "double transgenic" is meant a transgenic pig wherein two genes have been altered, removed or disrupted. By "triple transgenic" is meant a transgenic pig wherein three genes have been altered, removed or disrupted. By "quadruple transgenic" is meant a transgenic pig wherein four genes have been altered, removed or disrupted.

In principle, transgenic pigs may have one or both copies of the gene sequence of interest disrupted. In the case where only one copy or allele of the nucleic acid sequence of interest is disrupted, the knockout pig is termed a "heterozygous transgenic pig ". The term "null" mutation encompasses both instances in which the two copies of a nucleotide sequence of interest are disrupted differently but for which the disruptions overlap such that some genetic material has been removed from both alleles, and instances in which both alleles of the nucleotide sequence of interest share the same disruption. In various embodiments disruptions of the three genes of interest may occur in at least a plurality of the animal's cells, at least half the animal's cells, at least a majority of animal's cells, at least a supermajority of the animal's cells, at least 70%, 75", 80%, 85%, 90%, 95%, 98%, or 99% of the animal's cells.

The term "chimera", "mosaic" or "chimeric pig "refers to a transgenic pig with a knockout in some of its genome-containing cells. A chimera has at least one cell with an unaltered gene sequence, at least several cells with an unaltered gene sequence or a plurality of cells with an unaltered sequence.

The term "heterozygote" or "heterozygotic pig " refers to a transgenic pig with a disruption on one of a chromosome pair in all of its genome containing cells.

The term "homozygote" or "homozygotic pig " refers to a transgenic pig with a disruption on both members of a chromosome pair in all of its genome containing cells. A "homozygous alteration" refers to an alteration on both members of a chromosome pair.

A "mutation" is a detectable change in the genetic material in the animal that is transmitted to the animal's progeny. A mutation is usually a change in one or more deoxyribonucleotides, such as, for example adding, inserting, deleting, inverting or substituting nucleotides.

By "pig" is intended any pig known to the art including, but not limited to, a wild pig, domestic pig, mini pigs, a *Sus scrofa* pig, a *Sus scrofa domesticus* pig, as well as in-bred pigs. Without limitation the pig can be selected from the group comprising Landrace, Yorkshire, Hampshire, Duroc, Chinese Meishan, Chester White, Berkshire Goettingen, Landrace/York/Chester White, Yucatan, Bama Xiang Zhu, Wuzhishan, Xi Shuang Banna and Pietrain pigs. Porcine organs, tissues, cells or transfusion products are organs, tissues, devitalized animal tissues, cells or transfusion products from a pig.

The alpha 1,3 galactosyltransferase (αGal, GGTA, GGT1, GT, αGT, GGTA1, GGTA-1) gene encodes an enzyme (GT, αGal, α1,3 galactosyltransferase). Ensemble transcript ENSSSCG00000005518 includes the porcine GGTA1 nucleotide sequence. Functional α1,3 galactosyltransferase catalyzes formation of galactose-α1,3-galactose (αGal,Gal,Gal, gal1,3gal, gal1-3gal) residues on glycoproteins. The galactose-α1,3-galactose (αGal) residue is an antigenic epitope or antigen recognized by the human immunological system. Removing αGal from transgenic organ material does not eliminate the human immunological response to transplant of foreign material, suggesting an involvement of additional antibodies in the rapid immunological response to xenotransplant. (Mohiudden et al (2014), Am J. Transplantation 14:488-489 and Mohiudden et al 2014 Xenotransplantation 21:35-45). Disruptions of the αGal gene that result in decreased expression of functional αGal may include but are not limited to a 3 base pair deletion adjacent to a G to A substitution, a single base pair deletion, a single base pair insertion, a two base pair insertion, a six base pair deletion, a ten base pair deletion, a seven base pair deletion, an eight base pair insertions for a five base pair deletion, a five base pair insertion, an eleven base pair deletion, and an eighteen base pair deletion (see Table 1). The Crispr target sequence is in exon 3 of the gene, near the start codon.

The cytidine monophosphate-N-acetylneuraminic acid hydroxylase (CMP-Neu5Ac hydroxylase gene, CMAH) gene encodes an enzyme (CMAH). Functional CMAH catalyzes conversion of sialic acid N-acetylneuraminic acid (Neu5Ac) to N-glycolylneuraminic acid (Neu5Gc). The Neu5Gc residue is an antigenic epitope or antigen recognized by the human immunological system. The Ensembl database id Gene: ENSSSCG00000001099 includes the porcine CMAH nucleotide sequence, and the Crispr target area is near exon 6. Disruptions of the CMAH gene that result in decreased expression of functional CMAH may include but are not limited to a four base pair insertion, a one base pair deletion, a two base pair deletion, a three base pair deletion, a twenty base pair deletion, a five base pair deletion, an eight base pair deletion, an eleven base pair deletion, a twelve base pair deletion, a single base pair insertion, a two base pair insertion for single base pair deletion, a three base pair deletion for a four base pair insertion, a sixty-six base pair deletion/twelve base pair insertion, and a five base pair deletion/1 base pair substitution(see Table 1).

The β1,4 N-acetylgalactosaminyltransferase (B4GalNT2, β4GalNT2, B1,4GalNT2, β1,4GalNT2) gene encodes the β1,4 N-acetylgalactosaminyltransferase 2 glycosyltransferase (B4GalNT2). Functional B4GalNT2 produces Sd^{a}-like glycans (Dall'Olio et al (2014) Biochemica Biophysica Acta 1840:443-453 and Blanchard et al (1983) JBC 258:7691-7685). B4GalNT2 is thought to be expressed in most humans; prior work suggested that only 5% of humans lack expression of functional B4GalNT2. Disruption of β4GalNT2 in pig cells significantly decreases crossmatching in more than 5% of the human samples tested; this result was unexpected. Sd^{a} -like glycans may include Sd^{a} and similar glycans related to blood type or blood group determination and gastro-intestinal cancer inhibition. The Ensembl database ENSSSCG00000030269 entry includes the β4GalNT2 cDNA and amino acid sequences. Genomic porcine β4GalNT2 spans multiple exons across approximately 40,000 base pairs and may occur at multiple loci. The CrispR target region utilized in these experiment is found in exon 2 and is CTGTATCGAGGAACACGCTT. Disruptions of the β4GalNT2 gene that result in decreased expression of functional β4GalNT2 may include, but are not limited to, a one base pair insertion, a twelve base pair deletion, a five base pair deletion, a fourteen base pair deletion, a twelve base pair deletion/one base pair substitution, a 271 base pair deletion/one base pair insertion.

**Table 1. Examples of Disruptions of Genes of Interest in Viable Pigs with Decreased Functional Gene Product**

| wildtype | disruption | Disruption Class Descriptor |
|---|---|---|
| GGTA1-SEQ ID NO:1 GTCATCTTTTACATCATGGTGGAT GATATCTCCAGGATGCC | GGTA1- GTCATCTTTTACATCATG_ _ _AAT GATATCTCCAGGATGCC | 3 base pair deletion adjacent to a G to A substitution |
| GGTA1- CTTTTCCCAG GAGAAAATAAT GAATGTCAAA GGAAGAGTGG TTCT | GGTA1- CTTTTCCCAG GAGAAAATAAT GAATGT_AAA GGAAGAGTGG TTCT | Single base pair deletion |
| GGTA1- | GGTA1- | 6 base pair deletion |
| CTTTTCCCAG GAGAAAATAAT GAATGTCAAA GGAAGAGTGG TTCT | CTTTTCCCAG GAGAAAATAAT _ _ _ _ _ _ CAAA GGAAGAGTGG TTCT | |
| GGTA1- CTTTTCCCAG GAGAAAATAAT GAATGTCAAA GGAAGAGTGG TTCT | GGTA1- CTTTTCCCAG GAGAAAATAAT GAATGTATCAAA GGAAGAGTGG TTCT | 2 base pair insertion |
| GGTA1- CTTTTCCCAG GAGAAAATAAT GAATGTCAAA GGAAGAGTGG TTCT | GGTA1- CTTTTCCCAG GAGAAAATAA_ _ _ _ _ _ _ _ _ _ _ A GGAAGAGTGG TTCT | 10 base pair deletion |
| GGTA1- CTTTTCCCAG GAGAAAATAAT GAATGTCAAA GGAAGAGTGG TTCT | GGTA1 CTTTTCCCAG GAGAAAATAAT GAA_ _ _ _  _ _ _ GGAAGAGTGG TTCT | 7 base pair deletion |
| GGTA1- GAGAAAATAAT GAATGTCAAAGG | GGTA1 GAGAAAATAAT G_ _ _ _ _ _ _ AAGG | 7 base pair deletion |
| GGTA1- CTTTTCCCAG GAGAAAATAAT GAATGTCAAA GGAAGAGTGG TTCT | GGTA1 CTTTTCCCAG GAGAAAATAAT GAA GGAATAAT AA GGAAGAGTGG TTCT | 8 base pair substitution for 5 base pair deletion |
| GGTA1- CTTTTCCCAG GAGAAAATAAT GAATGTCAAA GGAAGAGTGG TTCT | GGTA1- CTTTTCCCAG GAGAAAATAAT GAATGTTCAAA GGAAGAGTGG TTCT | Single base pair insertion |
| GGTA1- GAGAAAATAAT GAATGTCAAAGG | GGTA1- GAGAAAATAAT _ _ _ _ _ TCAAAGG | 5 base pair deletion |
| GGTA1- AGGAGAAAATAAT GAATGTCAAAGG AAGAGTGGTTCTGT | GGTA1- AGGAGAAAATAAT---------------- -----GAAGAGTGGTTCTGT | 11 base pair deletion |
| GGTA1- AGGAGAAAATAAT GAATGTCAAAGGAAGAGTGGTTCTGT | GGTA1- AGGAGAAAAT---------------------- ------------AGTGGTTCTGT | 18 base pair deletion |
| CMAH- SEQ ID NO:2 | CMAH- | 4 base pair insertion |
| AAACTCCTGA ACTACAAGGC TCGGCTGGTG AAGGA | AAACTCCTGA ACTACAAGGAA GGC TCGGCTGGTG AAGGA | |
| CMAH- CAGGCGTGAG TAAGGTACGT GATCTGTTGGA AGACAGTGA GATTCAGATGAT | CMAH- CAGGCGTGAG TAAGGTACGT GATC_ _TTGGA AGACAGTGA GATTCAGATGAT | 2 base pair deletion |
| CMAH- CAGGCGTGAG TAAGGTACGT GATCTGTTGGA AGACAGTGA GATTCAGATGAT | CAGGCGTGAG TAAGGTACGT G _ _ _ _ _ _ _ _ GA AGACAGTGA GATTCAGATGAT | 8 base pair deletion |
| CMAH- CAGGCGTGAG TAAGGTACGT GATCTGTTGGA AGACAGTGA GATTCAGATGAT | CAGGCGTGAG TAAGGTACGT G _ _ _ _ _ TTGGA AGACAGTGA GATTCAGATGAT | 5 base pair deletion |
| CMAH- CAGGCGTGAG TAAGGTACGT GATCTGTTGGA AGACAGTGA GATTCAGATGAT | CAGGCGTGAG TAAGGTACGT GA _ _ _ GTTGGA AGACAGTGA GATTCAGATGAT | 3 base pair deletion |
| CMAH- CAGGCGTGAG TAAGGTACGT GATCTGTTGGA AGACAGTGA GATTCAGATGAT | CAGGCGTGAG TAAGGTACGT GATCACGTTGGA AGACAGTGA GATTCAGATGAT | 2 base pair insertion for single base pair deletion |
| CMAH- CAGGCGTGAG TAAGGTACGT GATCTGTTGGA AGACAGTGA GATTCAGATGAT | CAGGCGTGAG TAAGGTACGT GAT _ _ _ _ _ _ _ _ _ _ _ _ _ _ _ _ _ _ _ _ _ TCAGATGAT | 20 base pair deletion |
| CMAH- CAGGCGTGAG TAAGGTACGT GATCTGTTGGA AGACAGTGA GATTCAGATGAT | CAGGCGTGAG TAAGGTACGT GATCTTGTTGGA AGACAGTGA GATTCAGATGAT | 1 base pair insertion |
| CMAH- CAGGCGT GAG TAAGGTACGT GATCTGTTGGA AGACAGTGA GATTCAGATGAT | CAGGCGTGAG TAAGGTACGT GATC_GTTGGA AGACAGTGA GATTCAGATGAT | 1 base pair deletion |
| CMAH GAGTAAGGTACG TGATCTGTTGG AAGACAGT | CMAH GAGTAAGG _ _ _ _ _ _ _ _ _ _ _ _ TTGG AAGACAGT | 11 base pair deletion |
| CMAH GAGTAAGGTACG TGATCTGTTGG AAGACAGT | CMAH GAGTAAGGTACG TGA _ _ _ _ _ _ _ _ _ _ _ _ _ CAGT | 12 base pair deletions |
| CMAH GAGTAAGGTACG TGATCTGTTGGAAGACAGT | CMAH GAGTAAGGTACG TGAGTAAG TTGG AAGACAGT | 3 base pair deletion 4 base pair insertion |
| CMAH ATTAACCAAG GATCGAACAC CATCCTCTTG TCTCCCAGCT TGAGGTCCAT GCAGGCGT GAGTAAGGTACG TGATCTGTTGG AA | CMAH ATTAACtctcaataagaa------------- ---------------------------------------- ---------------------------------------- -------GA--CTGTTGGAA | 66 base pair deletion, 12 base pair insertion |
| CMAH ATTAACCAAG GATCGAACAC CATCCTCTTG TCTCCCAGCT TGAGGTCCAT GCAGGCGT GAGTAAGGTACG TGATCTGTTGG AA | CMAH ATTAACCAAG GgTCGAACAC CATCCTCTTG TCTCCCAGCT TGAGGTCCAT GCAGGCGTGA GTAAGGTACG TG----- TTGGAA | 5 base pair deletion, 1 base pair substitution |
| B4 Gal NT2 SEQ ID NO:3- CTGTATCGAGG AACACGCTTCGGAACATAAA | CTGTATCGAGG AACACG G CTTCGGAACATAAA | 1 base pair insertion |
| B4 Gal NT2 CTGTATCGAGG AACA CGCTTCGGAACATAAA | CTGTATCGAGG AAC A _ _ _ _ _ _ _ _ _ _ _ ______TAAA | 12 base pair deletion |
| B4 Gal NT2 CTGTATCGAGG AACACGCTTCGGAACATAAA | B4 Gal NT2 CTGTATCGAGG AACACG_ _ _ _ _ GAACATAAA | 5 base pair deletion |
| B4 Gal NT2 TCTGTATCGA GGAACACGCT TCGGAACATA AAGAGTCCAA CGCTCAGGACC | B4 Gal NT2 TCTGTATCG- ------------------- ---GAACATA AAGAGTCCAA CGCTCAGGACC | 14 base pair deletion |
| B4 Gal NT2 TCTGTATCGA GGAACACGCT TCGGAACATA AAGAGTCCAA CGCTCAGGACC | B4 Gal NT2 TCTGTATCGA GGAACA------------------- ---------------------------TA AAGAGCCCAA CGCTCAGGACC | 12 base pair deletion, 1 base pair substitution |
| B4 Gal NT2 SEQ ID NO:4 | B4 Gal NT2 | 271 nt deletion, 1 nt |
| GATGGGTG AGTTGAAG AGACTGAA GTCTGTATC GAGGAACAC //GCTTCGGAAC ATAAAGAG TCCAACGCT CAGGACCAA AAGCACCAT CGATATCTT GAGGATCGA CAGACATCT AGGGCTGTT GGGACACAA GAGAGCAAA CGCTGTTAAA ATCTTTTCTG AGTATGTTAA AAAAGATTT CATTGTGCGA CATAGATGGG AATAGCAACT TGAGCAAAAA TGCAAGTCAA ACCTGTTTTGT ACACTACGTAT CAAAATTGAT TTCTTCCCAAA GCAAAAGAGA AAGAAAAGCA AAAATAAACC TAAGCAAACT// GAGCAAGCTT TTGCACAGCA AAGGAAACCA TAAAATAACC CAAAAAGATC | GATGGGTG AGTTGAAG AGACTGAA GTCTGTATC GAGGAACAC //------------- // GAGCAAGCTT TTGCACAGCA AAGGAAACCA TAAAATAACC CAAAAAGATC | insertion |

The phrase "disrupted gene" is intended to encompass insertion, interruption, or deletion of a nucleotide sequence of interest wherein the disrupted gene either encodes a polypeptide having an altered amino acid sequence that differs from the amino acid sequence of the endogenous sequence, encodes a polypeptide having fewer amino acid residues than the endogenous amino acid sequence or does not encode a polypeptide although the wild-type nucleotide sequence of interest encodes a polypeptide.

The present specification provides a transgenic pig with reduced expression of functional αGal, β4GalNT2 and CMAH genes. "CMAH/αGAL double knockouts", "CMAH/αGAL DKO", "CMAH/αGal", "CMAH/αGal DKO", "CMAH^{-/-}/GAL^{-/-}", "αGal/CMAH DKOs", "αGAL/CMAH double knockouts", "GGTA1/CMAH DKO", "GT1/CMAH DKO", "GGTA1^{-/-}/CMAH^{-/-}", "GGT1^{-/-}/CMAH^{-/-}", "CMAH/GGTA DKO", "GT/CMAH-KO", "GGTA1/CMAH KO", "DKO (αGal/CMAH)", "DKO (αGAL & CMAH)", "CMAH-/αGal-", "αGal-/CMAH-", "CMAH-/αGAL-" and variants thereof refer to transgenic pigs, cells, or tissues that lack expression of functional alpha 1,3 galactosyltransferase and cytidine monophosphate-N-acetylneuraminic acid hydroxylase. A triple knockout product or pig may be created in a wild-type background or in a CMAH/αGal double knockout background. "CMAH/αGAL/B4GalNT2 triple knockouts", "CMAH/αGAL/β4GalNT2 triple knockouts', "CMAH/αGAL/B4GalNT2 TKO", "CMAH/αGal/β4GalNT2", "CMAH/αGal/B4GalNT2 TKO", "CMAH^{-/-}/GAL^{-/-}/B4GalNT2^{-/-}", "αGal/CMAH/β4GalNT2 TKOs", "αGAL/CMAH/β4Gal triple knockouts", "GGTA1/CMAH/B4GalNT2 TKO", "GT1/CMAH/β4GalNT2 TKO", "GGTA1^{-/-}/CMAH^{-/-} /β4GalNT2^{-/-}", "GGT1^{-/-}/CMAH^{-/-} / β4GalNT2^{-/-} ", "CMAH/GGTA/β4GalNT2 TKO", "GT/CMAH/β4GalNT2-KO", "GGTA1/CMAH/β4GalNT2 KO", "TKO (αGal/CMAH/β4GalNT2)", "TKO (αGAL , CMAH, β4GalNT2)", "CMAH-/αGal-/β4GalNT2-", "αGal-/CMAH-/β4GalNT2-", "CMAH-/αGAL-β4GalNT2-" and variants thereof refer to transgenic pigs, cells, or tissues that lack expression of functional alpha 1,3 galactosyltransferase, cytidine monophosphate-N-acetylneuraminic acid hydroxylase and β4GalNT2.

Transgenic transplant material. Transplant material encompasses organs, tissue, transfusion products and/or cells from a pig for use as xenografts. Transplant material for use as xenografts may be isolated from the transgenic pigs with decreased expression of αGal, β4GalNT2 and CMAH. Transgenic transplant material from transgenic or knockout pigs can be isolated from a prenatal, neonatal, immature or fully mature transgenic animal. The transplant material may be used as temporary or permanent organ replacement for a human subject in need of an organ transplant. Any porcine organ can be used including, but not limited to, the brain, heart, lungs, eye, stomach, pancreas, kidneys, liver, intestines, uterus, bladder, skin, hair, nails, ears, glands, nose, mouth, lips, spleen, gums, teeth, tongue, salivary glands, tonsils, pharynx, esophagus, large intestine, small intestine, small bowel, rectum, anus, thyroid gland, thymus gland, bones, cartilage, tendons, ligaments, suprarenal capsule, skeletal muscles, smooth muscles, blood vessels, blood, spinal cord, trachea, ureters, urethra, hypothalamus, pituitary, pylorus, adrenal glands, ovaries, oviducts, uterus, vagina, mammary glands, testes, seminal vesicles, penis, lymph, lymph nodes and lymph vessels.

The porcine tissues of the invention are useful for xenotransplantation. The porcine tissue is from a triple αGal/CMAH/β4GalNT2 transgenic pig. Any porcine tissue can be used including but not limited to, epithelium, connective tissue, blood, bone, cartilage, muscle, nerve, adenoid, adipose, areolar, brown adipose, cancellous muscle, cartilaginous, cavernous, chondroid, chromaffin, dartoic, elastic, epithelial, fatty, fibrohyaline, fibrous, Gamgee, gelatinous, granulation, gut-associated lymphoid, skeletal muscle, Haller's vascular, indifferent, interstitial, investing, islet, lymphatic, lymphoid, mesenchymal, mesonephric, multilocular adipose, thymus tissue, mucous connective, myeloid, nasion soft, nephrogenic, nodal, osteoid, osseus, osteogenic, bone marrow, retiform, periapical, reticular, smooth muscle, hard hemopoietic and subcutaneous tissue, devitalized porcine tissues including heart valves, skin, and tendons, and vital porcine skin.

Another embodiment provides cells and cell lines from the porcine triple transgenic animals with reduced or decreased expression of αGal, B4GalNT2 and CMAH. In one embodiment these cells or cell lines can be used for xenotransplantation. Cells from any porcine tissue or organ can be used including, but not limited to: epithelial cells, fibroblast cells, neural cells, keratinocytes, hematopoietic cells, melanocytes, chondrocytes, lymphocytes (B and T), macrophages, monocytes, mononuclear cells, cardiac muscle cells, other muscle cells, granulosa cells, cumulus cells, epidermal cells, endothelial cells, Islet of Langerhans cells, pancreatic insulin secreting cells, bone cells, bone precursor cells, neuronal stem cells, primordial stem cells, hepatocytes, aortic endothelial cells, microvascular endothelial cells, fibroblasts, liver stellate cells, aortic smooth muscle cells, cardiac myocytes, neurons, Kupferr cells, smooth muscle cells, Schwann cells, erythrocytes, platelets, neutrophils, lymphocytes, monocytes, eosinophils, basophils, adipocytes, chondrocytes, pancreatic islet cells, thyroid cells, thymus cells, parathyroid cells, parotid cells, glial cells, astrocytes, red blood cells, white blood cells, macrophages, somatic cells, pituitary cells, adrenal cells, hair cells, bladder cells, kidney cells, retinal cells, rod cells, cone cells, heart cells, pacemaker cells, spleen cells, antigen presenting cells, memory cells, T cells, B cells, plasma cells, muscle cells, ovarian cells, uterine cells, prostate cells, vaginal epithelial cells, sperm cells, testicular cells, germ cells, egg cells, leydig cells, peritubular cells, sertoli cells, lutein cells, cervical cells, endometrial cells, mammary cells, follicle cells, mucous cells, ciliated cells, nonkeratinized epithelial cells, keratinized epithelial cells, lung cells, goblet cells, columnar epithelial cells, dopaminergic cells, squamous epithelial cells, osteocytes, osteoblasts, osteoclasts, bone marrow, embryonic stem cells, fibroblasts and fetal fibroblasts.

Nonviable derivatives include tissues stripped of viable cells by enzymatic or chemical treatment these tissue derivatives can be further processed through crosslinking or other chemical treatments prior to use in transplantation. In a preferred embodiment, the derivatives include extracellular matrix derived from a variety of tissues, including skin, bone, urinary, bladder or organ submucosal tissues. In addition, tendons, joints, and bones stripped of viable tissue to including but not limited to heart valves and other nonviable tissues as medical devices are provided. In an embodiment, serum or medium suitable for cell culture and isolated from a transgenic pig of the invention are provided. Components of porcine transgenic organs, tissues or cells are also provided. Components may also be modified through any means known in the art including but not limited to crosslinking and aldehyde crosslinking. Components may vary depending on the larger organ or tissue from which the component is obtained. Skin components may include but are not limited to stripped skin, collagen, epithelial cells, fibroblasts and dermis. Bone components may include but are not limited to collagen and extracellular matrix. Heart components may include but are not limited to valves and valve tissue.

"Xenotransplantation" encompasses any procedure that involves the transplantation, implantation or infusion of cells, tissues or organs into a recipient subject from a different species. Xenotransplantation in which the recipient is a human is particularly envisioned. Thus xenotransplantation includes but is not limited to vascularized xenotransplant, partially vascularized xenotransplant, unvascularized xenotransplant, xenodressings, xenobandages, xenostructures and xenotransfusions.

In embodiments, cell culture reagents isolated from a transgenic pig comprising disrupted α(1,3)-galactosyltransferase, β4GalNT2 and CMAH genes are provided. Cell culture reagents are reagents utilized for tissue culture, *in vitro* tissue culture, microfluidic tissue culture, cell culture or other means of growing isolated cells or cell lines. Cell culture reagents may include but are not limited to cell culture media, cell culture serum, a cell culture additive, a feeder cell, and an isolated cell capable of proliferation. By an "isolated cell capable of proliferation" is intended a cell isolated or partially isolated from other cell types or other cells wherein the cell is capable of proliferating, dividing or multiplying into at least one additional clonal cell.

Cells grown in culture may synthesize or metabolically incorporate antigenic epitopes into a compound of interest produced by the cultured cell. The antigenic epitopes may result in increased binding by human antibodies and decreased efficacy of the compound of interest. See Ghaderi et al 2010 Nature Biotechnology 28(8):863-867 . Growing the producing cell in a cell culture reagent with an altered epitope profile such as a reduced level of αGal, B4GalNT2 or Neu5Gc may reduce the level of αGal antigens, Neu5Gc antigens, and/or Sd^{a}-like antigens, on the compound of interest. Compounds of interest may include but are not limited to glycoproteins and glycolipids. Glycoproteins of interest may include but are not limited to an antibody, growth factor, cytokine, hormone or clotting factor. Glycolipids of interest may include but are not limited to therapeutics, antigens, and biosurfactants.

The word "providing" is intended to encompass preparing, procuring, getting ready, making ready, supplying or furnishing. It is recognized that methods of providing a cell may differ from methods of providing a subject, methods of providing an organ may differ from methods of providing a pig, methods of providing a kidney may differ from methods of providing a liver and methods of providing an organ may differ from methods of providing a material suitable for transfusion.

Transplant rejection occurs when transplanted tissue, organs, cells or material are not accepted by the recipient's body. In transplant rejection, the recipient's immune system attacks the transplanted material. Multiple types of transplant rejection exist and may occur separately or together. Rejection processes included but are not limited to hyperacute rejection (HAR), acute humoral xenograft rejection reaction (AHXR), thrombocytopenia, acute humoral rejection, hyperacute vascular rejection, antibody mediated rejection and graft versus host disease. By "hyperacute rejection" we mean rejection of the transplanted material or tissue occurring or beginning within the first 24 hours post-transplant involving one or more mechanisms of rejection. Rejection encompasses but is not limited to "hyperacute rejection", "humoral rejection", "acute humoral rejection", "cellular rejection" and "antibody mediated rejection". The acute humoral xenograft reaction (AHXR) is characterized by a spectrum of pathologies including, but not limited to, acute antibody mediated rejection occurring within days of transplant, the development of thrombotic microangiopathy (TMA), microvascular angiopathy, pre-formed non-Gal IgM and IgG binding, complement activation, microvascular thrombosis and consumptive thrombocytopenia within the first few weeks post transplant. Thrombocytopenia is a quantity of platelets below the normal range of 140,000 to 440,000/µl. Thrombocytopenia related symptoms include, but are not limited to, internal hemorrhage, intracranial bleeding, hematuria, hematemesis, bleeding gums, abdominal distension, melena, prolonged menstruation, epistaxis, ecchymosis, petechiae or purpura. Uptake of human platelets by pig livers contributes to the development of thrombocytopenia in xenograft recipients. Thrombocytopenia may occur upon reperfusion of the xenotransplanted organ or after the immediate post-reperfusion period.

The porcine organs, tissues or cells of the invention are suitable for use in improving a rejection related symptom in a patient by transplanting said porcine organs, tissue or cells into a human, wherein one or more rejection related symptoms is improved as compared to when tissue from a wild-type swine is transplanted into a human. By "improving", "bettering", "ameliorating", "enhancing", and "helping" is intended advancing or making progress in what is desirable. It is also envisioned that improving a rejection related symptom may encompass a decrease, lessening, or diminishing of an undesirable symptom. It is further recognized that a rejection related symptom may be improved while another rejection related symptom is altered. The altered second rejection related symptom may be improved or increased. A second altered rejection related symptom may be altered in a less desirable manner. Rejection related symptoms include but are not limited to hyperacute rejection related symptoms and acute humoral xenograft reaction related symptoms. Rejection related symptoms may include, but are not limited to, thrombotic microangiopathy (TMA), microvascular angiopathy, pre-formed non-Gal IgM and IgG binding, complement activation, agglutination, fibrosis, microvascular thrombosis, consumptive thrombocytopenia, consumptive coagulopathy, profound thrombocytopenia, refractory coagulopathy, graft interstitial hemorrhage, mottling, cyanosis, edema, thrombosis, necrosis, fibrin thrombi formation, systemic disseminated intravascular coagulation, IgM deposition in glomerular capillaries, IgG deposition in glomerular capillaries, elevated creatinine levels, elevated BUN levels, T cell infiltrate, infiltrating eosinophils, infiltrating plasma cells, infiltrating neutrophils, arteritis, antibody binding to endothelium, altered expression of ICOS, CTLA-4, BTLA, PD-1, LAG-3, or TIM-3, and systemic inflammation.

"Hyperacute rejection related symptom" is intended to encompass any symptom known to the field as related to or caused by hyperacute rejection. It is recognized that hyperacute rejection related symptoms may vary depending upon the type of organ, tissue or cell that was transplanted. Hyperacute rejection related symptoms may include, but are not limited to, thrombotic occlusion, hemorrhage of the graft vasculature, neutrophil influx, ischemia, mottling, cyanosis, edema, organ failure, reduced organ function, necrosis, glomerular capillary thrombosis, lack of function, hemolysis, fever, clotting, decreased bile production, asthenia, hypotension, oliguria, coagulopathy, elevated serum aminotransferase levels, elevated alkaline phosphatase levels, jaundice, lethargy, acidosis and hyperbilirubenemia and thrombocytopenia.

Thrombocytopenia is a quantity of platelets below the normal range of 140,000 to 440,000/µl. Thrombocytopenia related symptoms include, but are not limited to, internal hemorrhage, intracranial bleeding, hematuria, hematemesis, bleeding gums, abdominal distension, melena, prolonged menstruation, epistaxis, ecchymosis, petechiae or purpura. Uptake of human platelets by pig livers contributes to the development of thrombocytopenia in xenograft recipients.

Platelets, also known as thrombocytes, are enucleate fragments of megakaryocytes involved in blood coagulation, hemostasis and blood thrombus formation. Human platelets are routinely isolated through a variety of methods including, but not limited to, platelet apheresis, plateletpheresis and ultracentrifugation.

The phrase "platelet uptake" is intended to encompass the incorporation of a platelet into a liver or liver cell. While not being limited by mechanism, such uptake may occur through a phagocytic process. Platelet uptake may be monitored by any platelet uptake monitoring assay known in the art. Platelet uptake monitoring assays include, but are not limited to immunological methods, western blots, immunoblotting, microscopy, confocal microscopy, transmission electron microscopy and phagosome isolation. It is recognized that the appropriate platelet uptake monitoring assay may depend upon the type of label used. Platelet uptake may be measured as a percentage of total platelets absorbed, percentage of total platelets not absorbed, a ratio of absorbed to unabsorbed platelets, percentage of cells absorbing at least one platelet, percentage of cells not absorbing a platelet, or number of platelets absorbed per cell. It is recognized that platelet uptake by more than one cell type may contribute to the total platelet uptake of the liver. Total platelet uptake by an animal liver may include platelet uptake by liver sinusoidal endothelial cells, platelet uptake by Kuppffer cells, platelet uptake by LSECs and Kupffer cells and platelet uptake by additional cell types. It is recognized that platelet uptake by different cell types may contribute similar or disparate fractions of the total platelet uptake by a liver. Thus an alteration, inhibition, reduction, decrease, or lowering of platelet uptake by a liver comprises an alteration, inhibition, reduction, decrease, or lowering of platelet uptake by one or more liver cell types.

While not being limited by mechanism, platelet uptake may occur through phagocytosis by LSEC and Kupffer cells. Phagocytosis is characterized by the formation of an endosome which by the fusion of lysosomes containing degradative enzymes becomes a phagosome.

Any method of evaluating, assessing, analyzing, measuring, quantifying, or determining a rejection related symptom known in the art may be used with the claimed compositions and methods. Methods of analyzing a rejection related symptom may include, but are not limited to, laboratory assessments including CBC with platelet count, coagulation studies, liver function tests, flow cytometry, immunohistochemistry, standard diagnostic criteria, immunological methods, western blots, immunoblotting, microscopy, confocal microscopy, transmission electron microscopy, IgG binding assays, IgM binding assays, expression asays, creatinine assays and phagosome isolation.

Expression of a gene product is decreased when total expression of the gene product is decreased, a gene product of an altered size is produced or when the gene product exhibits an altered functionality. Thus if a gene expresses a wild-type amount of product but the product has an altered enzymatic activity, altered size, altered cellular localization pattern, altered receptor-ligand binding or other altered activity, expression of that gene product is considered decreased. Expression may be analyzed by any means known in the art including, but not limited to, RT-PCR, Western blots, Northern blots, microarray analysis, immunoprecipitation, radiological assays, polypeptide purification, spectrophotometric analysis, Coomassie staining of acrylamide gels, ELISAs, 2-D gel electrophoresis, in situ hybridization, chemiluminescence, silver staining, enzymatic assays, ponceau S staining, multiplex RT-PCR, immunohistochemical assays, radioimmunoassay, colorimetric assays, immunoradiometric assays, positron emission tomography, fluorometric assays, fluorescence activated cell sorter staining of permeablized cells, radioimmunosorbent assays, real-time PCR, hybridization assays, sandwich immunoassays, flow cytometry, SAGE, differential amplification or electronic analysis. Expression may be analyzed directly or indirectly. Indirect expression analysis may include but is not limited to, analyzing levels of a product catalyzed by an enzyme to evaluate expression of the enzyme. See for example, Ausubel et al, eds (2013) Current Protocols in Molecular Biology, Wiley-Interscience, New York, N.Y. and Coligan et al (2013) Current Protocols in Protein Science, Wiley-Interscience New York, NY. Gene expression assays for porcine ASGR1 are commercially available (Applied Biosystems^{™}, Carlsbad CA).

"As compared to" is intended encompass comparing something to a similar but different thing, such as comparing a data point obtained from an experiment with a transgenic pig to a data point obtained from a similar experiment with a wildtype pig. The word "comparing" is intended to encompass examining character, qualities, values, quantities, or ratios in order to discover resemblances or differences between that which is being compared. Comparing may reveal a significant difference in that which is being compared. By "significant difference" is intended a statistically significant difference in results obtained for multiple groups such as the results for material from a transgenic pig and material from a wild-type pig. Generally statistical significance is assessed by a statistical significance test such as but not limited to the student's t-test, Chi-square, one-tailed t-test, two-tailed t-test, ANOVA, Dunett's post hoc test, Fisher's test and z-test. A significant difference between two results may be results with a p<0.1, p<0.05, p<0.04, p<0.03, p<0.02, p<0.01 or greater.

The word "isolated" is intended to encompass an entity that is physically separated from another entity or group. An isolated cell is physically separated from another group of cells. Examples of a group of cells include, but are not limited to, a developing cell mass, a cell culture, a cell line, a tissue, and an animal. The word "isolating" is intended to encompass physically separating an entity from another entity or group. Examples include physically separating a cell from other cells, physically separating a cell component from the remainder of the cell and physically separating tissue or organ from an animal. An isolated cell or cell component is separated by 10%, 15%, 20%, 25%, 30%, 35%, 40%, 45%, 50%, 55%, 60%, 65%, 70%, 75%, 80%, 85%, 90%, 95%, 96%, 97%, 98%, 99%, up to 100% from other naturally occurring cells or cell components. Methods for isolating one or more cells from another group of cells are known in the art. See for example Freshney (ED) Culture of Animal Cells: a manual of basic techniques (3rd Ed.) 1994, Wiley-Liss; Spector et al (Eds)(1998) Cells: a Laboratory Manual (vol.1) Cold Spring Harbor Laboratory Press and Darling et al (1994) Animal Cells: culture and media John Wiley & Sons. Methods of isolating a tissue or an organ from an animal are known in the art and vary depending on the tissue or organ to be isolated and the desired method of transplanting the tissue or organ. Methods of isolating a transfusion product from an animal or sample are known in the art and vary depending on the desired transfusion product. Such methods include but are not limited to centrifugation, dialysis, elution, apheresis and cryoprecipitation.

A "skin related product" encompasses products isolated from skin and products intended for use with skin. Skin related products isolated from skin or other tissues may be modified before use with skin. Skin related products include but are not limited to replacement dressings, burn coverings, dermal products, replacement dermis, dermal fibroblasts,collagen, chondroitin, connective tissue, keratinocytes, cell-free xenodermis,cell-free pig dermis,composite skin substitutes and epidermis and temporary wound coverings. See for example Matou-Kovd et al (1994) Ann Med Burn Club 7:143 .

The attachment period of a skin related product is the time between application of the skin related product to a human subject and natural separation of the skin related product from the human subject. When a human subject's skin wound has sealed, a skin related product may be removed by natural separation or mechanical separation. However natural separation of a skin related product from a human subject may occur prematurely. Premature natural separation occurs before separation is desired by a medical practitioner. By way of example and not limitation, premature natural separation may occur before the wound has been sealed. Premature natural separation may also be termed "sloughing", "shedding", or "flaking". Clinical management of premature natural separation may include reapplication of a skin related product, dressing application, bandage application, administering antibiotic, and administering fluids. A skin wound may be sealed by any means known in the art including but not limited to by growth of the subject's skin and by skin grafting. Reduced premature separation encompasses a decreased, lower, less frequent, diminished, smaller amount of natural separation of a skin related product before separation is desired by a medical practitioner. The reduced premature separation may relate to a lower number of complete, a lower number of partial premature separation events, and involvement of a smaller portion of the skin related product in a partial premature separation event than compared to a skin related product obtained from a wild-type pig. A skin related product of the instant application may also exhibit an increased, lengthened, improved, extended, or expanded attachment period. Use of a skin related product of the instant application may increase the duration of the attachment period.

A skin wound encompasses any injury to the integument including but not limited to an open wound, burn, laceration, ulcer, leg ulcer, foot ulcer, melanoma removal, cancer removal, plastic surgery, and bite.

By "surgically attaching" is intended joining, combining, uniting, attaching, fastening, connecting, joining or associating through any surgical method known in the art.

In an embodiment the application provides non-human material suitable for transfusions from multiple knockout porcine animals with reduced expression of the αGal, CMAH and β4GalNT2 genes. These materials suitable for transfusions may include, but are not limited to, blood, whole blood, plasma, serum, red blood cells, platelets, and white bloods cells. Such materials may be isolated, enriched or purified. Methods of isolating, enriching or purifying material suitable for transfusion are known in the art. Serologically porcine red blood cells (RBCs) share a number of common characteristics with human RBCs (Pond W.G, Houpt K.A, The Biology of the Pig Ithaca: Comstock Pub. Associates, 1978 and Jandl, J. H. Blood: Textbook of Hematology, Boston:Little, Brown, 1996). As in other mammals, the primary site for erythropoiesis in pigs is bone marrow. The pRBC is a biconcave disk of approximately 4-8 microns in diameter. The hematocrit of pig blood is 35-47%, with a hemoglobin concentration of 6-17 g/100 ml. The half-life of pRBC is approximately 40 days, in comparison to 60 days for human RBCs.

Fifteen pig blood group systems have so far been identified. The most important and well-studied is the A-O(H), which is closely related to the human ABO system. The A and O antigens on pRBC are passively adsorbed from circulating plasma glycosphingolipids, in a similar mechanism as human Lewis antigens (Marcus, D.M. & Cass, L.E. (1969) Science 164:553-555). pRBC phenotyping is not entirely reliable. Phenotyping of pigs can be achieved by immunohistochemical staining of buccal epithelial cells with an anti-A monoclonal antibody (mAb)(as used in Blood Banks) and an anti-H lectin antibody (Ulex europaeus)(Villarroya H et al 1990 Autoimmunity 6:47-60). The glycolipids bearing blood group A have been isolated from porcine stomach mucosa, epithelial cells and erythrocytes. Among a few of the well-characterized proteins derived from pRBC, porcine hemoglobin shares 85% sequence identity with its human counterpart and demonstrates a similar three-dimensional structure at 2.8Å resolution. Unlike most tissue cells, porcine RBCs do not contain a nucleus and therefore are less likely to harbor retroviruses such as, but not limited to, porcine endogenous retroviruses (PERVs). pRBCs also lack intracellular organelles and a relatively short half-life in vivo.

The following Examples are offered for illustrative purposes only and are not intended to limit the scope of the present invention in any way. Indeed various modifications in addition to those shown and described herein will become apparent to those skilled in the art from the foregoing description and the following examples and fall within the scope of the appended claims.

### EXAMPLES

### Example 1. DNA Sequencing Analysis of Targeted CMAH, GGTA1 and β4GalNT2 Regions

Genomic DNA from a cloned pig was extracted using GenElute Mammalian Genomic DNA Miniprep Kit (Sigma-Aldrich, St. Louis, MO). PCR amplification of the CMAH, GGTA1 and β4GalNT2 Crispr/Cas9 target regions was performed. Primers were used to sequence the targeted CMAH, GGTA1 and β4GalNT2 regions.

Pwo Master (Roche, Indianapolis IN) was used, Pwo SuperYield DNA polymerase, dNTPack (Roche Applied Science, Indianapolis, IN) was used. PCR conditions for GGTA1 were as follows: 94°C, 2 min; 94°C, 15 sec, 54°C, 30 sec, and 72°C, 45 sec for 15 cycles; 94°C, 15 sec, 54°C, 30 sec, 72°C, 45 sec with additional 5 sec each cycle for 25 cycles; and a final extension step of 72°C for 5 min. For *CMAH,* 94°C, 2 min; 94°C, 15 sec, 56°C, 30 sec, and 72°C, 45 sec for 15 cycles; 94°C, 15 sec, 56°C, 30 sec, 72°C, 45 sec with additional 5 sec each cycle for 25 cycles; and a final extension step of 72°C for 5 min. For *β4GALNT2,* 94°C, 2 min; 94°C, 15 sec, 62°C, 30 sec, 72°C, 40 sec for 15 cycles, 94°C 15 sec, 62°C, 30 sec, 72°C 40 sec with additional 5 sec each cycle for 25 cycles; and a final extension step of 72°C for 5 min. The PCR products were separated on 1% agarose gel and purified by GenElute Gel Extraction Kit (Sigma-Aldrich, St. Louis, MO). The PCR products were sequenced by the Sanger method (DNA Sequencing Core Facility, Indiana University School of Medicine) with the specific sequencing primer, 5' CCTTAGTATCCTTCCCAACCCAGAC 3' (SEQ ID NO:5) for GGTA1; 5' CATTTTCTTCGGAGTTGAGGGC 3' (SEQ ID NO:6) for *CMAH;* 5' AAAGCCACAGGAGGAGCCAG 3' (SEQ ID NO:7) for β4GALNT2. Once the mutations were detected, PCR products were inserted into pCR4blunt-TOPO vector and transformed into *E*. *coli.* Individual clones were sequenced again to further investigate the mutation in each allele of the individual gene.

Results from an exemplary DNA sequence analysis are summarized in Figure 1, panels A-C. DNA sequence analysis confirmed alterations of the GGTA1 and CMAH gene in both alleles in at least one pig. Sequence analysis showed overlapping five and seven base pair deletions in the GGTA1 target region (panel A). In the same pig, sequence analysis confirmed a disruption of the CMAH gene consisting of a 12 base pair deletion on one allele and an overlapping 5 base pair substitution for a 3 base pair deletion on the other allele (panel B). DNA sequence analysis confirmed alterations of the β4GalNT2 gene sequence in the same pig. The β4GalNT2 gene sequence data show three variations; the presence of three mutations may suggest the β4GalNT2 gene occurs in at least two loci. In an overlapping region of the β4GalNT2 gene, one allele shows a single base pair insertion, one allele shows a five base pair deletion and one allele shows a twelve base pair deletion/one base pair substitution. No evidence of a wildtype β4GalNT2 sequence was found (panel C). Figure 13 summarizes DNA sequence analysis of additional triple knockout pig isolates.

### Example 2. Production of Knockout Pigs (Triple Transgenic Pigs)

Oligo annealing and cloning into the PX330 plasmid to drive gRNA expression was performed using Addgene plasmid 42230 [http://www.addgene.org/42230 and 20]. Oligo pairs for the targeted genes are GGTA1 (NCB1 Accession:XM_005660398.1), 5'CACCGAGAGAAAATAATGAATGTCAA-3' forward) (SEQ ID NO:8), 5'AAATTGACATTCATTATTTTCTC-3' (reverse) (SEQ ID NO:9); CMAH (NCBI Accession: NM_001113015.1) 5'-CACCGAGTAAGGTACGTGATCTGT-3' (forward) (SEQ ID NO:10), 5'-AAACACAGATCACGTACCTTACTC-3' (reverse) (SEQ ID NO:11; β4GalNT2 (NCBI Accession: NM_001244330.1) 5'-CACCGTGTATCGAGGAACACGCTT-3' (forward) (SEQ ID NO:12), 5'-AAACAAGCGTGTTCCTCGATACAC-3' (reverse) (SEQ ID NO:13).

Liver-derived cells were cotransfected with all three gRNA/Cas9 plasmids. After 48 hr, the treated cells were passed over an IB4-lectin column to isolate α-Gal null cells. Two million α-Gal negative cells were further stained with fluorescein labeled *Dolichos biflorus* Agglutinin (DBA)-FITC (Vector Laboratories, Burlingame, CA, USA) at 2 µg/ml in 500 µl HBSS with 0.5% BSA and flow-sorted for DBA-negative cells using a BD FACSARia sorter (BD Bioscience, San Jose, CA, USA). The presence of Neu5Gc, an indicator of CMAH gene function, was not analyzed prior to somatic cell nuclear transfer.

Somatic cell nuclear transfer (SCNT) was performed using *in vitro* matured oocytes (DeSoto Biosciences Inc., St. Seymour TN and Minitube of America (Mount Horeb WI). Cumulus cells were removed from the oocytes by pipetting in 0.1% hyaluronidase. Oocytes with normal morphology and a visible polar body were selected and incubated in manipulation media (calcium-free NCSU-23 with 5% fetal bovine serum (FBS) containing 5 µg/ml bizbenzimide and 7.5 µg/ml cytochalasin B for 15 minutes. Following this incubation period, oocytes were enucleated by removing the first polar body and metaphase II plate. For the triple transgenic pigs, single cells of site-targeted, IB4 counter-selected, DBA negative liver derived cells (LDC) were injected into each enucleated oocyte. Some triple transgenic pigs were developed from SCNT of fetal fibroblasts obtained from an aborted triple knockout pig fetus created from site-targeted, IB4 counter-selected, DBA negative liver derived cells. Electrical fusion was induced with a BTX electroporator (Harvard Apparatus, Holliston MA). In various instances enucleated oocytes injected with a cell (couples) were exposed to two DC pulses of 140 V of 50 µs in 280 mM mannitol, 0.001 mM CaCl₂ and 0.05 mM MgCl₂ or 180 V for 50 µs in 280 mM mannitol, 0.1 mM CaCl₂, and 0.05 mM MgCl₂. After activation the oocytes were placed in NCSU-23 medium with 0.4% bovine serum albumin (BSA) and incubated at 38.5°C, 5% CO₂ in a humidified atmosphere for less than one hour. Within an hour after activation, oocytes were transferred into a recipient pig. Recipient pigs were synchronized occidental pigs on their first day of estrus. Pregnancies were verified by ultrasound at day 25 or day 26 after embryo transfer.

All animals used in this study were approved by the Institutional Biosafety Committee (IBC) and Institutional Animal Care and Use Committee (IACUC).

### Example 3. IB4 Counterselection of Triple Knockouts

Liver-Derived Cells (LDCs) were transfected with three sets of targeting constructs (αGal, β4GalNT2 and CMAH). Cells were selected with IB4, a substance that binds αGal. DNA from cells in the bulk population of cells that survived IB4 counterselection was obtained, and the target gene sequences were evaluated. The bulk population cells that survived IB4 counterselection were used directly in SCNT to make pregnant pigs.

### Example 4. Design of Targeting Vectors

Targeting vectors such as CrispR constructs, Zn finger constructs and TALEN constructs are designed to target the sequence of porcine CMAH (Ensemble transcript ENSSSCT00000001195) at a suitable site. Targeting vector constructs are designed to target GGTA1 (Ensemble transcript ENSSSCT00000006069) at a suitable site. Targeting vector constructs are designed to target β4GalNT2 at a suitable site in NCBI GeneID:100621328 and Ensemble: ENSSSCG00000030269.

A CMAH Crispr construct with a primer sequence that is the reverse complement of a portion of the sequence listed in the Ensemble transcript was created and utilized in the creation of a triple knockout pig. A Gal Crispr construct with a primer sequence identical to a portion of that in the appropriate Ensemble transcript was created and utilized in the creation of a triple transgenic pig. An β4GalNT2 Crispr construct with a primer sequence identical to a portion of the above indicated NCBI sequence NCBI GeneID:100621328 was created and utilized in the creation of a triple transgenic pig.

### Example 5. Crossmatch of Human Sera with Transgenic PBMCs

Porcine whole blood from transgenic (triple GGTA-1/β4GalNT2/CMAH for example) and wild-type pigs were collected in ACD from venous puncture. The whole blood was mixed 1:1 with PBS and separated with Ficoll. Porcine peripheral blood monocytes (PBMCs) were prepared from the whole blood using Ficoll-Paque Plus (GE Healthcare). PBMCs were removed from Ficoll layers and washed several times with PBS followed by lysis solution as need to remove red blood cells. PBMCs were suspended in EX-CELL media at 4 million cells/ml (EX-Cell 610 HSF-Serum free Medium for Hybridoma Cells 14610C).

Sera were obtained from 44 healthy human volunteers. Twenty-five percent heat inactivated serum was prepared. 25 µl of heat-inactivated serum, 25 µl of EX-CELL media and 50 µl of suspended cells were added to each well on a 96 well V-bottom plate. Cells were incubated at 4°C for 30 minutes, and then washed three times with EX-CELL media. Cells were stained with secondary antibody as follows: Alexa Flour 488 goat anti-human IgG 109-546-170 at 1:250 concentration in EX-CELL media, Alexa Flour 488 goat anti-human IgM 709-546-073 at 1:250 concentration in EX-CELL media, 100 µl of secondary was added. Cells were suspended with a pipet. Cells were incubated at 4°C for 30 minutes. Cells were washed once with EX-CELL media and resuspended with pipet in EX-Cell media or 1:1 EX-CELL media and Flow Fixative Solution (1% buffered paraformaldehyde). Flow cytometric analysis was completed with channel FL-1 gating for Alexa flour 488. Results from one such experiment are shown in Figure 3, panel B.

### Example 6. DBA Lectin Flow Cytometry Staining

Whole blood from triple transgenic pigs and other pigs of interest was collected in anticoagulant citrate dextrose (ACD). The whole blood was mixed 1:1 with PBS and separated with ficoll (Ficoll-Paque PLUS, GE Healthcare 17-1440-03). Flow wash and staining buffers containing calcium were obtained. The flow wash buffer was HBSS with 0.5% BSA IgG-free 0.1% sodium azide pH 7.4 ), 0.45 µM filtered to remove particulates. PBMCs were resuspended in Flow Wash Buffer at 2 x 10⁶ cells/ml and suspended homogenously. The cells were blocked for 15 minutes on ice. Cells were resuspended again. 100 µl (2 X 10⁵ cells) were added to 5 ml flow tubes. DBA-fluorescein (2 mg/ml stock) was obtained. The DBA-fluorescein lectin stock was diluted 1:10 in Flow Buffer to a final concentration of 0.2 µg/ml. DBA-fluorescein lectin was added to the cells at a ratio of 1 µg DBA: 1 x 10⁶ cells (0.2 µg/ 2 x 10⁵ cells or 1 µl). DBA lectin and cells were incubated 30 minutes at room temperature. Cells were washed thoroughly with 4 ml Flow Wash HBSS. Cells were spun at 400 x g for 5 minutes and the wash supernatant was removed. Cells were resuspended in 200 µl Flow Wash HBSS. The same parameters were utilized if additional washes were performed. If desired, cells were fixed in approximately 200 µl Flow Fixative/2 x 10⁵ cells after the last wash and stored at 4°C until analysis. Flow cytometry analysis was performed with the gate on forward and sidelight scatter. Cellular fluorescent events were collected in FL-1; 10,000 events were collected in scatter gate. For each cell line both cells only and DBA-fluorescein data were collected. IB4 lectin interacts with αGal linked carbohydrates produced by the αGal gene product. The HD antibody interacts with the Neu5Gc carbohydrate produced by the CMAH gene product. DBA lectin interacts with the carbohydrate structure produced by the β4GalNT2 gene product. Results from one such experiment are presented in Figure 3, panel A.

### Example 7. IgG Antibody Binding to RBC

Human A and two human O sera and RBC were obtained. Porcine wildtype and j/CMAH/GAL triple knockout RBC were obtained. Erythrocytes were isolated from whole blood collected in acid-citrate-dextrose tubes (Becton Dickinson & Co., Franklin Lakes NJ) using Ficoll-Paque Plus (GE Health, Uppsala Sweden). Fresh serum was isolated by collecting whole blood in the absence of anticoagulant and centrifuging to remove clotted material. After density isolation, RBC were washed three times with PBS and diluted 1:10 in PBS at room temperature. To determine the antibody binding to RBC (2 X 10⁵ cells/well) were incubated with diluted, heat-inactivated human serum for 30 min at 4°C, final serum concentration 25%. The cells were washed three times in PBS with azide and stained with goat anti-human IgG Alexa Fluor 488 or donkey anti-human IgM Alexa Fluor 488 (Jackson ImmunoResearch Laboratories, Inc., West Grove, PA, USA). Flow cytometry analysis was performed using an Accuri C6 flow cytometer and CFlow software (Accuri, Ann Arbor, MI USA). RBC gating was based on forward and side scatter. Exemplary IgG antibody binding to RBC flow cytometry traces are shown in Figure 5.

Cell surface analysis of porcine RBC cell surface glycans was performed using antibodies/lectin isolectin *Griffonia simplicifolia* GS-IB4 Alexa Fluor 647 (Invitrogen, Grand Island NY, USA) for αGal, a chicken anti-Neu5Gc antibody kit (BioLegend, San Diego CA) for Neu5Gc, and Fluorescein *Dolichos Biflorus Agglutinin* (DBA) (Vector Laboratories, Inc. Burlingame, CA USA) for β4GalNT2-derived carbohydrates. Traces from one such a series of experiments are presented in Figure 6. [Matt- please confirm experimental details.]

### Example 8. RBC Stripping and Associated Flow Cytometry

Following density isolation, RBCs were washed three times in PBS and suspended in 50% Alsever's solution. RBC's were pelleted at 21,300 x g for 2 minutes. Serum was added to the pelleted cells at a 1:1 of cell pellet volume to serum volume. Serum RBC mixture was mixed and incubated for 20 minutes at 4°C. Cells were pelleted at 21,300 x g for 2 minutes and washed once with Alsever's solution. Cells were mixed with acid stripping buffer (pH 2.75 Citric Acid/Phosphate at 300 mOs/kg) to remove bound antibodies and neutralized with 1 M Tris-base, pH 9.0 (Calbiochem, LaJolla CA). Material that eluted during the low pH treatment was analyzed by SDS-PAGE and mass spectrometry (see below). Matching flow cytometric analysis for antibody elution sample was completed using 2 x 10⁶ RBCs. Cells were suspended in EX-CELL 610-HSF Serum-Free Medium (Sigma, St. Louis, MO USA) with 0.1% sodium azide incubated for 30 min at 4°C with a mixture of 25% heat inactivated serum. RBCs were washed three times and stained with goat anti-human IgG Alexa Fluor 488 or donkey anti-human IgM Alexa Fluor 488 (Jackson ImmunoResearch Laboratories Inc., West Grove PA, USA) antibodies. Secondary antibodies were incubated with RBCs for 30 minutes at 4°C and washed. Flow cytometric analysis completed on BD Accuri C6 flow cytometer (Accuri, Ann Arbor, MI, USA) and histograms were generated using FlowJo 7.6.5 (FlowJo LLC, Ashland OR, USA). Representative traces are shown in Figure 8. RBC gating was based on forward and side scatter. A representative gating is shown in Figure 10.

### Example 9. SDS-PAGE gel analysis of proteins eluting from RBC

A single human serum was incubated with various RBC (wildtype pigs (W), CMAH/GAL DKO (D), CMAH/GAL/β4GalNT2 (T); and autologous human (A)). After the stripping described above, neutralized eluates were precipitated by diluting 100 µl elution into 900 µl acetone and then incubated in minus 20°C for 30 minutes. Precipitates were spun 20,000 X g at 4°C for 15 min. Supernatant was discarded and pellets were washed with 70% v/v ethanol in water. Precipitates were spun again at 20,000 x g at 4°C for 15 minutes. Supernatant was again discarded and pellets were dried in a Vacufuge Plus (Eppendorf, Hauppauge, NY, USA) at 37°C for 30 min. Samples were then dissolved in 100 microliters of 2 X Laemmeli Buffer (Bio-Rad, Hercules, CA, USA) containing 2-mercaptoethanol (Sigma-Aldrich, St. Louis, MO, USA) according to the manufacturer's instructions. Samples were then heated at 95°C for 5 min and then allowed to cool to room temperature. Fifteen microliters of each sample was then loaded onto a 26-well, 4-20% Stain-Free TGX gel (Bio-Rad) and electrophoresed under denaturing and reducing conditions at 200 V for 42 min using the Bio-Rad criterion system. After electrophoresis, gel was removed and stained for 30 min with 100 milliliters G-250 Bio-Safe Coomassie Stain (Bio-Rad). Gel was then destained in water and imaged using a 700 nm laser on a Li-Cor Classic Imager (LiCor Biosciences, Lincoln, NE USA). Respective samples were then shipped on dry ice for mass spectrometry preparation and analysis. A process schematic is shown in Figure 7, panel A; a representative gel of material eluted from RBC is shown in Figure 7, panel B.

### Example 10. Mass Spectrometry Quantitation of Immunoglobulin Eluted from RBC

Mass spectrometry analysis was performed by MSBioworks, LLC. 50 µl aliquots of each sample were treated with PNGase F (New England Biolabs) according to manufacturer's instructions. Each sample was acetone precipitated for 30 minutes followed by a wash with 70% ethanol at 4°C per client protocol. Resultant precipitates were dried and re-suspended in 40 µl 1.4 X LDS loading buffer with DTT. 20 µl was run on a 4-12%bis tris SDS PAGE gel in the MOPS buffer system.

The region containing the heavy chain (50kDa) was excised and trypsin digestion was performed using a robot (ProGest, DigiLab) with the following protocol: 1) Washed with 25mM ammonium bicarbonate followed by acetonitrile. 2) Reduced with 10 mM dithiothreitol at 60°C followed by alkylation with 50 mM iodoacetamide at RT. 3) Digested with trypsin (Promega) at 37°C for 4 h. 4) Quenched with formic acid and the supernatant was analyzed directly without further processing.

The gel digests were analyzed by nano LC/MS/MS with a Waters NanoAcquity HPLC system interfaced to a ThermoFisher Q Exactive. Peptides were loaded on a trapping column and eluted over a 75 µm analytical column at 350 nL/min; both columns were packed with Jupiter Proteo resin (Phenomenex). The mass spectrometer was operated in data-dependent mode, with MS and MS/MS performed in the Orbitrap at 70,000 FWHM resolution and 17,500 FWHM resolution, respectively. The fifteen most abundant ions were selected for MS/MS. The area under the curve (AUC) was identified and calculated for each isotype specific-peptide. Comparing AUC enables a quantitative evaluation of the levels of each antibody in a sample. Representative traces are shown in Figure 9. Sequences of the peptides used to identify and quantitate each antibody by mass spectroscopy are shown in Table 2.

| **Isotype** | **Peptide** | **Peptide (M/Z)** | **Missed-Cleaved Peptide** | **Missed-Cleaved Peptide (M/Z)** |
|---|---|---|---|---|
| IgG1 | EEQYNSTYR | 595.7516 | TKPREEQYNSTYR | 836.8999 |
| IgG2 | EEQFNSTFR | 579.7567 | TKPREEQFNSTFR | 820.9049 |
| IgG3 | EEQYNSTFR | 587.7542 | TKPREEQYNSTFR | 828.9024 |
| IgG4 | EEQFNSTYR | 587.7542 | TKPREEQFNSTYR | 828.9024 |
| IgM | YKnNSDISSTR | 643.3044 | | |

| | | | | |
|---|---|---|---|---|
| • Lower case n in the IgM peptide represents a deamidated asparagine residue presumably occurring as a consequence of PNGase F treatment. | | | | |

### Example 11. Data Processing

Data were searched using a local copy of Mascot with the following parameters: Enzyme: Trypsin, Database: Swissprot Human (forward and reverse appended with common contaminants), Fixed modification: Carbidomethyl (C), Variable modifications: Oxidation (M), Acetyl (Protein N-term), Deamidation (NQ), Pyro-Glu (N-term Q). Mass values: Monoisotopic Peptide Mass Tolerance: 10 ppm Fragment Mass Tolerance: 0.02 Da Max Missed Cleavages: 2 Mascot DAT files were parsed into the Scaffold software for validation, filtering and to create a nonredundant list per sample. Data were filtered 1% protein and peptide level false discover rate (FDR) and requiring at least two unique peptides per protein. Raw LC/MS data was inspected for the accurate m/z values of the target peptides the selected ion chromatograms were extracted in QualBrowser (Thermo); the peak area in each case was calculated.

### Example 12. Comparison of Mass Spectrometry and Flow Cytometry Techniques for Quantitating Antibody Binding to RBC

The use of fluorescent secondary antibodies, a potential for artificial masking and the inability to easily report on different IgG isotypes may impact flow cytometry results. Quantitative mass spectrometry may allow direct peptide analysis without secondary reagents and can provide information on individual isotype levels. Mass spectrometry and flow cytometry provide measures of antibody binding that may reflect different inherent biases in the methods. Individual isotype levels may contribute differently to various immune effector functions. Serum and RBC were collected from three people. Each serum was incubated with its autologous RBC (A) and with pig RBC (wildtype (W) or triple knockout (T)). Immunoglobulin binding was evaluated using flow cytometry and mass spectrometry. Figure 8, panels A and B provide flow cytometry traces from one such experiment. Mass spectrometry quantitation indicated that triple knockout swine cells (T) bound less IgG than autologous human RBC (A) for two of three sera and less IgM for all three sera. Both flow cytometry and quantitative mass spectrometry indicated low levels of human immunoglobulin binding to RBC from the tripe knockout pigs. Comparisons were made to human blood group O RBC because the antigenicity of human blood group O RBC is sufficiently low to avoid humoral damage even in the absence of immune suppression.

### Example 13. Isotype analysis of Antibody Binding to RBC

Mass spectrometry AUC was used to quantitate the binding of each isotype to the different RBC. Evaluations were performed using GGTA1/CMAH knockout RBC (D), GGTA1/CMAH/β4GalNT2 knockout RBC (T) and autologous human RBC (A). Zero values in serum 1 indicate no binding of the particular isotypes to a target cell. IgG4 binding to autologous human cells in sera 2, 3 and 4 increased in a range from 10-fold to 16-fold. IgG2 was the only other isotype to show increased binding to human cells (A) compared to the pig (T) RBC in multiple sera, but the increases were smaller than those seen for IgG4 (ranges 3 to 6-fold, see sera 2, 3, and 4). Results from one such series of experiments are shown in Figure 10.

### Example 14. Lectin Staining of Different Pigs

Porcine kidneys from GGTA1/CMAH DKO pigs were flushed with 0.025% of collagenase type IV from *Clostridium histolyticum* (Sigma, St. Louis, MO, USA). Primary RMEC were isolated and cultured with RPMI medium supplemented with 10% of DKO pig serum, 100 µg/ml endothelial cell-specific growth factor, penicillin, streptomycin, and amphotericin B. After a 3-day culture, the porcine RMEC were infected for 24 h with lentiviral supernatant, containing lentiviral vector in which a c-DNA expresses the large and small T antigen of SV40 (Applied Biological Materials Inc, Richmond, BC, Canada). Single-cell clones were isolated and amplified up to 10 passages. iRMEC were cultured with RPMI medium supplemented with 10% of DKO pig serum, 100 µg/ml endothelial cell-specific growth factor, penicillin, streptomycin, and were used for characterization within passages 15-40. αGal/CMAH/β4GalNT2/SLA antigen disrupted iREC's were obtained.

Primary aortic endothelial cells from aortic thoracic and abdominal branches of GGTA1/CMAH/B4GALNT2 KO pig were isolated with 0.025% of collagenase type IV from Clostridium histolyticum (Sigma, St.Louis, MO, USA) ². The AEC were cultured with RPMI 1640 medium supplemented with GGTA1/CMAH/B4GALNT2 KO pig serum, 100 ug/ml endothelial cell-specific growth factor, penicillin, streptomycin, and amphotericin B, as well as 10% FBS for WT/GGTA1 KO AEC or 5% GGTA1/CMAH DKO pig serum for GGTA1/CMAH DKO and GGTA1/CMAH/B4GALNT2 AEC. These cells were used within 5 passages. CMAH deficient porcine cells were grown in GGTA1/CMAH DKO pig serum to prevent uptake of Neu5Gc from bovine serum.

Primary AEC cells (2 x 10⁵ cells) were staining using Fluorescein labeled *Dolichos biflorus* agglutinin lectin (diluted at 1:1000 in HBSS) (DBA, Vector Laboratories, Burlingame, CA, USA), fluorescein labeled *Artocarpus integrifolia* lectin (diluted at 1:1000 in HBSS) (AIL, Vector Laboratories), fluorescein labeled *Vicia villosa* lectin (diluted at 1:1000 in HBSS) (VVL, Vector Laboratories), fluorescein labeled *Arachis hypogaea* lectin (diluted at 1:1000 in HBSS) (PNA, Vector Laboratories), and Alexa Fluor 488 labeled Isolectin IB₄ (diluted at 1:1000 in HBSS) from *Griffonia simplicifolia* (Invitrogen, Grand Island, NY, USA ). For Neu5GC, cells were stained at a ratio of 1:5000 final dilution from a 0.5mg/mL stock with Alexa Fluor labeled Anti-Neu5GC or normal chicken IgY (BioLegend). Cells were stained for 30 minutes at 4°C. Cells were then washed and analyzed using a C6 flow cytometer (BD Biosciences). Fluorescence intensity was then calculated compared to either unstained cells in the case of lectins or isotype control in the case of Neu5GC. Representative traces are shown in Figure 12.

### Example 15. Phenotype Analysis of PBMC

Peripheral blood mononuclear cells (PBMCs) were isolated by Ficoll-Paque (GE Healthcare, Uppsala, Sweden) and counted. Cells were then resuspended at a density of 2 x 10⁶ cells/milliliter in Neu5GC Blocking Buffer (Biolegend, San Diego, CA, USA) diluted in Hank's Balanced Salt Solution (HBSS) per manufacturer's instructions. Cells were incubated on ice for 15 minutes prior to staining. One hundred microliters (2 x 10⁵ cells) were added to 12x75mm polystyrene flow staining tubes (BD Biosciences, Bedford, MA, USA). Cells were stained at a ratio of one microgram per 1 x 10⁶ cells of : Fluorescein labeled *Dolichos biflorus* agglutinin lectin (DBA, Vector Laboratories, Burlingame, CA, USA), fluorescein labeled *Artocarpus integrifolia* lectin (AIL, Vector Laboratories), fluorescein labeled *Vicia villosa* lectin (VVL, Vector Laboratories), fluorescein labeled *Arachis hypogaea* lectin (PNA, Vector Laboratories), and Alexa Fluor 488 labeled Isolectin IB₄ from *Griffonia simplicifolia* (Invitrogen, Grand Island, NY, USA ). For Neu5GC, cells were stained at a ratio of 1:5000 final dilution from a 0.5mg/mL stock with Alexa Fluor labeled Anti-Neu5GC or normal chicken IgY (BioLegend). Cells were stained for 30 minutes while incubating on ice. Cells were then washed with four milliliters of Neu5GC Blocking Buffer and pelleted at 400 x g for five minutes. The supernatant was discarded and cells were resuspended in 200 microliters of the blocking buffer and analyzed immediately. Cells were analyzed using a C6 flow cytometer (BD Biosciences) by collecting 10,000 events using a forward and side scatter gating method. Fluorescence intensity was then calculated compared to either unstained cells in the case of lectins or isotype control in the case of Neu5GC.

### Example 16. Clinical Crossmatch Testing

PBMC from triple transgenic pigs were subjected to human clinical crossmatch testing utilized in allotransplantation. Sera were obtained from 31 human subjects with a panel of reactive antibodies (PRA) of 0 (top graph) and 19 human subjects with a panel PRA score exceeding 80 (bottom graph). Clinicians routinely transplant human organs with a cytotoxicity score of 1. Ficoll treated PBMC from triple transgenic pigs were adjusted to a cell concentration of 2 X 10⁶ cells/ml. Some sera aliquots were treated with DTT. Two sets of crossmatch trays were prepared. Sera and controls were loaded in the crossmatch trays. The cell preparation was thoroughly mixed and drawn up slowly in a Hamilton repeating dispenser. One microliter of cells were added to each well containing sera over an illuminated view box. Test cells were added last to positive control wells. Equal numbers of trays were placed into opposing buckets of a Sorvall centrifuge. The centrifuge was turned on and allowed to reach 1000 rpm, then the centrifuge was turned off. Trays were examined over an illuminated view box to ensure cell droplets were mixed with serum. Trays were incubated for 30 minutes at room temperature. All trays were washed four times with 15 µl PBS to each well using Jet Pipette. Cells were allowed to settle for 2-3 minutes using a gentle wash technique. PBS, oil and serum was removed.

A working dilution of AHG/C' Class 1 mixture was prepared. One set of trays was treated with 5 µl AHG/C'; the other set of tray was treated with 5 µl undiluted rabbit complement (No AHG trays). Trays were optionally placed on a rotator for 4 minutes at 60 rpm. Trays were incubated for 60 minutes at room temperature. Cells were stained with 5 µl Fluoroquench and allowed to stand for 5'. Trays were placed on an inverted phase fluorescent microscope; each well was evaluated using a total magnification of 160X.

The percentage of dead cells in each well is estimated. AO crosses intact cell membranes of living cells, intercalates DNA and fluoresces green (535 nm) when excited at 490 nm. Dead cells stained with ethidium bromide fluoresce red (605 nm) when excited at 490 nm. Each well is scored using the system described below:

| % Dead Cells | Score | Interpretation |
|---|---|---|
| 0 - 10% | 1 | Negative |
| 11 - 20% | 2 | Positive |
| 21 - 50% | 4 | Positive |
| 51 - 80% | 6 | Positive |
| 81 - 100% | 8 | Strong Positive |
| ? | 0 | Not Readable. |

Results from one such series of experiments are shown in Figure 14. Note multiple sera with cytotoxicity scores of 1.

### Example 17. Ex vivo Perfusion of Human Platelets through Transgenic Liver

A triple transgenic GGTA1/CMAH/β4GalNT2 pig is anesthetized and intubated. A midline abdominal incision is made. The liver is removed and placed in a perfusion device under normothermic conditions. Humidity, temperature and air flow are maintained in the perfusion device. The perfusion device maintains constant pressure by varying the flow rate. Centrifugal flow through the portal vein and pulsatile flow through the hepatic artery are used. Both flow rates are set at porcine physiological pressure. The base perfusion solution is an oxygenated Ringers solution with physiologic nutrition and insulin.

Human platelets are obtained from healthy volunteer subjects or purchased commercially less than six days from isolation and are stored at 20-24°C. Approximately 1 x 10¹¹ human platelets are washed in sterile phosphate buffered saline (PBS) containing the anti-coagulant citrate dextrose. Platelets may be labeled with CFSE according to the manufacturer's protocol.

Pig livers are perfused two hours prior to the addition of platelets. Platelet samples are obtained prior to addition to the perfusion system and after the addition of the platelets at predetermined time points. Platelet levels in the pre-perfusion and post-perfusion samples are evaluated. Pre and post-perfusion evaluation of the pig liver are performed. Wild-type pig livers are obtained, and the livers are perfused under similar conditions.

### Example 18. Evaluation of Response to a Transgenic Xenograft

Porcine livers are obtained from a triple knockout pig (αGal,CMAH,β4GalNT2). The livers are surgically transplanted into a recently deceased human cadaver using the piggyback method. After the surgery, biological samples are obtained from the human cadaver. Clinical indicia of a rejection related response are monitored.

### Example 19. Evaluation of Response to a Transgenic Xenograft

Porcine kidneys are obtained from a triple transgenic pig (αGal,CMAH,β4GalNT2). A highly sensitized human subject is administered compounds to manage preexisting and *de novo* donor-specific antibodies. The porcine kidneys are surgically transplanted into the subject. After the surgery, biological samples are obtained from the human cadaver. Clinical indicia of a graft rejection are monitored.

### Example 20. Confocal microscopy analysis

Piglets (triple GGTA1, CMAH, βGalNT2 knockouts, wild type or other piglets of interest) are euthanized. Liver, heart and kidney tissue are obtained from the pig. Frozen sections of each tissue are prepared. Mounted tissues are blocked in Odyssey blocking buffer (Li-Cor Biosciences, Lincoln NE) in HBSS for one hour. The slides are fixed in 4% paraformaldehyde for 10 minutes. Tissues are stained with IB4 lectin Alexa Fluor 647 (Invitrogen, Grand Island NY) to visualize the presence of the Gal epitope. To visualize the Neu5Gc epitope, tissues are stained with a chicken anti-Neu5Gc antibody or with a control antibody (Sialix, Vista CA) for an hour. Tissues are stained with DBA to visualize the presence of Sd^{a}-like epitopes Tissues are washed three times with HBSS. Donkey anti-chicken Dylight 649 (Jackson ImmunoResearch Laboratories Inc, West Grove PA) secondary antibody is incubated with the tissue for approximately an hour. Tissues are washed three times with 0.1 % HBSS Tween. To stain the nucleus, DAPI stain (Invitrogen, Grand Island NY) is added to all the slides for 1 minute followed by two 0.1% HBSS Tween washes. Tissues are mounted in ProLong Gold (Invitrogen, Grand Island NY). Confocal microscopy is performed using an Olympus FV1000.

### Example 21. Antibody-mediated Complement-Dependent Cytotoxicity

Antibody-mediated complement dependent cytotoxic assays are known in the art. A method of Diaz *et al* (Diaz et al., 2004 Transplant Immunology 13(4):313-317) is performed. Human serum is obtained from healthy volunteers. Twenty-five percent heat inactivated serum is prepared. Heat-inactivated human sera are serially diluted and 100 µl of each concentration is placed in a 96 well v-bottom assay plate. The sera is mixed with a 100 µl aliquot of PBMC obtained from a pig of interest (GGTA1/CMAH/β4GalNT2 triple transgenic or other). PBMC final concentrations are either 5 x 10⁶/ml or 1 x 10⁶/ml. Serum concentrations vary from 50%, 17%, 2%, 0.6%, 0.2%, and 0.07%. The mixtures are incubated for 30 minutes at 4°C. After 30 minutes, the plates are centrifuged for 4 minutes at 400 x g. The plates are decanted and washed with HBSS. Rabbit complement (150 µl of a 1:15 dilution) is added to each well and incubated for 30 minutes at 37°C. PBMC are labeled with a fluorescein diacetate (FDA) stock solution, prepared fresh daily in HBSS (1 µg/ml) from a 1 mg/ml stock solution in acetone and with propidium iodide (PI), prepared at 50 µg/ml in phosphate buffered saline (PBS). After incubation in complement, the samples are transferred by pipette to tubes containing 250 µl of HBSS and 10 µl of FDA/PI for analysis using an Accuri C6 flow cytometer.

The percentage of dead cells (PI+/FDA-), damaged cells (PI+/FDA+) and live cells is determined. Double negative events (PI-/FDA-) are excluded from calculations. The percentage of cytotoxicity in cells not exposed to serum is considered spontaneous killing. Values for cytotoxicity are corrected for spontaneous killing.

### Example 22. Porcine Liver Procurement

Pigs are premedicated, intubated and anesthetized with propofol and placed in the supine position. A midline incision to the abdomen is made. Ligamentous attachments to the liver are taken down. The portal vein and hepatic artery are cannulated and flushed with 2 liters of cold histidine-tryptophan-ketoglutarate solution (Essential Pharmaceuticals, LLC). Livers are removed from pigs and stored in histidine-tryptophan-ketoglutarate solution on ice at 4°C until being placed in a liver perfusion circuit. Cold-ischemia time varies between 45 minutes to 3 hours. In certain experiments porcine livers may be obtained from abbatoirs. Porcine livers from abbatoirs are flushed with histidine-tryptophan-ketoglutarate solution containing heparin (2000 U/L) within two minutes of exsanguinations.

### Example 23. Platelet Uptake Analysis I

Platelets are labeled with carboxyfluorecein diacetate succinimidyl ester (CFSE), a fluorescent green cytoplasmic marker. A normothermic pig liver perfusion system is used. Pig livers from knockout pigs of interest or wild-type pigs are perfused for two hours prior to the addition of platelets. Approximately 300 billion platelets (unlabeled 70%, labeled 30%) are added to the perfusion system. Biopsies are taken from the pig livers at various predetermined time points. Biopsies are examined by confocal microscopy. Biopsies are treated with a stain specific for Wieble-Palade bodies. Wieble-Palade bodies occur in platelets and endothelial cells. Fluorescent ELISA based assays are performed. Platelets are human or baboon. Alternatively biopsies are labeled with endothelial markers and a lysosomal marker, prior to confocal microscopy

### Example 24. Platelet Uptake Analysis II

Platelets are labeled with carboxyfluorecein diacetate succinimidyl ester (CFSE), a fluorescent green cytoplasmic marker. A normothermic pig liver perfusion system is used. Pig livers from knockout pigs of interest or wild-type pigs are perfused for two hours prior to the addition of platelets. Biopsies are analyzed by transmission electron microscopy (TEM).

### Example 25. In vitro Platelet Uptake

Primary liver sinusoidal endothelial cells (LSECs) are isolated from the sinusoid of a pig liver or livers of interest. Primary wild-type porcine LSECs loose phagocytic ability after 5 days in culture; these experiments are performed with day 3 and 4 primary LSECs. Human or baboon platelets are labeled with CFSE as described elsewhere herein. Isolated LSECs and labeled platelets are incubated together. Samples are analyzed by confocal microscopy.

### Example 26. Kidney Xenograft in NHP

Recipient non-human primates (NHP) are treated with one dose of anti-CD4/anti-CD8 (50 mg/kg), anti-CD154/anti-CD28 dAbs, MMF and steroids. In certain studies tacrolimus is used (target levels 8-12). Rhesus macaques (*Macaca mulatta*) are used as the NHP. In some experiments, macaques may be 3-5 years old and less than 6 kg. Knockout porcine kidneys (or wildtype control kidneys) are transplanted into the NHP recipients. Samples (blood, urine and kidney biopsy samples) are collected at defined time points for analysis. Renal function, serum creatinine, the presence and quantity of xenoantibodies (flow cytometry and multi-parameter flow cytometry), cytokine secretion, transcript profiles from peripheral blood, urine and graft biopsies, xenograft histology and development of anti-pig antibody (flow-based xenocrossmatch assay) are followed. The CMAH deletion is not helpful for study in NHP. For NHP studies, pigs with a wild-type CMAH gene are used (Gal-/β4GalNT2). Ultrasound guided needle biopsies are performed at 2, 5 and 10 weeks post transplant.

### Example 27. Veterinary Care of NHP

NHP are housed in individual cages and provided with clean, adequately sized living quarters; fed twice daily; and are checked at least twice daily by animal care technicians and once daily by clinical veterinary staff. Physical examinations are performed each time an animal is anesthetized for blood collection or other procedures.

### Example 28. NHP Phlebotomy and Tissue Sampling

Phlebotomy and tissue sampling (for example: blood collections, lymph node biopsies and bone marrow aspirates) of NHP's are performed either under ketamine (10 mg/kg) or Telazol (4 mg/kg) anesthesia on fasting animals. Buprenephrine (0.01 mg/kg every 6 hrs) is administered as post-operative analgesia for animals undergoing renal transplant and as needed as determined by the attending veterinarian. Animals are monitored for "irreversible critical illness" such as but not limited to loss of 25% of body weight from baseline; complete anorexia for 4 days; major organ failure or medical conditions unresponsive to treatment such as respiratory distress, icterus, uremia, intractable diarrhea, self-mutilation or persistent vomiting, and surgical complications unresponsive to immediate intervention: bleeding, vascular graft/circulation failure, infection and wound dehiscence.

### Example 29. Porcine Embryo Transfer Surgery, Phlebotomy and Harvesting Procedures

Embryo transfer surgery: Before surgery, the sow is anesthetized with TKX (Telazol (500 mg) + Ketamine (250 mg) and Xylazine (250 mg); 1 cc per 50 lbs, IM) for intubation plus isoflurane by inhalation through ET tube using a precision vaporizer and waste gas scavenging. During the recovery period, animals are monitored at least once every 15 minutes and vital signs (temperature, heart rate, respiration rate and capillary refill time) are assessed and recorded. Trained animal care technicians or veterinarians monitor the animals until they can maintain themselves in voluntary sternal recumbrance. Animals are returned to regular housing areas upon approval by the attending veterinarian. Post-operative analgesics include buprenorphine 0.01-0.05 mg/kg IM every 8-12 hours or carprofen 2-4 mg/kg SC daily. Approximately 26 days after embryo transfer, ultrasound is performed to confirm establishment of pregnancy while the sow is distracted by food. About 10 days later a second ultrasound is performed. Birth occurs through natural parturition unless clinical difficulty arises. Caesarian section is performed recommended by the veterinary staff. Standard caesarian section protocols are used with the general anesthesia protocol utilized in the embryo transfer surgery. Experimental piglets are cleaned and the umbilical cord is disinfected. Every piglet receives colostrum during the first hours after birth. Piglets are watched 24/7 until they are at least 7 days old. Farrowing crates are used to protect the piglets from their mother while maintaining the piglets' ability to nurse.

All phlebotomy is performed under either ketamine (10 mg/k) or Telazol (4 mg/kg) anesthesia on fasting animals. Organ harvesting, a terminal surgical procedure, uses the anesthesia protocol (Telazol (500 mg) + ketamine (250 mg) +xylazine (250 mg); 1 cc per 50 Ibs; IM) +/- pentothal (10-20 mg/kg) IV if needed for intubation and isoflurane by inhalation through ET tube using a precision vaporizer, to effect with waste gas scavenging. Swine are perfused with saline followed by removal of the heart and other tissue/organs. Alternatively swine are anesthetized with inhaled anesthetic and treated with a barbituric acid derivative (100-150 mg/kg) and a bilateral pneumothorax is performed.

### Example 30. CFSE MLR Assessment of Immunosuppressive Agents on T Cell Proliferative Response

PBMCs from rhesus macaques are incubated with pig PBMCs from GGTA-/β4GalNT2-double knockout or control pigs. Dilution of CFSE is used to asses T cell proliferation in T cell subsets.

### Example 31. Assessment of Immunosuppressive Agents in Renal Xenograft

Recipient macaques are immunologically mature (CMV+, LCV+, SV40+, >4 kg), MHC- and pedigree-defined. Immunosuppressive candidates (anti-CD154 dAb, clone 5C8 anti-CD154) are administered to the rhesus macaques. Recipient macaques are treated with T-cell depletion (anti-CD4/anti-CD8, single dose), MMF, steroids and the immunosuppressive candidate prior to transplant. Renal function is assessed using serum creatinine. Increase in creatinine and/or BUN > 5.0 mg/dl or 100 mg/dl respectively are considered negative outcomes. Ultrasound guided needle biopsies are performed at 2, 5 and 10 weeks post-transplant. Pre-transplant and weekly post-transplant peripheral blood samples are collected for immunophenotyping using multi-parameter flow cytometry (T, B and other cellular subsets). Functional assays including *ex vivo* assessment of cytokine secretion as well as transcript profiles are peripheral blood, urine and graft biopsies at defined time points. Expression of costimulatory and co-inhibitory receptors (such as, but not limited to, ICOS, CTLA-4, BTLA, PD-1, LAG-3, TIM-3) in peripheral blood samples may be evaluated.

The invention is not limited to the embodiments set forth herein for illustration but includes everything that is within the scope of the claims. Having described the invention with reference to the exemplary embodiments, it is to be understood that it is not intended that any limitations or elements describing the exemplary embodiments set forth herein are to be incorporated into the meanings of the patent claims unless such limitations or elements are explicitly listed in the claims. Likewise, it is to be understood that it is not necessary to meet any or all of the identified advantages or objects of the invention disclosed herein in order to fall within the scope of any claims, since the invention is defined by the claims, and since inherent and/or unforeseen advantages of the present invention may exist even though they may not be explicitly discussed herein.

## Claims

1. A transgenic pig comprising a disrupted α(1,3)-galactosyltransferasegene, a disrupted cytidine monophosphate-N-acetylneuraminic acid hydroxylase (CMAH) gene and a disrupted β1,4 N-acetylgalactosaminyltransferase (β4GalNT2) gene in the nuclear genome of cells of said pig, wherein expression of α(1,3)-galactosyltransferase, CMAH, and β4GalNT2 is decreased as compared to a wild-type pig.

2. A porcine organ, tissue, or cell isolated from said transgenic pig of claim 1, wherein the porcine organ, tissue, or cell has reduced α(1,3)-galactosyltransferase, CMAH and β4GalNT2 expression as compared to a porcine organ, tissue, or cell isolated from a wild-type pig.

3. The porcine organ, tissue, or cell of claim 2, wherein said porcine organ, tissue, or cell is selected from the group consisting of skin, heart, liver, kidney, lung, pancreas, thyroid, small bowel, blood and components of any thereof.

4. The transgenic pig of claim 1 wherein when an organ, tissue, transfusion product or cell from said transgenic pig is transplanted into a human subject, a rejection related symptom is improved as compared to when an organ, tissue, transfusion product or cell from a wild-type pig is transplanted into a human subject, further wherein said disruption of the α(1,3)-galactosyltransferase, CMAH and β4GalNT2 genes reduces the level of αGal, Sd^{a}-like and Neu5Gc antigens in said organ, tissue, transfusion product or cell from said transgenic pig.

5. The transgenic pig of claim 4 wherein when said organ, tissue, transfusion product or cell from said transgenic pig is transplanted into a human subject, said rejection related symptom is selected from the group comprising a cellular rejection response related symptom, a humoral rejection response related symptom, a hyperacute rejection related symptom, an acute humoral xenograft reaction rejection related symptom and an acute vascular rejection response related symptom.

6. The transgenic pig of claim 4 wherein when said organ, tissue, transfusion product or cell from said transgenic pig is transplanted into a human subject, thrombocytopenia is decreased as compared to when an organ, tissue, transfusion product or cell from a wild-type pig is transplanted into a human subject.

7. The transgenic pig of claim 1 wherein when a liver from said transgenic pig is exposed to human platelets, said liver exhibits reduced platelet uptake as compared to when a liver from a wild-type pig is exposed to human platelets, further wherein said disruption of the α(1,3)-galactosyltransferase, CMAH and β4GalNT2 genes reduces the level of αGal, Sd^{a}-like and Neu5Gc antigens in said liver from said transgenic pig.

8. A transfusion product or skin related product obtained from the transgenic pig of claim 1, wherein said transfusion product or skin related product is for transplantation into a human, said disruption of the α(1,3)-galactosyltransferase, CMAH and β4GalNT2 genes reduces the level of αGal, Sd^{a}-like and Neu5Gc antigens in the transfusion product or skin related product, and said skin related product exhibits reduced premature separation from a wound.

9. The skin related product of claim 8, wherein said wound is a human skin wound.

10. The transgenic pig of claim 4 wherein said organ is a kidney.

11. A method of preparing transplant material for xenotransplantation into a human subject, the method comprising providing the transgenic pig of claim 1 as a source of said transplant material and wherein said transplant material is selected from the group consisting of organs, tissues, transfusion products and cells, and wherein said transplant material has a reduced level of αGal antigens, a reduced level of Neu5GC antigens and a reduced level of Sd^{a}-like antigens.

12. A transgenic pig comprising a disrupted α(1,3)-galactosyltransferasegene, a disrupted CMAH gene and a disrupted β4GalNT2 gene in the nuclear genome of cells of said pig, wherein the disruption of said α(1,3)-galactosyltransferase gene is selected from the group of disruptions comprising a 3 base pair deletion adjacent to a G to A substitution, a single base pair deletion, a single base pair insertion, a two base pair insertion, a six base pair deletion, a ten base pair deletion, a seven base pair deletion, an eight base pair insertion for a five base pair deletion, a five base pair insertion, an eleven base pair deletion and an eighteen base pair deletion; wherein the disruption of said CMAH gene is selected from the group of disruptions comprising a four base pair insertion, a one base pair deletion, a two base pair deletion, a three base pair deletion, a five base pair deletion, an eight base pair deletion, a twenty base pair deletion, an eleven base pair deletion, a twelve base pair deletion, a single base pair insertion, a two base pair insertion for single base pair deletion, a three base pair deletion for a four base pair insertion, a sixty-six base pair deletion and twelve base pair insertion, and a five base pair deletion with a one base pair substitution wherein the disruption of said β4GalNT2 gene is selected from the group of disruptions comprising a twelve base pair deletion, a five base pair deletion, a fourteen base pair deletion, a twelve base pair deletion and one base pair substitution, a 271 base pair deletion with a one base pair insertion, and a single base pair insertion, and wherein expression of α(1,3)-galactosyltransferase, CMAH and β4GalNT2 are decreased as compared to a wild-type pig, and when tissue from said transgenic pig is transplanted into a human subject, a hyperacute rejection related symptom is improved as compared to when tissue from a wild-type pig is transplanted into a human subject.

13. The transgenic pig of claim 12, wherein the disruption of said α(1,3)-galactosyltransferase gene is selected from the group comprising a five base pair deletion, a seven base pair deletion, and both a five base pair deletion and a seven base pair deletion, wherein the disruption of said CMAH gene is selected from the group of disruptions comprising a twelve base pair deletion and a four base pair substitution for a three base pair deletion, wherein the disruption of said β4GalNT2 gene is selected from the group of disruptions comprising a twelve base pair deletion, a five base pair deletion and a single base pair insertion, and wherein expression of α(1,3)-galactosyltransferase, CMAH and β4GalNT2 are decreased as compared to a wild-type pig, and when tissue from said transgenic pig is transplanted into a human subject, a hyperacute rejection related symptom is improved as compared to when tissue from a wild-type pig is transplanted into a human subject.

14. The transgenic pig of claim 12, wherein the disruption of the α(1,3)-galactosyltransferase gene is selected from the group of disruptions comprising an eleven base pair deletion and an eighteen base pair deletion, wherein the disruption of the CMAH gene is selected from the group of disruptions comprising a sixty-six base pair deletion/twelve base pair insertion and a five base pair deletion/one base pair substitution, wherein the disruption of the β4GalNT2 gene is selected from the group of disruptions comprising a fourteen base pair deletion, σ twelve base pair deletion, and a 271 base pair deletion/1 base pair insertion, and wherein expression of α(1,3)-galactosyltransferase, CMAH and β4GalNT2 are decreased as compared to a wild-type pig, and when tissue from said transgenic pig is transplanted into a human subject, a hyperacute rejection related symptom is improved as compared to when tissue from a wild-type pig is transplanted into a human subject.

15. The organ of claim 2 for use in a method of increasing the duration of the period between when a human subject is identified as a subject in need of a human organ transplant and when the human organ transplant occurs, said method comprising providing an organ of claim 2, and surgically attaching said organ to said human subject in a therapeutically effective manner.

16. The organ for use of claim 15, wherein said organ is surgically attached internal or external to said human subject.

17. The organ for use of claim 15, wherein said organ is directly or indirectly attached to said human subject.

18. The liver of claim 3 for use in a method of increasing the duration of the period between when a human subject is identified as a subject in need of a human liver transplant and when said human liver transplant occurs, said method comprising providing a liver of claim 3, and surgically attaching said liver to said human subject in a therapeutically effective manner.

19. The skin related product of claim 8 for use in treating a skin wound in a human subject, the use comprising the steps of providing the transgenic pig of claim 1, preparing the skin related product of claim 8, and applying said skin related product to the human subject, wherein premature separation of the skin related product from the human subject is reduced compared to a skin related product obtained from a wild type pig.

20. A porcine transplant material for use in a method of improving a hyperacute rejection related symptom in a human subject, wherein said porcine transplant material is isolated from the transgenic pig of claim 1, further wherein said disruption of the α(1,3)-galactosyltransferase, CMAH and β4GalNT2 genes reduces the level of αGal, Sd^{a}-like and Neu5GC antigens in said porcine transplant material, the method comprising transplanting the porcine transplant material into a subject in need of a transplant, wherein a hyperacute rejection related symptom is improved as compared to when porcine transplant material from a wild-type pig is transplanted into a human subject.

21. The porcine transplant material for use of claim 20, wherein the porcine transplant material is cells

22. A cell culture reagent that exhibits an altered epitope profile, wherein said cell culture reagent is isolated from the transgenic pig of claim 1, further wherein said disruption of the α(1,3)-galactosyltransferase, CMAH and β4GalNT2 genes reduces the level of αGal, Sd^{a}-like and Neu5Gc epitopes in the cell culture reagent.

23. The cell culture reagent of claim 22, wherein said cell culture reagent is selected from the group comprising cell culture serum, cell culture additive and an isolated cell capable of proliferation.

24. The cell culture reagent of claim 22, wherein said cell culture reagent is isolated from a transgenic pig wherein the disruption of said α(1,3)-galactosyltransferasegene is selected from the group comprising a five base pair deletion, a seven base pair deletion, and both a five base pair deletion and a seven base pair deletion, wherein the disruption of said CMAH gene is selected from the group of disruptions comprising a twelve base pair deletion and a five base pair substitution for a three base pair deletion, wherein the disruption of said β4GalNT2 gene is selected from the group of disruptions comprising a twelve base pair deletion, a five base pair deletion and a single base pair insertion.

25. The cell culture reagent of claim 22, wherein said cell culture reagent is isolated from a transgenic pig wherein the disruption of said α(1,3)-galactosyltransferase gene is selected from the group comprising disruption of the α(1,3)-galactosyltransferase gene is selected from the group of disruptions comprising an eleven base pair deletion and an eighteen base pair deletion, wherein the disruption of the CMAH gene is selected from the group of disruptions comprising a sixty-six base pair deletion/twelve base pair insertion and a five base pair deletion/one base pair substitution, wherein the disruption of the β4GalNT2 gene is selected from the group of disruptions comprising a fourteen base pair deletion, a twelve base pair deletion/one base pair substitution, and a 271 base pair deletion/1 base pair insertion.

26. A method of producing a compound of interest with an altered epitope profile, said method comprising the steps of providing the cell culture reagent of claim 22, and incubating an isolated cell capable of expressing said compound of interest with said cell culture reagent; and wherein the level of Neu5Gc, Sd^{a}-like or αGal epitopes on said compound of interest is lower than the level of said epitopes on said compound of interest when said compound of interest is produced from an isolated cell incubated with a cell culture reagent isolated from a wild-type pig.

27. The method of claim 26, wherein said compound of interest is selected from the group comprising glycolipids and glycoproteins.

28. The method of claim 27, wherein the compound of interest is a glycoprotein selected from the group of glycoproteins comprising antibodies, growth factors, cytokines, hormones and clotting factors.

29. The method of claim 26 wherein said cell culture reagent is isolated from a transgenic pig wherein the disruption of said α(1,3)-galactosyltransferase gene is selected from the group comprising a five base pair deletion, a seven base pair deletion, and both a five base pair deletion and a seven base pair deletion, wherein the disruption of said CMAH gene is selected from the group of disruptions comprising a twelve base pair deletion and a five base pair substitution for a three base pair deletion, wherein the disruption of said β4GalNT2 gene is selected from the group of disruptions comprising a twelve base pair deletion, a five base pair deletion and a single base pair insertion.

30. The method of claim 26 wherein said cell culture reagent is isolated from a transgenic pig wherein the disruption of said α(1,3)-galactosyltransferase gene is selected from the group of disruptions comprising an eleven base pair deletion and an eighteen base pair deletion, wherein the disruption of said CMAH gene is selected from the group of disruptions comprising a sixty-six base pair deletion/twelve base pair insertion and a five base pair deletion/one base pair substitution, wherein the disruption of said β4GalNT2 gene is selected from the group of disruptions comprising a fourteen base pair deletion, a twelve base pair deletion/one base pair substitution, and a 271 base pair deletion/1 base pair insertion, and wherein expression of α(1,3)-galactosyltransferase, CMAH and β4GalNT2 are decreased as compared to a wild-type pig.

31. A porcine transplant material for transplantation into a human subject, wherein said transplant material is isolated from the pig of claim 1, further wherein said disruption of the α(1,3)-galactosyltransferase, CMAH and β4GalNT2 genes reduces the level of αGal, Sd^{a}-like and Neu5Gc antigens in said transplant material.

32. The transgenic pig of claim 1, wherein *Vicia villosa* lectin (VVL) binding is reduced.

## Patentansprüche

1. Transgenes Schwein, umfassend ein gestörtes Gen für α(1,3)-Galactosyltransferase, ein gestörtes Gen für Cytidinmonophosphat-N-acetylneuraminsäurehydroxylase (CMAH) und ein gestörtes Gen für β1,4 N-Acetylgalactosaminyltransferase (β4GalNT2) im Kerngenom von Zellen des Schweins, wobei die Expression von α(1,3)-Galactosyltransferase, CMAH und β4GalNT2 im Vergleich zu einem Wildtypschwein verringert ist.

2. Porzines Organ, porzines Gewebe oder porzine Zelle, isoliert aus dem transgenen Schwein nach Anspruch 1, wobei das porzine Organ, das porzines Gewebe oder die porzine Zelle eine verringerte Expression von α(1,3)-Galactosyltransferase, CMAH und β4GalNT2 im Vergleich zu einem porzinen Organ, einem porzinen Gewebe oder einer porzinen Zelle, isoliert aus einem Wildtypschwein, aufweist.

3. Porzines Organ, porzines Gewebe oder porzine Zelle nach Anspruch 2, wobei das porzine Organ, das porzines Gewebe oder die porzine Zelle ausgewählt ist aus der Gruppe bestehend aus Haut, Herz, Leber, Niere, Lunge, Bauchspeicheldrüse, Schilddrüse, Dünndarm, Blut und Bestandteilen von jedem davon.

4. Transgenes Schwein nach Anspruch 1, wobei, wenn ein Organ, Gewebe, Transfusionsprodukt oder eine Zelle von dem transgenen Schwein in einen menschlichen Patienten transplantiert wird, ein mit Abstoßung zusammenhängendes Symptom im Vergleich zu dem Fall verbessert wird, in dem ein Organ, Gewebe, Transfusionsprodukt oder eine Zelle von einem Wildtypschwein in einen menschlichen Patienten transplantiert wird, ferner wobei die Störung der Gene für α(1,3)-Galactosyltransferase, CMAH und β4GalNT2 die Menge an αGal, Sd^{a}-ähnlichen und Neu5Gc-Antigenen in dem Organ, Gewebe, Transfusionsprodukt oder der Zelle von dem transgenen Schwein verringert.

5. Transgenes Schwein nach Anspruch 4, wobei, wenn das Organ, Gewebe, Transfusionsprodukt oder die Zelle von dem transgenen Schwein in einen menschlichen Patienten transplantiert wird, das mit Abstoßung zusammenhängende Symptom ausgewählt ist aus der Gruppe, umfassend ein mit einer zellulären Abstoßungsreaktion zusammenhängendes Symptom, ein mit einer humoralen Abstoßungsreaktion zusammenhängendes Symptom, ein mit einer hyperakuten Abstoßung zusammenhängendes Symptom, ein mit einer akuten humoralen Xenotransplantatreaktion zusammenhängendes Symptom und ein mit einer akuten vaskulären Abstoßungsreaktion zusammenhängendes Symptom.

6. Transgenes Schwein nach Anspruch 4, wobei, wenn das Organ, Gewebe, Transfusionsprodukt oder die Zelle von dem transgenen Schwein in einen menschlichen Patienten transplantiert wird, die Thrombozytopenie im Vergleich zu dem Fall verringert ist, in dem ein Organ, Gewebe, Transfusionsprodukt oder eine Zelle von einem Wildtypschwein in einen menschlichen Patienten transplantiert wird.

7. Transgenes Schwein nach Anspruch 1, wobei, wenn eine Leber von dem transgenen Schwein menschlichen Blutplättchen ausgesetzt wird, die Leber eine verringerte Blutplättchenaufnahme aufzeigt im Vergleich zu dem Fall, in dem eine Leber von einem Wildtypschwein menschlichen Blutplättchen ausgesetzt wird, ferner wobei die Störung der Gene für α(1,3)-Galactosyltransferase, CMAH und β4GalNT2 die Menge an αGal-, Sd^{a}-ähnlichen und Neu5Gc-Antigenen in der Leber von dem transgenen Schwein verringert.

8. Transfusionsprodukt oder hautbezogenes Produkt, erhalten von dem transgenen Schwein nach Anspruch 1, wobei das Transfusionsprodukt oder hautbezogene Produkt zur Transplantation in einen Menschen ist, die Störung der Gene für α(1,3)-Galactosyltransferase, CMAH und β4GalNT2 die Menge an αGal, Sd^{a}-ähnlichen und Neu5Gc-Antigenen in dem Transfusionsprodukt oder hautbezogenen Produkt verringert, und das hautbezogene Produkt eine verringerte vorzeitige Ablösung von einer Wunde aufzeigt.

9. Hautbezogenes Produkt nach Anspruch 8, wobei die Wunde eine menschliche Hautwunde ist.

10. Transgenes Schwein nach Anspruch 4, wobei das Organ eine Niere ist.

11. Verfahren zum Erzeugen von Transplantatmaterial zur Xenotransplantation in einen menschlichen Patienten, wobei das Verfahren das Bereitstellen des transgenen Schweins nach Anspruch 1 als eine Quelle des Transplantatmaterials umfasst und wobei das Transplantatmaterial ausgewählt ist aus der Gruppe bestehend aus Organen, Geweben, Transfusionsprodukten und Zellen, und wobei das Transplantatmaterial eine reduzierte Menge an αGal-Antigenen, eine reduzierte Menge an Neu5GC-Antigenen und eine reduzierte Menge an Sd^{a}-ähnlichen Antigenen aufweist.

12. Transgenes Schwein, umfassend ein gestörtes Gen für α(1,3)-Galactosyltransferase, ein gestörtes Gen für CMAH und ein gestörtes Gen für β4GalNT2 im Kerngenom von Zellen des Schweins, wobei die Störung des Gens für α(1,3)-Galactosyltransferase ausgewählt ist aus der Gruppe von Störungen, umfassend eine 3-Basenpaar-Deletion benachbart zu einer G-zu-A-Substitution, eine Ein-Basenpaar-Deletion, eine Ein-Basenpaar-Insertion, eine Zwei-Basenpaar-Insertion, eine Sechs-Basenpaar-Deletion, eine Zehn-Basenpaar-Deletion, eine Sieben-Basenpaar-Deletion, eine Acht-Basenpaar-Insertion für eine Fünf-Basenpaar-Deletion, eine Fünf-Basenpaar-Insertion, eine Elf-Basenpaar-Deletion und eine Achtzehn-Basenpaar-Deletion; wobei die Störung des Gens für CMAH ausgewählt ist aus der Gruppe von Störungen, umfassend eine Vier-Basenpaar-Insertion, eine Ein-Basenpaar-Deletion, eine Zwei-Basenpaar-Deletion, eine Drei-Basenpaar-Deletion, eine Fünf-Basenpaar-Deletion, eine Acht-Basenpaar-Deletion, eine Zwanzig-Basenpaar-Deletion, eine Elf-Basenpaar-Deletion, eine Zwölf-Basenpaar-Deletion, eine Ein-Basenpaar-Insertion, eine Zwei-Basenpaar-Insertion für eine Ein-Basenpaar-Deletion, eine Drei-Basenpaar-Deletion für eine Vier-Basenpaar-Insertion, eine Sechsundsechzig-Basenpaar-Deletion und Zwölf-Basenpaar-Insertion und eine Fünf-Basenpaar-Deletion mit einer Ein-Basenpaar-Substitution; wobei die Störung des Gens für β4GalNT2 ausgewählt ist aus der Gruppe von Störungen, umfassend eine Zwölf-Basenpaar-Deletion, eine Fünf-Basenpaar-Deletion, eine Vierzehn-Basenpaar-Deletion, eine Zwölf-Basenpaar-Deletion und eine Ein-Basenpaar-Substitution, eine 271-Basenpaar-Deletion mit einer Ein-Basenpaar-Insertion und eine Ein-Basenpaar-Insertion, und wobei die Expression von α(1,3)-Galactosyltransferase, CMAH und β4GalNT2 im Vergleich zu einem Wildtypschwein verringert ist, und wenn Gewebe von dem transgenen Schwein in einen menschlichen Patienten transplantiert wird, ein mit hyperakuter Abstoßung zusammenhängendes Symptom im Vergleich zu dem Fall verbessert wird, in dem Gewebe von einem Wildtypschwein in einen menschlichen Patienten transplantiert wird.

13. Transgenes Schwein nach Anspruch 12, wobei die Störung des Gens für α(1,3)-Galactosyltransferase ausgewählt ist aus der Gruppe, umfassend eine Fünf-Basenpaar-Deletion, eine Sieben-Basenpaar-Deletion und sowohl eine Fünf-Basenpaar-Deletion als auch eine Sieben-Basenpaar-Deletion, wobei die Störung des Gens für CMAH ausgewählt ist aus der Gruppe von Störungen, umfassend eine Zwölf-Basenpaar-Deletion und eine Vier-Basenpaar-Substitution für eine Drei-Basenpaar-Deletion, wobei die Störung des Gens für β4GalNT2 ausgewählt ist aus der Gruppe von Störungen, umfassend eine Zwölf-Basenpaar-Deletion, eine Fünf-Basenpaar-Deletion und eine Ein-Basenpaar-Insertion, und wobei die Expression von α(1,3)-Galactosyltransferase, CMAH und β4GalNT2 im Vergleich zu einem Wildtypschwein verringert ist, und wenn Gewebe von dem transgenen Schwein in einen menschlichen Patienten transplantiert wird, ein mit hyperakuter Abstoßung zusammenhängendes Symptom im Vergleich zu dem Fall verbessert wird, in dem Gewebe von einem Wildtypschwein in einen menschlichen Patienten transplantiert wird.

14. Transgenes Schwein nach Anspruch 12, wobei die Störung des Gens für α(1,3)-Galactosyltransferase ausgewählt ist aus der Gruppe von Störungen, umfassend eine Elf-Basenpaar-Deletion und eine Achtzehn-Basenpaar-Deletion, wobei die Störung des Gens für CMAH ausgewählt ist aus der Gruppe von Störungen, umfassend eine Sechsundsechzig-Basenpaar-Deletion/Zwölf-Basenpaar-Insertion und eine Fünf-Basenpaar-Deletion/Ein-Basenpaar-Substitution, wobei die Störung des Gens für β4GalNT2 ausgewählt ist aus der Gruppe von Störungen, umfassend eine Vierzehn-Basenpaar-Deletion, eine Zwölf-Basenpaar-Deletion und eine 271-Basenpaar-Deletion/1-Basenpaar-Insertion, und wobei die Expression von α(1,3)-Galactosyltransferase, CMAH und β4GalNT2 im Vergleich zu einem Wildtypschwein verringert ist, und wenn Gewebe von dem transgenen Schwein in einen menschlichen Patienten transplantiert wird, ein mit hyperakuter Abstoßung zusammenhängendes Symptom im Vergleich zu dem Fall verbessert wird, in dem Gewebe von einem Wildtypschwein in einen menschlichen Patienten transplantiert wird.

15. Organ nach Anspruch 2 zur Verwendung in einem Verfahren zum Verlängern der Dauer des Zeitraums zwischen dem Zeitpunkt, zu dem ein menschlicher Patient als ein Patient identifiziert wird, der einer menschlichen Organtransplantation bedarf, und dem Zeitpunkt, zu dem die menschliche Organtransplantation erfolgt, wobei das Verfahren das Bereitstellen eines Organs nach Anspruch 2 und das chirurgische Anbringen des Organs an den menschlichen Patienten in einer therapeutisch wirksamen Weise umfasst.

16. Organ zur Verwendung nach Anspruch 15, wobei das Organ intern oder extern an den menschlichen Patienten chirurgisch angebracht wird.

17. Organ zur Verwendung nach Anspruch 15, wobei das Organ direkt oder indirekt an den menschlichen Patienten angebracht wird.

18. Leber nach Anspruch 3 zur Verwendung in einem Verfahren zum Verlängern der Dauer des Zeitraums zwischen dem Zeitpunkt, zu dem ein menschlicher Patient als ein Patient identifiziert wird, der einer menschlichen Lebertransplantation bedarf, und dem Zeitpunkt, zu dem die menschliche Lebertransplantation erfolgt, wobei das Verfahren das Bereitstellen einer Leber nach Anspruch 3 und das chirurgische Anbringen der Leber an den menschlichen Patienten in einer therapeutisch wirksamen Weise umfasst.

19. Hautbezogenes Produkt nach Anspruch 8 zur Verwendung bei der Behandlung einer Hautwunde bei einem menschlichen Patienten, wobei die Verwendung die Schritte des Bereitstellens des transgenen Schweins nach Anspruch 1, des Erzeugens des hautbezogenen Produkts nach Anspruch 8 und des Aufbringens des hautbezogenen Produkts auf den menschlichen Patienten umfasst, wobei die vorzeitige Ablösung des hautbezogenen Produkts von dem menschlichen Patienten im Vergleich zu einem hautbezogenen Produkt, erhalten von einem Wildtypschwein, verringert ist.

20. Porzines Transplantatmaterial zur Verwendung in einem Verfahren zum Verbessern eines mit hyperakuter Abstoßung zusammenhängenden Symptoms bei einem menschlichen Patienten, wobei das porzine Transplantatmaterial aus dem transgenen Schwein nach Anspruch 1 isoliert ist, ferner wobei die Störung der Gene für α(1,3)-Galactosyltransferase, CMAH und β4GalNT2 die Menge an αGal-, Sd^{a}-ähnlichen und Neu5GC-Antigenen in dem porzinen Transplantatmaterial verringert, wobei das Verfahren das Transplantieren des porzinen Transplantatmaterials in einen Patienten, der einer Transplantation bedarf, umfasst, wobei ein mit hyperakuter Abstoßung zusammenhängendes Symptom im Vergleich zu dem Fall verbessert wird, in dem porzines Transplantatmaterial von einem Wildtypschwein in einen menschlichen Patienten transplantiert wird.

21. Porzines Transplantatmaterial zur Verwendung nach Anspruch 20, wobei das porzine Transplantatmaterial Zellen sind.

22. Zellkulturreagenz, das ein verändertes Epitopprofil aufzeigt, wobei das Zellkulturreagenz aus dem transgenen Schwein nach Anspruch 1 isoliert ist, ferner wobei die Störung der Gene für α(1,3)-Galactosyltransferase, CMAH und β4GalNT2 die Menge an αGal-, Sd^{a}-ähnlichen und Neu5Gc-Epitopen in dem Zellkulturreagenz verringert.

23. Zellkulturreagenz nach Anspruch 22, wobei das Zellkulturreagenz ausgewählt ist aus der Gruppe, umfassend Zellkulturserum, Zellkulturzusatz und eine isolierte Zelle, die zur Proliferation fähig ist.

24. Zellkulturreagenz nach Anspruch 22, wobei das Zellkulturreagenz aus einem transgenen Schwein isoliert ist, wobei die Störung des Gens für α(1,3)-Galactosyltransferase ausgewählt ist aus der Gruppe, umfassend eine Fünf-Basenpaar-Deletion, eine Sieben-Basenpaar-Deletion und sowohl eine Fünf-Basenpaar-Deletion als auch eine Sieben-Basenpaar-Deletion, wobei die Störung des Gens für CMAH ausgewählt ist aus der Gruppe von Störungen, umfassend eine Zwölf-Basenpaar-Deletion und eine Fünf-Basenpaar-Substitution für eine Drei-Basenpaar-Deletion, wobei die Störung des Gens für β4GalNT2 ausgewählt ist aus der Gruppe von Störungen, umfassend eine Zwölf-Basenpaar-Deletion, eine Fünf-Basenpaar-Deletion und eine Ein-Basenpaar-Insertion.

25. Zellkulturreagenz nach Anspruch 22, wobei das Zellkulturreagenz aus einem transgenen Schwein isoliert ist, wobei die Störung des Gens für α(1,3)-Galactosyltransferase ausgewählt ist aus der Gruppe, umfassend Störung des Gens für α(1,3)-Galactosyltransferase ausgewählt ist aus der Gruppe von Störungen, umfassend eine Elf-Basenpaar-Deletion und eine Achtzehn-Basenpaar-Deletion, wobei die Störung des Gens für CMAH ausgewählt ist aus der Gruppe von Störungen, umfassend eine Sechsundsechzig-Basenpaar-Deletion/Zwölf-Basenpaar-Insertion und eine Fünf-Basenpaar-Deletion/Ein-Basenpaar-Substitution, wobei die Störung des Gens für β4GalNT2 ausgewählt ist aus der Gruppe von Störungen, umfassend eine Vierzehn-Basenpaar-Deletion, eine Zwölf-Basenpaar-Deletion/Ein-Basenpaar-Substitution und eine 271-Basenpaar-Deletion/1-Basenpaar-Insertion.

26. Verfahren zum Erzeugen einer Verbindung von Interesse mit einem veränderten Epitopprofil, wobei das Verfahren die Schritte des Bereitstellens des Zellkulturreagenzes nach Anspruch 22 und des Inkubierens einer isolierten Zelle, die zur Expression der Verbindung von Interesse fähig ist, mit dem Zellkulturreagenz umfasst; und wobei die Menge an Neu5Gc-, Sd^{a}-ähnlichen oder αGal-Epitopen auf der Verbindung von Interesse niedriger ist als die Menge der Epitopen auf der Verbindung von Interesse, wenn die Verbindung von Interesse aus einer isolierten Zelle erzeugt wird, die mit einem Zellkulturreagenz, isoliert aus einem Wildtypschwein, inkubiert wird.

27. Verfahren nach Anspruch 26, wobei die Verbindung von Interesse ausgewählt ist aus der Gruppe, umfassend Glykolipide und Glykoproteine.

28. Verfahren nach Anspruch 27, wobei die Verbindung von Interesse ein Glykoprotein ist, ausgewählt aus der Gruppe von Glykoproteinen, umfassend Antikörper, Wachstumsfaktoren, Zytokine, Hormone und Gerinnungsfaktoren.

29. Verfahren nach Anspruch 26, wobei das Zellkulturreagenz aus einem transgenen Schwein isoliert ist, wobei die Störung des Gens für α(1,3)-Galactosyltransferase ausgewählt ist aus der Gruppe, umfassend eine Fünf-Basenpaar-Deletion, eine Sieben-Basenpaar-Deletion und sowohl eine Fünf-Basenpaar-Deletion als auch eine Sieben-Basenpaar-Deletion, wobei die Störung des Gens für CMAH ausgewählt ist aus der Gruppe von Störungen, umfassend eine Zwölf-Basenpaar-Deletion und eine Fünf-Basenpaar-Substitution für eine Drei-Basenpaar-Deletion, wobei die Störung des Gens für β4GalNT2 ausgewählt ist aus der Gruppe von Störungen, umfassend eine Zwölf-Basenpaar-Deletion, eine Fünf-Basenpaar-Deletion und eine Ein-Basenpaar-Insertion.

30. Verfahren nach Anspruch 26, wobei das Zellkulturreagenz aus einem transgenen Schwein isoliert ist, wobei die Störung des Gens für α(1,3)-Galactosyltransferase ausgewählt ist aus der Gruppe von Störungen, umfassend eine Elf-Basenpaar-Deletion und eine Achtzehn-Basenpaar-Deletion, wobei die Störung des Gens für CMAH ausgewählt ist aus der Gruppe von Störungen, umfassend eine Sechsundsechzig-Basenpaar-Deletion/Zwölf-Basenpaar-Insertion und eine Fünf-Basenpaar-Deletion/Ein-Basenpaar-Substitution, wobei die Störung des Gens für β4GalNT2 ausgewählt ist aus der Gruppe von Störungen, umfassend eine Vierzehn-Basenpaar-Deletion, eine Zwölf-Basenpaar-Deletion/Ein-Basenpaar-Substitution und eine 271-Basenpaar-Deletion/1-Basenpaar-Insertion, und wobei die Expression von α(1,3)-Galactosyltransferase, CMAH und β4GalNT2 im Vergleich zu einem Wildtypschwein verringert ist.

31. Porzines Transplantatmaterial zur Transplantation in einen menschlichen Patienten, wobei das Transplantatmaterial aus dem Schwein nach Anspruch 1 isoliert ist, ferner wobei die Störung der Gene für α(1,3)-Galactosyltransferase, CMAH und β4GalNT2 die Menge an αGal-, Sd^{a}-ähnlichen und Neu5Gc-Antigenen in dem Transplantatmaterial verringert.

32. Transgenes Schwein nach Anspruch 1, wobei die Bindung von *Vicia-villosa-*Lectin (VVL) verringert ist.

## Revendications

1. Porc transgénique comprenant un gène d'α(1,3)-galactosyltransférase perturbé, un gène de cytidine monophosphate-acide N-acétylneuraminique hydroxylase (CMAH) perturbé et un gène de β1,4 N-acétylgalactosaminyltransférase (β4GalNT2) perturbé dans le génome nucléaire de cellules dudit porc, dans lequel l'expression d'α(1,3)-galactosyltransférase, de CMAH, et de β4GalNT2 est diminuée par comparaison avec un porc de type sauvage.

2. Organe, tissu, ou cellule porcin isolé à partir dudit porc transgénique selon la revendication 1, dans lequel l'organe, le tissu, ou la cellule porcin a une expression réduite d'α(1,3)-galactosyltransférase, de CMAH et de β4GalNT2 par comparaison avec un organe, un tissu, ou une cellule porcin d'un porc de type sauvage.

3. Organe, tissu, ou cellule porcin selon la revendication 2, dans lequel ledit organe, tissu, ou cellule porcin est choisi dans le groupe constitué par la peau, le cœur, le foie, le rein, le poumon, le pancréas, la thyroïde, le petit intestin, le sang et des composants correspondants.

4. Porc transgénique selon la revendication 1, dans lequel quand un organe, un tissu, un produit de transfusion ou une cellule dudit porc transgénique est transplanté dans un sujet humain, un symptôme lié à un rejet est amélioré par comparaison avec un cas où un organe, un tissu, un produit de transfusion ou une cellule d'un porc de type sauvage est transplanté dans un sujet humain, en outre dans lequel ladite perturbation des gènes d'α(1,3)-galactosyltransférase, de CMAH et de β4GalNT2 réduit le taux d'antigènes αGal, de type Sd^{a} et Neu5Gc dans ledit organe, tissu, produit de transfusion ou cellule dudit porc transgénique.

5. Porc transgénique selon la revendication 4 dans lequel ledit organe, tissu, produit de transfusion ou cellule dudit porc transgénique est transplanté dans un sujet humain, ledit symptôme lié à un rejet est choisi dans le groupe comprenant un symptôme lié à une réponse de rejet cellulaire, un symptôme lié à une réponse de rejet humoral, un symptôme lié à un rejet hyperaigu, un symptôme lié à un rejet humoral aigu lié à une réaction de xénogreffe et un symptôme lié à une réponse de rejet vasculaire aigu.

6. Porc transgénique selon la revendication 4 dans lequel quand ledit organe, tissu, produit de transfusion ou cellule dudit porc transgénique est transplanté dans un sujet humain, une thrombocytopénie est diminuée par comparaison avec un cas où un organe, un tissu, un produit de transfusion ou une cellule d'un porc de type sauvage est transplanté dans un sujet humain.

7. Porc transgénique selon la revendication 1, dans lequel quand un foie dudit porc transgénique est exposé à des plaquettes humaines, ledit foie présente une captation plaquettaire réduite par comparaison avec un cas où un foie d'un porc de type sauvage est exposé à des plaquettes humaines, en outre dans lequel ladite perturbation des gènes d'α(1,3)-galactosyltransférase, de CMAH et de β4GalNT2 réduit le taux d'antigènes αGal, de type Sd^{a} et Neu5Gc dans ledit foie dudit porc transgénique.

8. Produit de transfusion ou produit lié à la peau obtenu auprès du porc transgénique selon la revendication 1, dans lequel ledit produit de transfusion ou le produit lié à la peau est destiné à une transplantation dans un humain, ladite perturbation des gènes d'α(1,3)-galactosyltransférase, de CMAH et de β4GalNT2 réduit le taux d'antigènes αGal, de type Sd^{a} etNeu5Gc dans le produit de transfusion ou le produit lié à la peau, et ledit produit lié à la peau présente une séparation prématurée réduite d'une plaie.

9. Produit lié à la peau selon la revendication 8, dans lequel ladite plaie est une plaie cutanée humaine.

10. Porc transgénique selon la revendication 4 dans lequel ledit organe est un rein.

11. Procédé de préparation d'un matériau de transplantation destiné à une xénotransplantation dans un sujet humain, le procédé comprenant la mise à disposition du porc transgénique selon la revendication 1 comme source dudit matériau de transplantation et dans lequel ledit matériau de transplantation est choisi dans le groupe constitué par des organes, des tissus, des produits de transfusion et des cellules, et dans lequel ledit matériau de transplantation a un taux réduit d'antigènes αGal, un taux réduit d'antigènes Neu5GC et un taux réduits d'antigènes de type Sd^{a}.

12. Porc transgénique comprenant un gène d'α(1,3)-galactosyltransférase perturbé, un gène de CMAH perturbé et un gène de β4GalNT2 perturbé dans le génome nucléaire de cellules dudit porc, dans lequel la perturbation dudit gène d'α(1,3)-galactosyltransférase est choisie dans le groupe de perturbations comprenant une délétion de 3 paires de bases adjacentes à une substitution de G en A, une délétion d'une seule paire de bases, une insertion d'une seule paire de bases, une insertion de deux paires de bases, une délétion de six paires de bases, une délétion de dix paires de bases, une délétion de sept paires de bases, une insertion de huit paires de bases pour une délétion de cinq paires de base, une insertion de cinq paires de base, une délétion de onze paires de bases et une délétion de dix-huit paires de base ; dans lequel la perturbation dudit gène de CMAH est choisie dans le groupe de perturbations comprenant une insertion de quatre paires de bases, une délétion d'une paire de bases, une délétion de deux paires de bases, une délétions de trois paires de bases, une délétion de cinq paires de bases, une délétion de huit paires de bases, une délétion de vingt paires de bases, une délétion de onze paires de bases, une délétion de douze paires de base, une insertion d'une seule paire de bases, une insertion de deux paires de bases pour une délétion d'une seule paire de bases, une délétion de trois paires de bases pour une insertion de quatre paires de bases, une délétion de soixante-six paires de bases et une insertion de douze paires de bases, et une délétion de cinq paires de bases avec une substitution d'une paire de bases dans lequel la perturbation dudit gène de β4GalNT2 est choisie dans le groupe de perturbations comprenant une délétion de douze paires de bases, une délétion de cinq paires de bases, une délétion de quatorze paires de bases, une délétion de douze paires de bases et une substitution d'une paire de bases, une délétion de 271 paires de bases avec une insertion d'une paire de bases, et une insertion d'une seule paire de bases, et dans lequel l'expression d'α(1,3)-galactosyltransférase, de CMAH et de β4GalNT2 sont diminuées par comparaison avec un porc de type sauvage, et quand un tissu dudit cochon transgénique est transplanté dans un sujet humain, un symptôme lié à un rejet hyperaigu est amélioré par comparaison avec un cas où un porc de type sauvage est transplanté dans un sujet humain.

13. Porc transgénique selon la revendication 12, dans lequel la perturbation dudit gène d'α(1,3)-galactosyltransférase est choisie dans le groupe comprenant une délétion de cinq paires de bases, une délétion de sept paires de base, et à la fois une délétion de cinq paires de bases et une délétion de sept paires de bases, dans lequel la perturbation dudit gène de CMAH est choisie dans le groupe de perturbations comprenant une délétion de douze paires de bases et une substitution de quatre paires de bases pour une délétion de trois paires de bases, dans lequel la perturbation dudit gène de β4GalNT2 est choisie dans le groupe de perturbations comprenant une délétion de douze paires de bases, une délétion de cinq paires de bases et une insertion d'une seule paire de bases, et dans lequel l'expression d'α(1,3)-galactosyltransférase, de CMAH et de β4GalNT2 sont diminuées par comparaison avec un porc de type sauvage, et quand un tissu dudit cochon transgénique est transplanté dans un sujet humain, un symptôme lié à un rejet hyperaigu est amélioré par comparaison avec un cas où un porc de type sauvage est transplanté dans un sujet humain.

14. Porc transgénique selon la revendication 12, dans lequel la perturbation du gène d'α(1,3)-galactosyltransférase est choisie dans le groupe de perturbations comprenant une délétion de onze paires de bases et une délétion de dix-huit paires de base, dans lequel la perturbation du gène de CMAH est choisie dans le groupe de perturbations comprenant une délétion de soixante-six paires de bases/insertion de douze paires de bases et une délétion de cinq paires de bases/substitution d'une paire de bases, dans lequel la perturbation dudit gène de β4GalNT2 est choisie dans le groupe de perturbations comprenant une délétion de quatorze paires de bases, une délétion de douze paires de bases, et une délétion de 271 paires de bases/insertion de 1 paire de bases, et dans lequel l'expression d'α(1,3)-galactosyltransférase, de CMAH et de β4GalNT2 sont diminuées par comparaison avec un porc de type sauvage, et quand un tissu dudit cochon transgénique est transplanté dans un sujet humain, un symptôme lié à un rejet hyperaigu est amélioré par comparaison avec un cas où un porc de type sauvage est transplanté dans un sujet humain.

15. Organe selon la revendication 2 destiné à être utilisé dans un procédé d'augmentation de la durée de la période entre le moment où un sujet humain est identifié comme un sujet ayant besoin d'une transplantation d'organe humain et le moment où la transplantation d'organe humain a lieu, ledit procédé comprenant la mise à disposition d'un organe selon la revendication 2, et le fait d'attacher chirurgicalement ledit organe audit sujet humain d'une manière thérapeutiquement efficace.

16. Organe destiné à être utilisé selon la revendication 15, dans lequel ledit organe est attaché chirurgicalement à l'intérieur ou à l'extérieur dudit sujet humain.

17. Organe destiné à être utilisé selon la revendication 15, dans lequel ledit organe est attaché directement ou indirectement audit sujet humain.

18. Foie selon la revendication 3 destiné à être utilisé dans un procédé d'augmentation de la durée de la période entre le moment où un sujet humain est identifié comme un sujet ayant besoin d'une transplantation de foie humain et le moment où ladite transplantation de foie humain a lieu, ledit procédé comprenant la mise à disposition d'un foie selon la revendication 3, et le fait d'attacher chirurgicalement ledit foie audit sujet humain d'une manière thérapeutiquement efficace.

19. Produit lié à la peau selon la revendication 8 destiné à être utilisé dans le traitement d'une plaie cutanée dans un sujet humain, l'utilisation comprenant les étapes de mise à disposition du porc transgénique selon la revendication 1, de préparation du produit lié à la peau selon la revendication 8, et d'application dudit produit lié à la peau au sujet humain, dans lequel la séparation prématurée du produit lié à la peau d'avec le sujet humain est réduite par comparaison à un produit lié à la peau provenant d'un porc de type sauvage.

20. Matériau de transplantation porcin destiné à être utilisé dans un procédé d'amélioration d'un symptôme lié à un rejet hyperaigu dans un sujet humain, dans lequel ledit matériau de transplantation porcin est isolé à partir du porc transgénique selon la revendication 1, en outre dans lequel ladite perturbation des gènes d'α(1,3)-galactosyltransférase, de CMAH et de β4GalNT2 réduit le taux d'antigènes αGal, de type Sd^{a} et Neu5GC dans ledit matériau de transplantation porcin, le procédé comprenant la transplantation du matériau de transplantation porcin dans un sujet ayant besoin d'une transplantation, dans lequel un symptôme lié à un rejet hyperaigu est amélioré par comparaison avec un cas où un matériau de transplantation porcin provenant d'un porc de type sauvage est transplanté dans un sujet humain.

21. Matériau de transplantation porcin destiné à être utilisé selon la revendication 20, dans lequel le matériau de transplantation porcin est des cellules.

22. Réactif de culture cellulaire qui présente un profil d'épitope altéré, dans lequel ledit réactif de culture cellulaire est isolé à partir du porc transgénique selon la revendication 1, en outre dans lequel ladite perturbation des gènes d'α(1,3)-galactosyltransférase, de CMAH et de β4GalNT2 réduit le niveau des épitopes αGal, de type Sd^{a} et Neu5Gc dans le réactif de culture cellulaire.

23. Réactif de culture cellulaire selon la revendication 22, dans lequel ledit réactif de culture cellulaire est choisi dans le groupe comprenant un sérum de culture cellulaire, un additif de culture cellulaire et une cellule isolée capable de prolifération.

24. Réactif de culture cellulaire selon la revendication 22, dans lequel ledit réactif de culture cellulaire est isolé à partir d'un porc transgénique dans lequel la perturbation dudit gène d'α(1,3)-galactosyltransférase est choisie dans le groupe comprenant une délétion de cinq paires de bases, une délétion de sept paires de bases, et à la fois une délétion de cinq paires de bases et une délétion de sept paires de bases, dans lequel la perturbation dudit gène de CMAH est choisie dans le groupe de perturbations comprenant une délétion de douze paires de bases et une substitution de cinq paires de bases pour une délétion de trois paires de bases, dans lequel la perturbation dudit gène de β4GalNT2 est choisie dans le groupe de perturbations comprenant une délétion de douze paires de base, une délétion de cinq paires de bases et une insertion d'une seule paire de bases.

25. Réactif de culture cellulaire selon la revendication 22, dans lequel ledit réactif de culture cellulaire est isolé à partir d'un porc transgénique dans lequel la perturbation dudit gène d'α(1,3)-galactosyltransférase est choisie dans le groupe comprenant la perturbation du gène d'α(1,3)-galactosyltransférase est choisie dans le groupe de perturbations comprenant une délétion de onze paires de bases et une délétion de dix-huit paires de bases, dans lequel la perturbation dudit gène de CMAH est choisie dans le groupe de perturbations comprenant une délétion de soixante-six paires de bases/insertion de douze paires de bases et une délétion de cinq paires de bases/une substitution d'une paire de bases, dans lequel la perturbation dudit gène de β4GalNT2 est choisie dans le groupe de perturbations comprenant une délétion de quatorze paires de base, une délétion de douze paires de bases/une substitution d'une paire de bases, et une délétion de 271 paires de bases/insertion de 1 paire de bases.

26. Procédé de production d'un composé d'intérêt avec un profil d'épitope altéré, ledit procédé comprenant les étapes de mise à disposition du réactif de culture cellulaire selon la revendication 22, et l'incubation d'une cellule isolée capable d'exprimer ledit composé d'intérêt avec ledit réactif de culture cellulaire ; et dans lequel le taux d'épitopes Neu5Gc, de type Sd^{a} ou αGal sur ledit composé d'intérêt est inférieur au taux desdits épitopes sur ledit composé d'intérêt quand ledit composé d'intérêt est produit à partir d'une cellule isolée incubée avec un réactif de culture cellulaire isolé à partir d'un porc de type sauvage.

27. Procédé selon la revendication 26, dans lequel ledit composé d'intérêt est choisi dans le groupe comprenant des glycolipides et des glycoprotéines.

28. Procédé selon la revendication 27, dans lequel le composé d'intérêt est une glycoprotéine choisie dans le groupe de glycoprotéines comprenant des anticorps, des facteurs de croissance, des cytokines, des hormones et des facteurs de coagulation.

29. Procédé selon la revendication 26, dans lequel ledit réactif de culture cellulaire est isolé à partir d'un porc transgénique dans lequel la perturbation dudit gène d'α(1,3)-galactosyltransférase est choisie dans le groupe comprenant une délétion de cinq paires de bases, une délétion de sept paires de bases, et à la fois une délétion de cinq paires de bases et une délétion de sept paires de bases, dans lequel la perturbation dudit gène de CMAH est choisie dans le groupe de perturbations comprenant une délétion de douze paires de bases et une substitution de cinq paires de bases pour une délétion de trois paires de bases, dans lequel la perturbation dudit gène de β4GalNT2 est choisie dans le groupe de perturbations comprenant une délétion de douze paires de base, une délétion de cinq paires de bases et une insertion d'une paire de bases.

30. Procédé selon la revendication 26, dans lequel ledit réactif de culture cellulaire est isolé à partir d'un porc transgénique dans lequel la perturbation dudit gène d'α(1,3)-galactosyltransférase est choisie dans le groupe de perturbations comprenant une délétion de onze paires de bases et une délétion de dix-huit paires de bases, dans lequel la perturbation dudit gène de CMAH est choisie dans le groupe de perturbations comprenant une délétion de soixante-six paires de bases/insertion de douze paires de bases et une délétion de cinq paires de bases/une substitution d'une paire de bases, dans lequel la perturbation dudit gène de β4GalNT2 est choisie dans le groupe de perturbations comprenant une délétion de quatorze paires de base, une délétion de douze paires de bases/une substitution d'une paire de bases, et une délétion de 271 paires de bases/insertion de 1 paire de bases et dans lequel l'expression d'α(1,3)-galactosyltransférase, de CMAH et de β4GalNT2 sont diminuées par comparaison avec un cochon de type sauvage.

31. Matériau de transplantation porcin pour une transplantation dans un sujet humain, dans lequel ledit matériau de transplantation est isolé à partir du porc selon la revendication 1, en outre dans lequel ladite perturbation des gènes d'α(1,3)-galactosyltransférase, de CMAH et de β4GalNT2 réduit le taux d'antigènes αGal, de type Sd^{a} et Neu5Gc dans ledit matériau de transplantation.

32. Porc transgénique selon la revendication 1, dans lequel la liaison à la lectine *Vicia villosa* (VVL) est réduite.
